# EUROPEAN PATENT APPLICATION

(11) **EP 4 442 700 A1**
(43) Date of publication of application: **09.10.2024**
(21) Application number: 22900581.4
(22) Date of filing: 01.12.2022
(51) Int. Cl.: C07K 14/605, A61K 38/26, A61K 47/26, A61K 47/38, A61K 47/18, A61P 3/10, A61P 3/04

(54) **PHARMACEUTICAL COMPOSITION OF GLP-1 AND GIP RECEPTOR DUAL AGONIST AND USE THEREOF**

(30) Priority: 01.12.2021 CN 202111454626
(71) Applicant: Jiangsu Hengrui Pharmaceuticals Co., Ltd., Lianyungang, Jiangsu 222047 (CN)
(72) Inventor: HE, Hua, Lianyungang, Jiangsu 222047 (CN); CHEN, Hao, Lianyungang, Jiangsu 222047 (CN); ZHANG, Feiran, Lianyungang, Jiangsu 222047 (CN); XU, Jiajia, Lianyungang, Jiangsu 222047 (CN); ZHANG, Qiang, Lianyungang, Jiangsu 222047 (CN); CAO, Xiaoli, Lianyungang, Jiangsu 222047 (CN)
(74) Representative: Regimbeau
(86) International application number: PCT/CN2022/135769
(87) International publication number: WO 2023/098777

(57) **Abstract**

Provided are a pharmaceutical composition of GLP-1 and GIP receptor dual agonist and the use thereof. The pharmaceutical composition has good dissolution characteristics and stability.

## Description

The present disclosure claims priority to the Chinese Patent Application (Application No. 202111454626.6) filed on Dec. 01, 2021.

### TECHNICAL FIELD

The present disclosure relates to the field of pharmaceutical formulations, in particular to a pharmaceutical composition comprising a GLP-1 and GIP receptor dual-agonist and pharmaceutical use thereof.

### BACKGROUND

At present, many glucagon-like peptide-1 (GLP-1) drugs are available on the market and are mainly used for treating type 2 diabetes mellitus (T2DM), such as liraglutide, semaglutide, and dulaglutide, where liraglutide is also approved for marketing by FDA as a weight-loss drug. Under physiological conditions, active GLP-1 consists of 30 amino acids and is secreted by cleavage of pro-glucagon by the PC1/3 enzyme in L cells of the intestinal tract after meals. In a T2DM patient, the secretion of GLP-1 after meals is significantly inhibited, but the response of GLP-1R of the patient to GLP-1 at a pharmacological concentration is not significantly different from that of a normal person, which further proves that the target is of great treatment potential. Although GLP-1R agonists exhibit great advantages in terms of the therapeutic effects such as blood glucose lowering and weight loss, their actions on the central nervous system and stomach would cause nausea and vomiting, that is, there would be dose-dependent gastrointestinal adverse reactions. If the therapeutic dose of the GLP-1 drugs is limited and one can not continue to increase the dose to achieve more remarkable treatment effects in glucose lowering, weight loss, etc., there is a need to supplement with other treatment schemes to enhance the treatment effect or reduce the occurrence rate of adverse reactions of the GLP-1 drugs.

Glucose-dependent insulinotropic polypeptide (GIP) also belongs to the incretins. The active GIP contains 42 amino acids and is produced by the cleavage of the GIP precursor by the PC1/3 enzyme in K cells secreted in the intestines. The GIP can play a role in comprehensively regulating the nervous system and the endocrine system at the same time and thereby improve metabolism. GIP can be effective by directly influencing the central nervous system, pancreas and stomach and indirectly acting on the liver, plus the active ingredient taking effect on fat and muscle tissues, thereby comprehensively improving metabolism. Studies have shown that in non-insulin-dependent diabetic patients, the incretin function of GIP polypeptide is greatly reduced, resulting in a lack or loss of incretin effect in the patients. The inhibitory properties of the GIP polypeptide produced by those diabetic patients are greatly diminished when the blood glucose level returns to normal.

Therefore, there is a clinical need for a method for treating non-insulin-dependent diabetes using the GIP polypeptide in combination with a clinically effective drug for lowering blood glucose to restore the tolerance of the non-insulin-dependent diabetic patients to the GIP polypeptide, and further in combination with the incretin effect of the GIP polypeptide to obtain a stronger clinical glucose lowering effect.

PCT/CN2021/096568 provides a derivative of a GLP-1 analog having agonist activity for human GIP receptor, which has dual-agonist effect on human GLP-1 receptor and human GIP receptor; compared with GLP-1 receptor agonists known in the art, the derivative has stronger curative effects of blood glucose lowering and weight loss, has extremely high plasma stability, and has the pharmacokinetic characteristics of once-a-week subcutaneous injection on human subjects. However, chemically modified polypeptide drugs have a complex structure, are easily degraded, polymerized, or undesirably chemically modified, and thus become unstable; in order to make them suitable for administration, maintain stability during storage and subsequent use, and exert a better therapeutic effect, it is particularly important to develop stable formulations of the chemically modified polypeptide drugs.

### SUMMARY

The present disclosure provides a pharmaceutical composition, which comprises:
(a) a GLP-1 analog of general formula (I) or a pharmaceutically acceptable salt, amide or ester thereof, 
R1-X1-X2-Glu-Gly-Thr-Phe-Thr-Ser-Asp-X10-Ser-X12-X13-X14-X15-X16-X17-X18-X19-
X20-Glu-Phe-X23-X24-Trp-Leu-X27-X28-X29-X30-Pro-Ser-Ser-Gly-Ala-Pro-Pro-Pro-Ser-
R2
(I) (SEQ ID NO: 19)
 wherein:
   R₁ is hydrogen (H), alkyl, acetyl, formyl, benzoyl, trifluoroacetyl, pGlu, or absent;
   R₂ is -NH₂, -OH, or absent;
   X₁, X₂, X₁₀, X₁₂, X₁₃, X₁₄, X₁₅, X₁₆, X₁₇, X₁₈, X₁₉, X₂₀, X₂₃, X₂₄, X₂₇, X₂₈, X₂₉, and X₃₀ are independently selected from the group consisting of any natural amino acid residues, any non-natural amino acid residues, and peptide fragments composed of natural amino acid residues and/or non-natural amino acid residues; and
(b) an absorption enhancer.

In some embodiments, the absorption enhancer is selected from one or more of *N-*(8-(2-hydroxybenzoyl)amino)caprylate, sodium caprylate, sodium caprate, sodium taurodeoxycholate, lauroyl carnitine, dodecyl phosphatidylcholine, an amino acid, cyclodextrin and a derivative thereof, a surfactant, laurocapram and a homolog thereof, an alcohol-based organic solvent, an organic acid, and fatty alcohol.

In some embodiments, the amino acid includes, but is not limited to, one or more of isoleucine, leucine, lysine, methionine, phenylalanine, threonine, tryptophan, valine, arginine, histidine, alanine, glycine, proline, serine, and salts thereof, such as one or more of arginine, histidine, leucine and lysine and salts thereof.

In some embodiments, the cyclodextrin and the derivative thereof include, but are not limited to, one or more of α-cyclodextrin, β-cyclodextrin, γ-cyclodextrin, sulfobutyl-β-cyclodextrin (SBE-β-CD), 2,6-dimethyl-β-cyclodextrin, hydroxypropyl-β-cyclodextrin, hydroxyethyl-β-cyclodextrin, methylated-β-cyclodextrin, glucosyl-β-cyclodextrin, diglucosyl-β-cyclodextrin, maltosyl-β-cyclodextrin, dimaltosy-β-cyclodextrin, and carboxymethyl-β-cyclodextrin, such as β-cyclodextrin.

In some embodiments, the surfactant includes, but is not limited to, sodium dodecyl sulfate, polysorbate, betaine, quaternary ammonium salt, sorbitan ester, poloxamer, or any mixtures thereof.

In some embodiments, the alcohol-based organic solvent includes, but is not limited to, ethanol, glycerol, polyethylene glycol, propylene glycol, or any mixtures thereof.

In some embodiments, the organic acid includes, but is not limited to, stearic acid, palmitic acid, oleic acid, lauric acid, or any mixtures thereof; and/or in some embodiments, the fatty alcohol includes, but is not limited to, lauryl alcohol, cetyl alcohol, stearyl alcohol, or any mixtures thereof.

In some embodiments, the absorption enhancer is selected from any one of the following:
(1) *N-*(8-(2-hydroxybenzoyl)amino)caprylate;
(2) sodium caprate;
(3) *N-*(8-(2-hydroxybenzoyl)amino)caprylate and sodium caprylate;
(4) *N-*(8-(2-hydroxybenzoyl)amino)caprylate and an amino acid or a salt thereof;
(5) *N-*(8-(2-hydroxybenzoyl)amino)caprylate and β-cyclodextrin;
(6) *N*-(8-(2-hydroxybenzoyl)amino)caprylate, an amino acid or a salt thereof, and sodium caprate; and
(7) *N*-(8-(2-hydroxybenzoyl)amino)caprylate, an amino acid or a salt thereof, and sodium caprylate.

In some embodiments, the *N-*(8-(2-hydroxybenzoyl)amino)caprylate comprises one monovalent cation, two monovalent cations, or one divalent cation. In some embodiments, the *N-*(8-(2-hydroxybenzoyl)amino)caprylate is selected from the group consisting of sodium salt, potassium salt, and calcium salt of *N*-(8-(2-hydroxybenzoyl)amino)caprylic acid. The *N-*(8-(2-hydroxybenzoyl)amino)caprylate may be crystalline and/or amorphous. In some embodiments, the delivery agent comprises an anhydrate, monohydrate, dihydrate, trihydrate, solvate, or one-third hydrate of *N-*(8-(2-hydroxybenzoyl)amino)caprylate and combinations thereof.

In some embodiments, the *N-*(8-(2-hydroxybenzoyl)amino)caprylate is sodium *N*-(8-(2-hydroxybenzoyl)amino)caprylate (also referred to herein as "SNAC"), also referred to as sodium 8-(salicylamido)caprylate.

In some embodiments, the absorption enhancer is selected from any one of the following:
(1) SNAC;
(2) sodium caprate;
(3) SNAC and sodium caprylate;
(4) SNAC and arginine or a salt thereof;
(5) SNAC and β-cyclodextrin;
(6) SNAC, arginine or a salt thereof, and sodium caprate;
(7) SNAC, arginine or a salt thereof, and leucine or a salt thereof;
(8) SNAC, arginine or a salt thereof, and lysine or a salt thereof; and
(9) SNAC, arginine or a salt thereof, and sodium caprylate. In some embodiments, the absorption enhancer accounts for at least 20% of the total mass of the composition.

In some embodiments, the absorption enhancer accounts for at least 50% of the total mass of the composition.

In some embodiments, the absorption enhancer accounts for at least 70% of the total mass of the composition.

In some embodiments, the absorption enhancer accounts for at least 80% or at least 90% of the total mass of the composition.

In some embodiments, the absorption enhancer is SNAC, and the SNAC accounts for at least 20%, at least 50%, at least 55%, at least 60%, at least 65%, at least 70%, at least 75%, at least 80%, at least 85%, at least 90%, or at least 95% of the total mass of the composition.

In some embodiments, the absorption enhancer is sodium caprate, and the sodium caprate accounts for at least 50%, at least 55%, at least 60%, at least 65%, at least 70%, at least 75%, or at least 80% of the total mass of the composition.

In some embodiments, the absorption enhancer is SNAC and sodium caprylate, and the total mass of the SNAC and the sodium caprylate accounts for at least 50%, at least 55%, at least 60%, at least 65%, at least 70%, at least 75%, at least 80%, at least 85%, at least 90%, or at least 95% of the total mass of the composition. In some embodiments, the absorption enhancer is SNAC and sodium caprylate; the SNAC accounts for 70%-90% of the total mass of the composition, and the sodium caprylate accounts for 5%-10% of the total mass of the composition. In some embodiments, the absorption enhancer is SNAC and sodium caprylate; the SNAC accounts for 50%-70% or 50%-85% of the total mass of the composition, and the sodium caprylate accounts for 10%-45% of the total mass of the composition. In some embodiments, the absorption enhancer is SNAC and sodium caprylate; the SNAC accounts for 20%-50% of the total mass of the composition, and the sodium caprylate accounts for 10%-75% or 40%-75% of the total mass of the composition.

In some embodiments, the absorption enhancer is SNAC and arginine or a salt thereof, and the total mass of the SNAC and the arginine or the salt thereof accounts for at least 50%, at least 55%, at least 60%, at least 65%, at least 70%, at least 75%, at least 80%, at least 85%, at least 90%, or at least 95% of the total mass of the composition. In some embodiments, the absorption enhancer is SNAC and arginine or a salt thereof; the SNAC accounts for 70%-90% of the total mass of the composition, and the arginine or the salt thereof accounts for 5%-10% of the total mass of the composition. In some embodiments, the absorption enhancer is SNAC and arginine or a salt thereof; the SNAC accounts for 70%-90% of the total mass of the composition, and the arginine or the salt thereof accounts for 1%-20% of the total mass of the composition. In some embodiments, the absorption enhancer is SNAC and arginine or a salt thereof; the SNAC accounts for 70%-90% of the total mass of the composition, and the arginine or the salt thereof accounts for 5%-25% of the total mass of the composition. In some embodiments, the absorption enhancer is SNAC and arginine or a salt thereof; the SNAC accounts for 50%-70% or 50%-85% of the total mass of the composition, and the arginine or the salt thereof accounts for 10%-45% of the total mass of the composition. In some embodiments, the absorption enhancer is SNAC and arginine or a salt thereof; the SNAC accounts for 20%-50% of the total mass of the composition and the arginine or the salt thereof accounts for 10%-75% or 40%-75%, of the total mass of the composition.

In some embodiments, the absorption enhancer is SNAC and β-cyclodextrin, and the total mass of the SNAC and the β-cyclodextrin accounts for at least 50%, at least 55%, at least 60%, at least 65%, at least 70%, at least 75%, at least 80%, at least 85%, at least 90%, or at least 95% of the total mass of the composition. In some embodiments, the absorption enhancer is SNAC and β-cyclodextrin; the SNAC accounts for 70%-90% of the total mass of the composition, and the β-cyclodextrin accounts for 5%-10% or 1%-20% of the total mass of the composition. In some embodiments, the absorption enhancer is SNAC and β-cyclodextrin; the SNAC accounts for 50%-70% or 50%-85% of the total mass of the composition, and the β-cyclodextrin accounts for 10%-45% of the total mass of the composition. In some embodiments, the absorption enhancer is SNAC and β-cyclodextrin; the SNAC accounts for 20%-50% of the total mass of the composition, and the β-cyclodextrin accounts for 10%-75% or 40%-75% of the total mass of the composition.

In some embodiments, the absorption enhancer is SNAC, arginine or a salt thereof, and sodium caprate, and the total mass of the SNAC, the arginine or the salt thereof, and the sodium caprate accounts for at least 50%, at least 55%, at least 60%, at least 65%, at least 70%, at least 75%, at least 80%, at least 85%, at least 90%, or at least 95% of the total mass of the composition. In some embodiments, the absorption enhancer is SNAC, arginine or a salt thereof, and sodium caprate; the SNAC accounts for 50%-90% of the total mass of the composition, the arginine or the salt thereof accounts for 1%-30% of the total mass of the composition, and the sodium caprate accounts for 1%-30% of the total mass of the composition. In some embodiments, the absorption enhancer is SNAC, arginine or a salt thereof, and sodium caprate; the SNAC accounts for 20%-50% of the total mass of the composition, the arginine or the salt thereof accounts for 10%-35% of the total mass of the composition, and the sodium caprate accounts for 15%-40% of the total mass of the composition. In some embodiments, the absorption enhancer is SNAC, arginine or a salt thereof, and sodium caprate; the SNAC accounts for 50%-70% of the total mass of the composition, the arginine or the salt thereof accounts for 10%-35% of the total mass of the composition, and the sodium caprate accounts for 10%-35% of the total mass of the composition.

In some embodiments, the absorption enhancer is SNAC, arginine or a salt thereof, and leucine or a salt thereof, and the total mass of the SNAC, the arginine or the salt thereof, and the leucine or the salt thereof accounts for at least 50%, at least 55%, at least 60%, at least 65%, at least 70%, at least 75%, at least 80%, at least 85%, at least 90%, or at least 95% of the total mass of the composition. In some embodiments, the absorption enhancer is SNAC, arginine or a salt thereof, and leucine or a salt thereof; the SNAC accounts for 50%-90% of the total mass of the composition, the arginine or the salt thereof accounts for 1%-30% of the total mass of the composition, and the leucine or the salt thereof accounts for 1%-30% of the total mass of the composition. In some embodiments, the absorption enhancer is SNAC, arginine or a salt thereof, and leucine or a salt thereof; the SNAC accounts for 20%-50% of the total mass of the composition, the arginine or the salt thereof accounts for 10%-35% of the total mass of the composition, and the leucine or the salt thereof accounts for 15%-40% of the total mass of the composition. In some embodiments, the absorption enhancer is SNAC, arginine or a salt thereof, and leucine or a salt thereof; the SNAC accounts for 50%-70% of the total mass of the composition, the arginine or the salt thereof accounts for 10%-35% of the total mass of the composition, and the leucine or the salt thereof accounts for 10%-35% of the total mass of the composition.

In some embodiments, the absorption enhancer is SNAC, arginine or a salt thereof, and lysine or a salt thereof, and the total mass of the SNAC, the arginine or the salt thereof, and the lysine or the salt thereof accounts for at least 50%, at least 55%, at least 60%, at least 65%, at least 70%, at least 75%, at least 80%, at least 85%, at least 90%, or at least 95% of the total mass of the composition. In some embodiments, the absorption enhancer is SNAC, arginine or a salt thereof, and lysine or a salt thereof; the SNAC accounts for 50%-90% of the total mass of the composition, the arginine or the salt thereof accounts for 1%-30% of the total mass of the composition, and the lysine or the salt thereof accounts for 1%-30% of the total mass of the composition. In some embodiments, the absorption enhancer is SNAC, arginine or a salt thereof, and lysine or a salt thereof; the SNAC accounts for 20%-50% of the total mass of the composition, the arginine or the salt thereof accounts for 10%-35% of the total mass of the composition, and the lysine or the salt thereof accounts for 15%-40% of the total mass of the composition. In some embodiments, the absorption enhancer is SNAC, arginine or a salt thereof, and lysine or a salt thereof; the SNAC accounts for 50%-70% of the total mass of the composition, the arginine or the salt thereof accounts for 10%-35% of the total mass of the composition, and the lysine or the salt thereof accounts for 10%-35% of the total mass of the composition.

In some embodiments, the absorption enhancer is SNAC, arginine or a salt thereof, and sodium caprylate, and the total mass of the SNAC, the arginine or the salt thereof, and the sodium caprylate accounts for at least 50%, at least 55%, at least 60%, at least 65%, at least 70%, at least 75%, at least 80%, at least 85%, at least 90%, or at least 95% of the total mass of the composition. In some embodiments, the absorption enhancer is SNAC, arginine or a salt thereof, and sodium caprylate; the SNAC accounts for 50%-90% of the total mass of the composition, the arginine or the salt thereof accounts for 1%-30% of the total mass of the composition, and the sodium caprylate accounts for 1%-30% of the total mass of the composition. In some embodiments, the absorption enhancer is SNAC, arginine or a salt thereof, and sodium caprylate; the SNAC accounts for 20%-50% of the total mass of the composition, the arginine or the salt thereof accounts for 15%-40% of the total mass of the composition, and the sodium caprylate accounts for 10%-35% of the total mass of the composition. In some embodiments, the absorption enhancer is SNAC, arginine or a salt thereof, and sodium caprylate; the SNAC accounts for 50%-70% of the total mass of the composition, the arginine or the salt thereof accounts for 10%-35% of the total mass of the composition, and the sodium caprylate accounts for 10%-35% of the total mass of the composition.

In some embodiments, the GLP-1 analog or the pharmaceutically acceptable salt, amide or ester thereof accounts for 0.1%-30%, 1%-20%, 1%-10%, or 1%-5% of the total mass of the composition.

In some embodiments, for the GLP-1 analog of general formula (I), X₁ is Tyr or His; X₂ is Aib or D-Ala; X₁₀ is Val or Tyr; X₁₂ is Ser or Ile; X₁₃ is Tyr or Ala; X₁₄ is Leu or Nle; X₁₅ is Asp or Glu; X₁₆ is Arg, Glu, Gly, Lys, or Aib; X₁₇ is Glu, Ile, or Gln; X₁₈ is Ala, Aib, or His; X₁₉ is Ala, Aib, or Gln; X₂₀ is Gln, Glu, or Lys; X₂₃ is Ile or Val; X₂₄ is Ala, Asn, or Gln; X₂₇ is Val or Leu; X₂₈ is Arg or Ala; X₂₉ is Gly or Gln; X₃₀ is Gly or Lys. In some embodiments, X₁ is Tyr or His; X₂ is Aib or D-Ala; X₁₀ is Val, Tyr, or Y1; X₁₂ is Ser, Ile, or Y1; X₁₃ is Tyr, Ala, or Y1; X₁₄ is Leu, Nle, or Y1; X₁₅ is Asp or Glu; X₁₆ is Arg, Glu, Gly, Lys, Aib, or Y1; X₁₇ is Glu, Ile, Gln, or Y1; X₁₈ is Ala, Aib, or His; X₁₉ is Ala, Aib, or Gln; X₂₀ is Gln, Glu, or Lys; X₂₃ is Ile or Val; X₂₄ is Ala, Asn, or Gln; X₂₇ is Val or Leu; X₂₈ is Arg or Ala; X₂₉ is Gly or Gln; X₃₀ is Gly or Lys; Y1 is a substituted Lys, Orn, Dap, Dab, or Cys residue, for example, a modified group is present on a side chain of the Lys, Orn, Dap, Dab, or Cys residue.

In some embodiments, Y1 is a Lys, Orn, Dap, Dab, or Cys residue with a substituent on a side chain, the substituent having a structure of formula {[2-(2-amino-ethoxy)-ethoxy]-acetyl}ₐ-(γ-Glu)_{b}-CO-(CH₂)_{c}-COOH; wherein: a is an integer of 1-3 (may be 1, 2, or 3); b is 1 or 2; c is an integer of 10-30 (may be 10, 11, 12, 13, 14, 15, 16, 17, 18, 19, 20, 21, 22, 23, 24, 25, 26, 27, 28, 29, or 30).

In some embodiments, X₁ is Tyr; X₂ is Aib; X₁₀ is Tyr; X₁₂ is Ile; X₁₃ is Tyr; X₁₄ is Y1; X₁₅ is Asp or Glu; X₁₆ is Arg or Lys; X₁₇ is Ile; X₁₈ is Ala; X₁₉ is Ala; X₂₀ is Gln; X₂₃ is Ile or Val; X₂₄ is Asn; X₂₇ is Ile or Leu; X₂₈ is Ala; X₂₉ is Gly; X₃₀ is Gly; Y1 is a Lys, Orn, Dap, Dab, or Cys residue with a side chain connected to a substituent of formula {[2-(2-amino-ethoxy)-ethoxy]-acetyl}ₐ-(γ-Glu)_{b}-CO-(CH₂)_{c}-COOH; a is an integer of 1-3; b is 1 or 2; c is an integer of 10-30.

In some embodiments, X₁ is Tyr; X₂ is Aib; X₁₀ is Tyr; X₁₂ is Ile; X₁₃ is Tyr; X₁₄ is Y1; X₁₅ is Asp or Glu; X₁₆ is Lys; X₁₇ is Ile; X₁₈ is Ala; X₁₉ is Ala; X₂₀ is Gln; X₂₃ is Val; X₂₄ is Asn; X₂₇ is Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(₇-Glu)_{b}-CO-(CH₂)_{c}-COOH; a is an integer of 1-3; b is 1 or 2; c is an integer of 10-30.

In some embodiments, X₁ is Tyr; X₂ is Aib or D-Ala; X₁₀ is Y1; X₁₂ is Ile; X₁₃ is Tyr; X₁₄ is Leu or Nle; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(γ-Glu)_{b}-CO-(CH₂)_{c}-COOH, wherein a is an integer of 1-3, b is 1 or 2, and c is an integer of 10-30.

In some embodiments, X₁ is Tyr; X₂ is Aib or D-Ala; X₁₀ is Tyr; X₁₂ is Y1; X₁₃ is Tyr; X₁₄ is Leu or Nle; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(γ-Glu)_{b}-CO-(CH₂)_{c}-COOH, wherein a is an integer of 1-3, b is 1 or 2, and c is an integer of 10-30.

In some embodiments, X₁ is Tyr; X₂ is Aib or D-Ala; X₁₀ is Tyr; X₁₂ is Ile; X₁₃ is Y1; X₁₄ is Leu or Nle; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(γ-Glu)_{b}-CO-(CH₂)_{c}-COOH, wherein a is an integer of 1-3, b is 1 or 2, and c is an integer of 10-30.

In some embodiments, X₁ is Tyr; X₂ is Aib or D-Ala; X₁₀ is Tyr; X₁₂ is Ile; X₁₃ is Tyr; X₁₄ is Y1; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(γ-Glu)_{b}-CO-(CH₂)_{c}-COOH, wherein a is an integer of 1-3, b is 1 or 2, and c is an integer of 10-30.

In some embodiments, X₁ is Tyr; X₂ is Aib; X₁₀ is Tyr; X₁₂ is Ile; X₁₃ is Tyr; X₁₄ is Y1; X₁₅ is Glu; X₁₆ is Arg or Lys; X₁₇ is Ile; X₁₈ is Ala; X₁₉ is Ala; X₂₀ is Gln; X₂₃ is Ile or Val; X₂₄ is Asn; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(₇-Glu)_{b}-CO-(CH₂)_{c}-COOH; a is an integer of 1-3; b is 1 or 2; c is an integer of 10-30.

In some embodiments, X₁ is Tyr; X₂ is Aib or D-Ala; X₁₀ is Tyr; X₁₂ is Ile; X₁₃ is Tyr; X₁₄ is Leu or Nle; X₁₅ is Glu; X₁₆ is Y1; X₁₇ is Ile; X₁₈ is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(γ-Glu)_{b}-CO-(CH₂)_{c}-COOH, wherein a is an integer of 1-3, b is 1 or 2, and c is an integer of 10-30.

In some embodiments, X₁ is Tyr; X₂ is Aib or D-Ala; X₁₀ is Tyr; X₁₂ is Ile; X₁₃ is Tyr; X₁₄ is Leu or Nle; X₁₅ is Glu; X₁₆ is Arg or Lys; X₁₇ is Y1; X₁₈ is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(γ-Glu)_{b}-CO-(CH₂)_{c}-COOH, wherein a is an integer of 1-3, b is 1 or 2, and c is an integer of 10-30.

In some embodiments, X₁₀, X₁₂, X₁₃, X₁₄, X₁₆, and X₁₇ are each independently selected from Y1, wherein Y1 is a Lys, Orn, Dap, Dab, or Cys residue with a substituent on a side chain, the substituent having a structure of formula {[2-(2-amino-ethoxy)-ethoxy]-acetyl}ₐ-(γ-Glu)_{b}-CO-(CH₂)_{c}-COOH; a is an integer of 1-3; b is 1 or 2; c is an integer of 10-30.

In some embodiments, a is 2, b is 1 or 2, and c is an integer of 16-20 (e.g., c is 16, 17, 18, 19, or 20).

In some embodiments, a is 2, b is 1 or 2, and c is 16, 18, or 20.

In some embodiments, X₁₀ is Y1; Y1 is Lys with a substituent on a side chain, the substituent having a structure of formula {[2-(2-amino-ethoxy)-ethoxy]-acetyl}ₐ-(γ-Glu)_{b}-CO-(CH₂)_{c}-COOH; a is 2; b is 1 or 2; c is 16 or 18.

In some embodiments, X₁₂ is Y1; Y1 is Lys with a substituent on a side chain, the substituent having a structure of formula {[2-(2-amino-ethoxy)-ethoxy]-acetyl}ₐ-(γ-Glu)_{b}-CO-(CH₂)_{c}-COOH; a is 2; b is 1 or 2; c is 16 or 18.

In some embodiments, X₁₃ is Y1; Y1 is Lys with a substituent on a side chain, the substituent having a structure of formula {[2-(2-amino-ethoxy)-ethoxy]-acetyl}ₐ-(γ-Glu)_{b}-CO-(CH₂)_{c}-COOH; a is 2; b is 1 or 2; c is 16 or 18.

In some embodiments, X₁₄ is Y1; Y1 is Lys with a substituent on a side chain, the substituent having a structure of formula {[2-(2-amino-ethoxy)-ethoxy]-acetyl}ₐ-(γ-Glu)_{b}-CO-(CH₂)_{c}-COOH; a is 2; b is 1 or 2; c is 16 or 18.

In some embodiments, X₁₆ is Y1; Y1 is Lys with a substituent on a side chain, the substituent having a structure of formula {[2-(2-amino-ethoxy)-ethoxy]-acetyl}ₐ-(γ-Glu)_{b}-CO-(CH₂)_{c}-COOH; a is 2; b is 1 or 2; c is 16 or 18.

In some embodiments, X₁₇ is Y1; Y1 is Lys with a substituent on a side chain, the substituent having a structure of formula {[2-(2-amino-ethoxy)-ethoxy]-acetyl}ₐ-(γ-Glu)_{b}-CO-(CH₂)_{c}-COOH; a is 2; b is 1 or 2; c is 16 or 18.

In some embodiments, the side chain amino of the Lys residue in Y1 is covalently connected to a substituent by formation of an amide bond.

In some embodiments, Y1 is K(-OEG-OEG-γGlu-C18-OH) or K(-OEG-OEG-yGlu-C20-OH), wherein K(-OEG-OEG-γGlu-C18-OH) has a structure shown below: 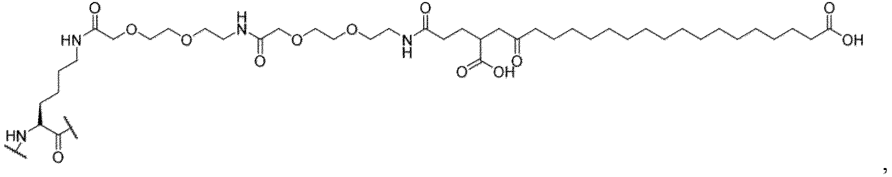 and K(-OEG-OEG-yGlu-C20-OH) has a structure shown below:

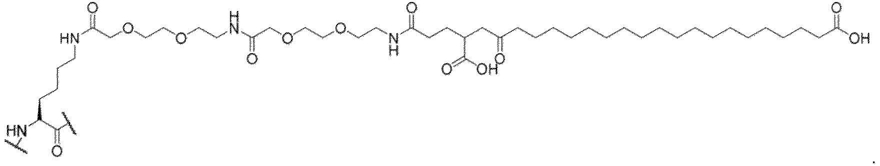

In some embodiments, Y1 is K(-OEG-OEG-γGlu-C18-OH) or K(-OEG-OEG-yGlu-C20-OH), wherein:
K(-OEG-OEG-γGlu-C18-OH) has a structure shown below: 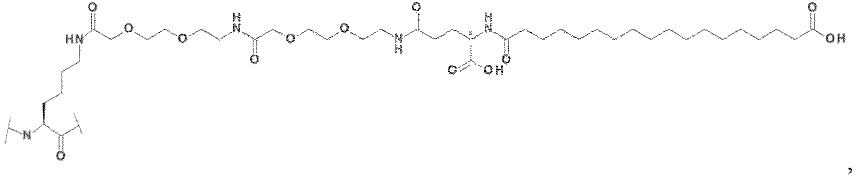 and
K(-OEG-OEG-yGlu-C20-OH) has a structure shown below:

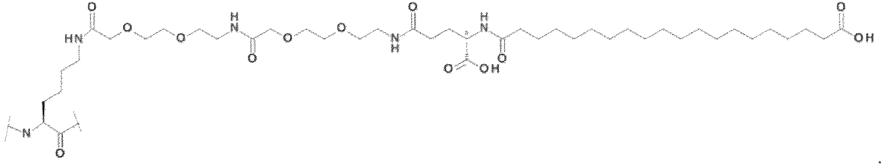

In some embodiments, in Y1, the ε amino of the Lys residue is covalently connected to a substituent by an amide bond, and the α amino of the Lys residue is connected to a peptide chain.

In some embodiments, X₁ is selected from Tyr; X₂ is selected from Aib; X₁₀ is selected from Tyr; X₁₂ is selected from Ile; X₁₃ is selected from Tyr; X₁₄ is selected from Y1; X₁₅ is selected from the group consisting of Asp and Glu; X₁₆ is selected from the group consisting of Arg and Lys; X₁₇ is selected from Ile; X₁₈ is selected from Ala; X₁₉ is selected from Ala; X₂₀ is selected from Gin; X₂₃ is selected from Ile or Val; X₂₄ is selected from Asn; X₂₇ is selected from the group consisting of Ile and Leu; X₂₈ is selected from Ala; X₂₉ is selected from Gly; X₃₀ is selected from Gly; Y1 is K(-OEG-OEG-yGlu-C18-OH) or K(-OEG-OEG-γGlu-C20-OH), wherein K(-OEG-OEG-γGlu-C18-OH) has a structure shown below: 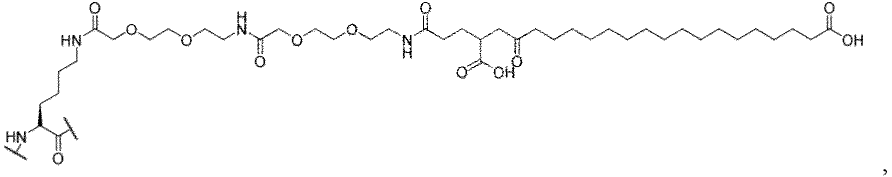 and K(-OEG-OEG-yGlu-C20-OH) has a structure shown below:

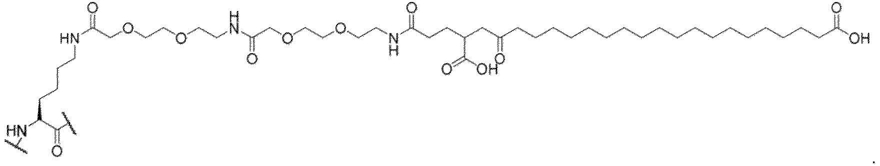

In some embodiments, X₁ is selected from Tyr; X₂ is selected from Aib; X₁₀ is selected from Tyr; X₁₂ is selected from Ile; X₁₃ is selected from Tyr; X₁₄ is selected from Y1; X₁₅ is selected from the group consisting of Asp and Glu; X₁₆ is selected from the group consisting of Arg and Lys; X₁₇ is selected from Ile; X₁₈ is selected from Ala; X₁₉ is selected from Ala; X₂₀ is selected from Gin; X₂₃ is selected from Ile or Val; X₂₄ is selected from Asn; X₂₇ is selected from the group consisting of Ile and Leu; X₂₈ is selected from Ala; X₂₉ is selected from Gly; X₃₀ is selected from Gly; Y1 is K(-OEG-OEG-γGlu-C18-OH) or K(-OEG-OEG-γGlu-C20-OH), wherein K(-OEG-OEG-γGlu-C18-OH) has a structure shown below: 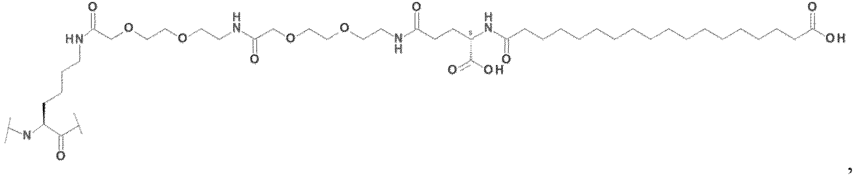 and
K(-OEG-OEG-yGlu-C20-OH) has a structure shown below:

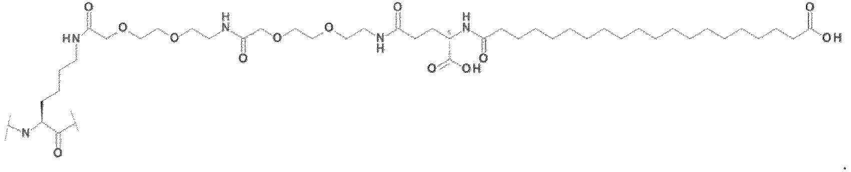

In some embodiments of the present disclosure, the GLP-1 analog is represented by the general formula (II) (SEQ ID NO: 20):

H-YAibEGTFTSDYSIYX₁₄X₁₅X₁₆IAAQEFX₂₃NWLX₂₇AGGPSSGAPPPS-NH₂ (II),

wherein X₁₄ is K or L, X₁₅ is D or E, X₁₆ is K or R, X₂₃ is V or I, and X₂₇ is I or L.

In some embodiments of the present disclosure, the GLP-1 analog is selected from the group consisting of the following compounds numbered 1 to 18:

| SEQ ID NO | Sequence |
|---|---|
| 1 | H-YAibEGTFTSDYSIYKDKIAAQEFVNWLIAGGPSSGAPPPS-NH₂ |
| 2 | H-YAibEGTFTSDYSIYKDRIAAQEFVNWLIAGGPSSGAPPPS-NH₂ |
| 3 | H-YAibEGTFTSDYSIYKDKIAAQEFINWLIAGGPSSGAPPPS-NH₂ |
| 4 | H-YAibEGTFTSDYSIYKDRIAAQEFINWLIAGGPSSGAPPPS-NH₂ |
| 5 | H-YAibEGTFTSDYSIYKDKIAAQEFINWLLAGGPSSGAPPPS-NH₂ |
| 6 | H-YAibEGTFTSDYSIYKDRIAAQEFVNWLLAGGPSSGAPPPS-NH₂ |
| 7 | H-YAibEGTFTSDYSIYKDKIAAQEFVNWLLAGGPSSGAPPPS-NH₂ |
| 8 | H-YAibEGTFTSDYSIYLEKIAAQEFVNWLLAGGPSSGAPPPS-NH₂ |
| 9 | H-YAibEGTFTSDYSIYLEKIAAQEFVNWLIAGGPSSGAPPPS-NH₂ |
| 10 | H-YAibEGTFTSDYSIYLEKIAAQEFINWLIAGGPSSGAPPPS-NH₂ |
| 11 | H-YAibEGTFTSDYSIYLEKIAAQEFINWLLAGGPSSGAPPPS-NH₂ |
| 12 | H-YAibEGTFTSDYSIYKEKIAAQEFVNWLIAGGPSSGAPPPS-NH₂ |
| 13 | H-YAibEGTFTSDYSIYKERIAAQEFVNWLIAGGPSSGAPPPS-NH₂ |
| 14 | H-YAibEGTFTSDYSIYKEKIAAQEFINWLIAGGPSSGAPPPS-NH₂ |
| 15 | H-YAibEGTFTSDYSIYKERIAAQEFINWLIAGGPSSGAPPPS-NH₂ |
| 16 | H-YAibEGTFTSDYSIYKEKIAAQEFINWLLAGGPSSGAPPPS-NH₂ |
| 17 | H-YAibEGTFTSDYSIYEERIAAQEFVNWLLAGGPSSGAPPPS-NH₂ |
| 18 | H-YAibEGTFTSDYSIYKEKIAAQEFVNWLLAGGPSSGAPPPS-NH₂. |

In some embodiments of the present disclosure, the GLP-1 analog is selected from the group consisting of the following compounds numbered 1# to 18#:

| No. | Sequence |
|---|---|
| 1# | H-YAibEGTFTSDYSIYK(OEG-OEG-γGlu-C20-OH)DKIAAQEFVNWLIAGGPSSGAPPPS- NH2 |
| 2# | H-YAibEGTFTSDYSIYK(OEG-OEG-γGlu-C20-OH)DRIAAQEFVNWLIAGGPSSGAPPPS- NH2 |
| 3# | H-YAibEGTFTSDYSIYK(OEG-OEG-γGlu-C20-OH)DKIAAQEFINWLIAGGPSSGAPPPS- NH2 |
| 4# | H-YAibEGTFTSDYSIYK(OEG-OEG-γGlu-C20-OH)DRIAAQEFINWLIAGGPSSGAPPPS- NH2 |
| 5# | H-YAibEGTFTSDYSIYK(OEG-OEG-γGlu-C20-OH)DKIAAQEFINWLLAGGPSSGAPPPS- NH2 |
| 6# | H-YAibEGTFTSDYSIYK(OEG-OEG-γGlu-C20-OH)DRIAAQEFVNWLLAGGPSSGAPPPS- NH2 |
| 7# | H-YAibEGTFTSDYSIYK(OEG-OEG-γGlu-C20-OH)DKIAAQEFVNWLLAGGPSSGAPPPS- NH2 |
| 8# | H-YAibEGTFTSDYSIYLEK(OEG-OEG-γGlu-C20-OH)IAAQEFVNWLLAGGPSSGAPPPS- NH2 |
| 9# | H-YAibEGTFTSDYSIYLEK(OEG-OEG-γGlu-C20-OH)IAAQEFVNWLIAGGPSSGAPPPS- NH2 |
| 10# | H-YAibEGTFTSDYSIYLEK(OEG-OEG-γGlu-C20-OH)IAAQEFINWLIAGGPSSGAPPPS- NH2 |
| 11# | H-YAibEGTFTSDYSIYLEK(OEG-OEG-γGlu-C20-OH)IAAQEFINWLLAGGPSSGAPPPS- NH2 |
| 12# | H-YAibEGTFTSDYSIYK(OEG-OEG-γGlu-C20-OH)EKIAAQEFVNWLIAGGPSSGAPPPS- NH2 |
| 13# | H-YAibEGTFTSDYSIYK(OEG-OEG-γGlu-C20-OH)ERIAAQEFVNWLIAGGPSSGAPPPS- NH2 |
| 14# | H-YAibEGTFTSDYSIYK(OEG-OEG-γGlu-C20-OH)EKIAAQEFINWLIAGGPSSGAPPPS- NH2 |
| 15# | H-YAibEGTFTSDYSIYK(OEG-OEG-γGlu-C20-OH)ERIAAQEFINWLIAGGPSSGAPPPS- NH2 |
| 16# | H-YAibEGTFTSDYSIYK(OEG-OEG-γGlu-C20-OH)EKIAAQEFINWLLAGGPSSGAPPPS- NH2 |
| 17# | H-YAibEGTFTSDYSIYK(OEG-OEG-γGlu-C20-OH)ERIAAQEFVNWLLAGGPSSGAPPPS- NH2 |
| 18# | H-YAibEGTFTSDYSIYK(OEG-OEG-γGlu-C20-OH)EKIAAQEFVNWLLAGGPSSGAPPPS- NH2. |

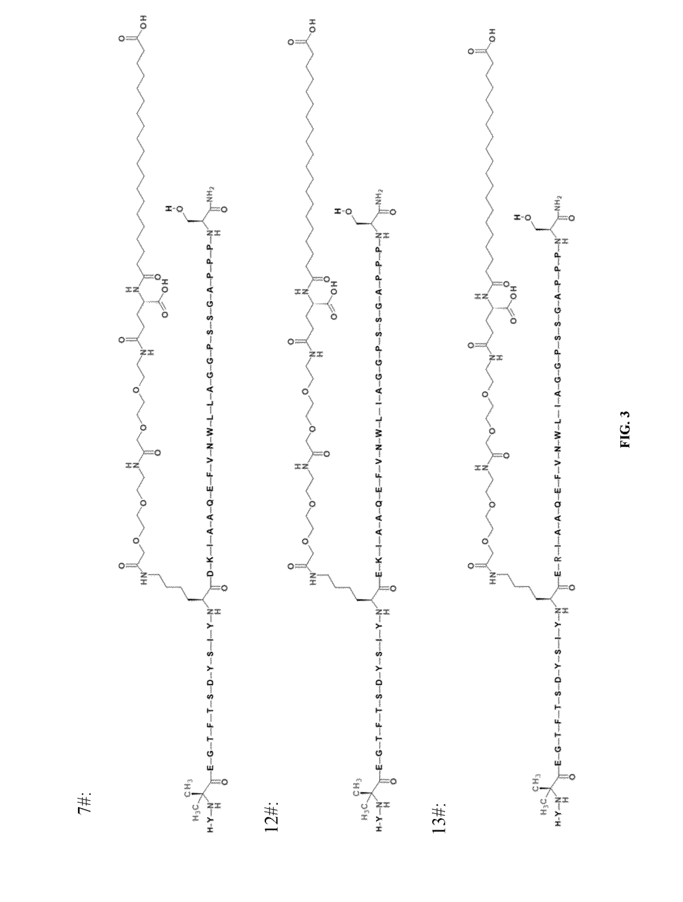

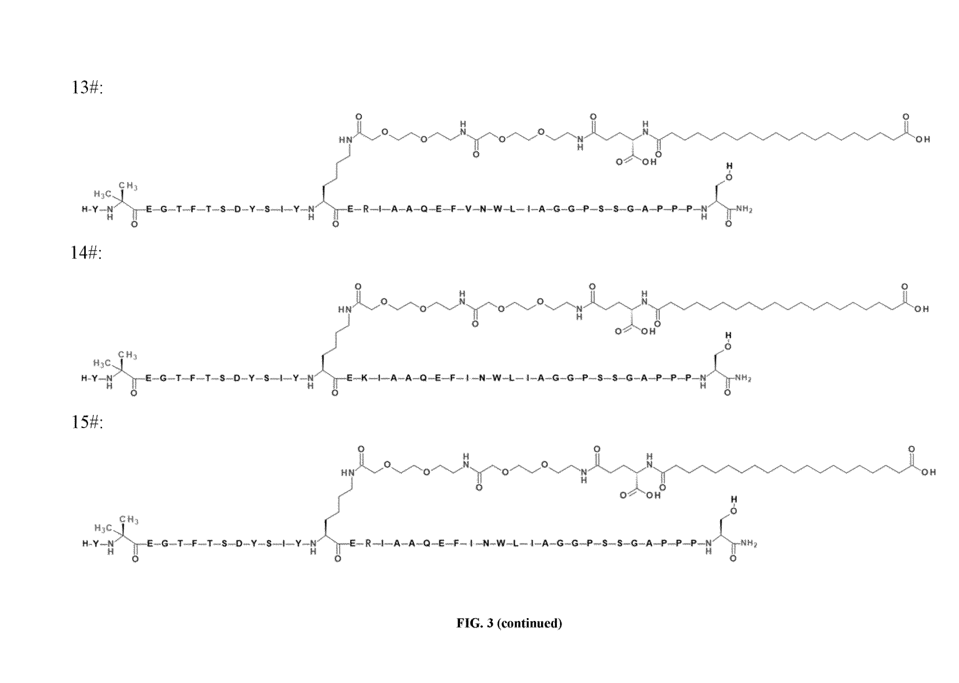

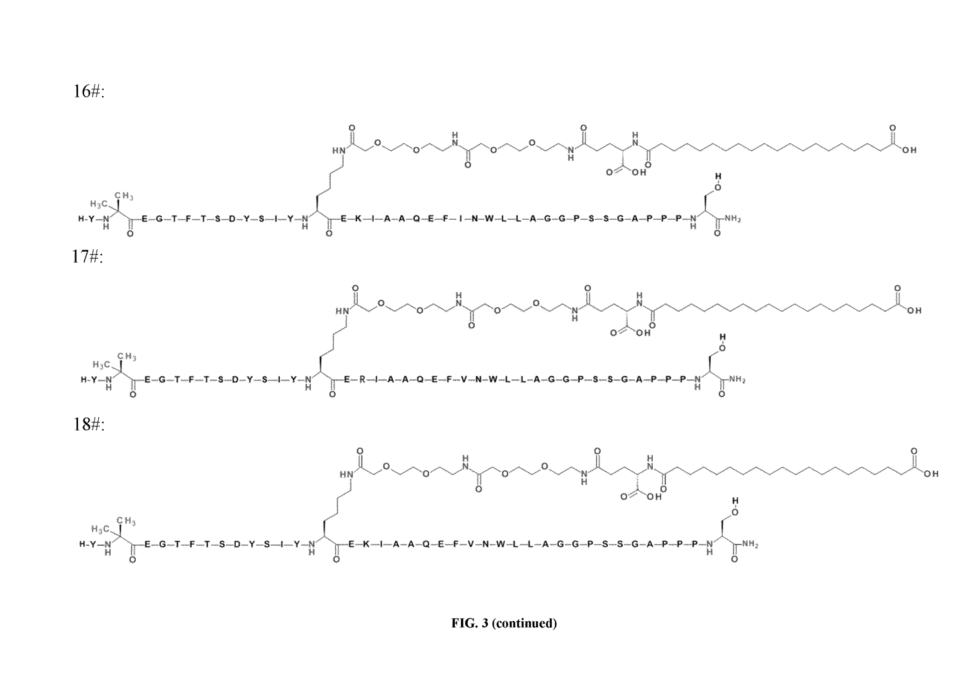

In some embodiments, the GLP-1 analog of the present disclosure is selected from the group consisting of compounds shown as 7#, 12#, 13#, 14#, 15#, 16#, 17#, and 18 # in FIG. 3.

In some embodiments, the GLP-1 analog of the present disclosure is an amphoteric compound, and pharmaceutically acceptable salts thereof include salts formed by reacting an acidic or alkaline compound therewith. In some embodiments, the acidic compound is selected from the group consisting of hydrochloric acid, hydrobromic acid, hydroiodic acid, sulfuric acid, phosphoric acid, *p*-toluenesulfonic acid, methanesulfonic acid, oxalic acid, *p*-bromophenylsulfonic acid, carbonic acid, succinic acid, citric acid, benzoic acid, and acetic acid. The alkaline compound is selected from the group consisting of ammonium, alkali or alkaline earth metal hydroxides, carbonate, and bicarbonate, such as sodium hydroxide, potassium hydroxide, ammonium hydroxide, sodium carbonate, and potassium carbonate.

In some embodiments, the C-terminus of the GLP-1 analog has a carboxyl protecting group. In some embodiments, an amide or an ester is formed at the C-terminus of the GLP-1 analog. In some embodiments, the pharmaceutical composition of the present disclosure is selected from any one of the following compositions:
a composition (a) comprising the compound shown as 18# in FIG. 3 or a pharmaceutically acceptable salt, amide or ester thereof and *N-*(8-(2-hydroxybenzoyl)amino)caprylate;
a composition (b) comprising the compound shown as 18# in FIG. 3 or a pharmaceutically acceptable salt, amide or ester thereof, *N-*(8-(2-hydroxybenzoyl)amino)caprylate, and sodium caprylate;
a composition (c) comprising the compound shown as 18# in FIG. 3 or a pharmaceutically acceptable salt, amide or ester thereof, *N-*(8-(2-hydroxybenzoyl)amino)caprylate, and an amino acid or a salt thereof (such as one or more of histidine, arginine, leucine and lysine or salts thereof);
a composition (d) comprising the compound shown as 18# in FIG. 3 or a pharmaceutically acceptable salt, amide or ester thereof, *N-*(8-(2-hydroxybenzoyl)amino)caprylate, and β-cyclodextrin;
a composition (e) comprising the compound shown as 18# in FIG. 3 or a pharmaceutically acceptable salt, amide or ester thereof, and sodium caprate;
a composition (f) comprising the compound shown as 18# in FIG. 3 or a pharmaceutically acceptable salt, amide or ester thereof, *N-*(8-(2-hydroxybenzoyl)amino)caprylate, arginine or a salt thereof, and sodium caprate;
a composition (g) comprising the compound shown as 18# in FIG. 3 or a pharmaceutically acceptable salt, amide or ester thereof, *N-*(8-(2-hydroxybenzoyl)amino)caprylate, and histidine or a salt thereof;
a composition (h) comprising the compound shown as 18# in FIG. 3 or a pharmaceutically acceptable salt, amide or ester thereof, *N-*(8-(2-hydroxybenzoyl)amino)caprylate, and arginine or a salt thereof;
a composition (i) comprising the compound shown as 18# in FIG. 3 or a pharmaceutically acceptable salt, amide or ester thereof, *N-*(8-(2-hydroxybenzoyl)amino)caprylate, arginine or a salt thereof, and leucine or a salt thereof;
a composition (j) comprising the compound shown as 18# in FIG. 3 or a pharmaceutically acceptable salt, amide or ester thereof, *N-*(8-(2-hydroxybenzoyl)amino)caprylate, arginine or a salt thereof, and lysine or a salt thereof; and
a composition (k) comprising the compound shown as 18# in FIG. 3 or a pharmaceutically acceptable salt, amide or ester thereof, *N-*(8-(2-hydroxybenzoyl)amino)caprylate, arginine or a salt thereof, and sodium caprylate.

In some embodiments, the pharmaceutical composition of the present disclosure is selected from any one of the following compositions:
a composition (a1) comprising, relative to the total mass of the composition, 1%-20% of the compound shown as 18# in FIG. 3 or a pharmaceutically acceptable salt, amide or ester thereof and at least 50% of *N-*(8-(2-hydroxybenzoyl)amino)caprylate;
a composition (b 1) comprising, relative to the total mass of the composition, 1%-20% of the compound shown as 18# in FIG. 3 or a pharmaceutically acceptable salt, amide or ester thereof, 20%-90% of *N*-(8-(2-hydroxybenzoyl)amino)caprylate, and 5%-75% of sodium caprylate;
a composition (c1) comprising, relative to the total mass of the composition, 1%-20% of the compound shown as 18# in FIG. 3 or a pharmaceutically acceptable salt, amide or ester thereof, 20%-90% of *N-*(8-(2-hydroxybenzoyl)amino)caprylate, and 5%-75% of an amino acid (e.g., one or more of histidine, arginine, leucine and lysine or salts thereof);
a composition (d1) comprising, relative to the total mass of the composition, 1%-20% of the compound shown as 18# in FIG. 3 or a pharmaceutically acceptable salt, amide or ester thereof, 20%-90% of *N-*(8-(2-hydroxybenzoyl)amino)caprylate, and 5%-75% of β-cyclodextrin;
a composition (e1) comprising, relative to the total mass of the composition, 1%-20% of the compound shown as 18# in FIG. 3 or a pharmaceutically acceptable salt, amide or ester thereof and at least 50% of sodium caprate;
a composition (f1) comprising, relative to the total mass of the composition, 1%-20% of the compound shown as 18# in FIG. 3 or a pharmaceutically acceptable salt, amide or ester thereof, 20%-90% of *N-*(8-(2-hydroxybenzoyl)amino)caprylate, 5%-60% of arginine or a salt thereof, and 1%-50% of sodium caprate;
a composition (g1) comprising, relative to the total mass of the composition, 1%-20% of the compound shown as 18# in FIG. 3 or a pharmaceutically acceptable salt, amide or ester thereof, 20%-90% of *N-*(8-(2-hydroxybenzoyl)amino)caprylate, and 5%-75% of arginine or a salt thereof;
a composition (h1) comprising, relative to the total mass of the composition, 1%-20% of the compound shown as 18# in FIG. 3 or a pharmaceutically acceptable salt, amide or ester thereof, 20%-90% of *N-*(8-(2-hydroxybenzoyl)amino)caprylate, 1%-50% of arginine or a salt thereof, and 1%-50% of leucine or a salt thereof;
a composition (i1) comprising, relative to the total mass of the composition, 1%-20% of the compound shown as 18# in FIG. 3 or a pharmaceutically acceptable salt, amide or ester thereof, 20%-90% of *N-*(8-(2-hydroxybenzoyl)amino)caprylate, 1%-50% of arginine or a salt thereof, and 1%-50% of lysine or a salt thereof; and
a composition (j 1) comprising, relative to the total mass of the composition, 1%-20% of the compound shown as 18# in FIG. 3 or a pharmaceutically acceptable salt, amide or ester thereof, 20%-90% of *N-*(8-(2-hydroxybenzoyl)amino)caprylate, 1%-50% of arginine or a salt thereof, and 1%-50% of sodium caprylate.

In some embodiments, the pharmaceutical composition of the present disclosure is selected from any one of the following compositions:
a composition (a2) comprising, relative to the total mass of the composition, 1%-5% of the compound shown as 18# in FIG. 3 or a pharmaceutically acceptable salt, amide or ester thereof and at least 70%, e.g., at least 90%, of *N*-(8-(2-hydroxybenzoyl)amino)caprylate;
a composition (b2) comprising, relative to the total mass of the composition, 1%-20% of the compound shown as 18# in FIG. 3 or a pharmaceutically acceptable salt, amide or ester thereof, 70%-90% of *N-*(8-(2-hydroxybenzoyl)amino)caprylate, and 5%-10% of sodium caprylate;
a composition (b3) comprising, relative to the total mass of the composition, 1%-20% of the compound shown as 18# in FIG. 3 or a pharmaceutically acceptable salt, amide or ester thereof, 50%-70% or 50%-85% of *N-*(8-(2-hydroxybenzoyl)amino)caprylate, and 10%-45% of sodium caprylate;
a composition (b4) comprising, relative to the total mass of the composition, 1%-20% of the compound shown as 18# in FIG. 3 or a pharmaceutically acceptable salt, amide or ester thereof, 20%-50% of *N-*(8-(2-hydroxybenzoyl)amino)caprylate, and 10%-75% or 40%-75% of sodium caprylate;
a composition (c2) comprising, relative to the total mass of the composition, 1%-20% of the compound shown as 18# in FIG. 3 or a pharmaceutically acceptable salt, amide or ester thereof, 70%-90% of *N-*(8-(2-hydroxybenzoyl)amino)caprylate, and 5%-10% or 1%-20% of arginine or a salt thereof;
a composition (c3) comprising, relative to the total mass of the composition, 1%-20% of the compound shown as 18# in FIG. 3 or a pharmaceutically acceptable salt, amide or ester thereof, 50%-70% or 50%-85% of *N-*(8-(2-hydroxybenzoyl)amino)caprylate, and 10%-45% of arginine or a salt thereof;
a composition (c4) comprising, relative to the total mass of the composition, 1%-20% of the compound shown as 18# in FIG. 3 or a pharmaceutically acceptable salt, amide or ester thereof, 20%-50% of *N-*(8-(2-hydroxybenzoyl)amino)caprylate, and 10%-75% or 40%-75% of arginine or a salt thereof;
a composition (c5) comprising, relative to the total mass of the composition, 1%-20% of the compound shown as 18# in FIG. 3 or a pharmaceutically acceptable salt, amide or ester thereof, 70%-90% of *N-*(8-(2-hydroxybenzoyl)amino)caprylate, and 5%-25% of arginine or a salt thereof;
a composition (d2) comprising, relative to the total mass of the composition, 1%-20% of the compound shown as 18# in FIG. 3 or a pharmaceutically acceptable salt, amide or ester thereof, 70%-90% of *N-*(8-(2-hydroxybenzoyl)amino)caprylate, and 5%-10% or 1%-20% of β-cyclodextrin;
a composition (d3) comprising, relative to the total mass of the composition, 1%-20% of the compound shown as 18# in FIG. 3 or a pharmaceutically acceptable salt, amide or ester thereof, 50%-70% or 50%-85% of *N-*(8-(2-hydroxybenzoyl)amino)caprylate, and 10%-45% of β-cyclodextrin;
a composition (d4) comprising, relative to the total mass of the composition, 1%-20% of the compound shown as 18# in FIG. 3 or a pharmaceutically acceptable salt, amide or ester thereof, 20%-50% of *N-*(8-(2-hydroxybenzoyl)amino)caprylate, and 10%-75% or 40%-75% of β-cyclodextrin; and
a composition (e2) comprising, relative to the total mass of the composition, 1%-5% of the compound shown as 18# in FIG. 3 or a pharmaceutically acceptable salt, amide or ester thereof and 70%-80% of sodium caprate;
a composition (f1) comprising, relative to the total mass of the composition, 1%-20% of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, and 1%-30% of sodium caprate;
a composition (f2) comprising, relative to the total mass of the composition, 1%-20% of the compound shown as 18# in FIG. 3 or a pharmaceutically acceptable salt, amide or ester thereof, 20%-50% of *N-*(8-(2-hydroxybenzoyl)amino)caprylate, 10%-35% of arginine or a salt thereof, and 15%-40% of sodium caprate;
a composition (f3) comprising, relative to the total mass of the composition, 1%-20% of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, and 10%-35% of sodium caprate;
a composition (g1) comprising, relative to the total mass of the composition, 1%-20% of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, and 1%-30% of leucine or a salt thereof;
a composition (g2) comprising, relative to the total mass of the composition, 1%-20% of the compound shown as 18# in FIG. 3 or a pharmaceutically acceptable salt, amide or ester thereof, 20%-50% of *N-*(8-(2-hydroxybenzoyl)amino)caprylate, 10%-35% of arginine or a salt thereof, and 15%-40% of leucine or a salt thereof;
a composition (g3) comprising, relative to the total mass of the composition, 1%-20% of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, and 10%-35% of leucine or a salt thereof;
a composition (h1) comprising, relative to the total mass of the composition, 1%-20% of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, and 1%-30% of lysine or a salt thereof;
a composition (h2) comprising, relative to the total mass of the composition, 1%-20% of the compound shown as 18# in FIG. 3 or a pharmaceutically acceptable salt, amide or ester thereof, 20%-50% of *N-*(8-(2-hydroxybenzoyl)amino)caprylate, 10%-35% of arginine or a salt thereof, and 15%-40% of lysine or a salt thereof;
a composition (h3) comprising, relative to the total mass of the composition, 1%-20% of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, and 10%-35% of lysine or a salt thereof;
a composition (i1) comprising, relative to the total mass of the composition, 1%-20% of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, and 1%-30% of sodium caprylate;
a composition (i2) comprising, relative to the total mass of the composition, 1%-20% of the compound shown as 18# in FIG. 3 or a pharmaceutically acceptable salt, amide or ester thereof, 20%-50% of *N-*(8-(2-hydroxybenzoyl)amino)caprylate, 15%-40% of arginine or a salt thereof, and 10%-35% of sodium caprylate; and
a composition (i3) comprising, relative to the total mass of the composition, 1%-20% of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, and 10%-35% of sodium caprylate.

In some embodiments, the pharmaceutical composition of the present disclosure further comprises one or more excipients selected from the group consisting of a filler, a binder, a disintegrant, a lubricant, a glidant, a coating agent, and a protease inhibitor.

In some embodiments, the filler does not necessarily function as a filler in the composition of the present disclosure; the binder does not necessarily function as a binder in the composition of the present disclosure; the disintegrant does not necessarily function as a disintegrant in the composition of the present disclosure; the lubricant does not necessarily function as a lubricant in the composition of the present disclosure; a glidant does not necessarily function as a glidant in the composition of the present disclosure; the coating agent does not necessarily function as a coating agent in the composition of the present disclosure; the protease inhibitor does not necessarily act as a protease inhibitor in the composition.

In some embodiments, the filler includes, but is not limited to, microcrystalline cellulose, mannitol, lactose, pregelatinized starch, calcium hydrogen phosphate, sorbitol, or any mixtures thereof.

In some embodiments, the binder includes, but is not limited to, povidone and a homolog thereof, hydroxypropyl cellulose, hydroxypropyl methyl cellulose and a homolog thereof, or any mixtures thereof.

In some embodiments, the disintegrant includes, but is not limited to, microcrystalline cellulose, alginic acid, alginate, polacrilin potassium, calcium hydrogen phosphate, corn starch, pregelatinized starch, sodium carboxymethyl starch, sodium starch glycolate, crospovidone, cellulose powder, and cellulose derivatives, such as hydroxymethyl cellulose, hydroxyethyl cellulose, hydroxypropyl cellulose, hydroxypropyl methylcellulose, and croscarmellose sodium.

In some embodiments, the lubricant includes, but is not limited to, magnesium stearate, glyceryl behenate, sodium stearyl fumarate, stearic acid, palmitic acid, calcium stearate, talc, carnauba wax, or any mixtures thereof.

In some embodiments, the glidant is selected from the group consisting of colloidal silicon dioxide and talc.

In some embodiments, the coating agent includes, but is not limited to, water-soluble polymers, water-insoluble polymers, gastro-soluble polymers, and enteric polymers. Water-soluble polymers include natural polymers or polysaccharides and derivatives thereof, such as gum arabic powder, gelatin, pullulan, dextrin, sodium carboxymethyl starch, and sodium alginate; cellulose derivatives, such as sodium carboxymethylcellulose, calcium carboxymethylcellulose, hydroxypropyl cellulose, hydroxypropyl methyl cellulose, hydroxyethyl cellulose, hydroxymethyl cellulose, methyl cellulose, and carboxymethylcellulose; and water-soluble vinyl derivatives, such as polyvinylpyrrolidone and polyvinyl alcohol. Water-insoluble polymers include, e.g., ethyl cellulose (an aqueous ethyl cellulose dispersion (e.g., trade name: AQUACOAT, manufactured by FMC), a vinyl acetate polymer (e.g., trade name: Kollicoat SR30D, manufactured by BASF), an aminoalkyl methacrylate copolymer (particularly an aqueous dispersion thereof (AmmonioMethacrylateCopolymerDispersion) (e.g., trade names: EUDRAGITRL30D and EUDRAGITRS30D, manufactured by EVONIC)), and an ethyl acrylate-methyl methacrylate copolymer dispersion (e.g., trade name: EUDRAGITNE30D, manufactured by EVONIC). Gastro-soluble polymers include aminoacetal compounds such as polyvinyl acetal diethylamino acetate (e.g., trade name: AEA, manufactured by Mitsubishi-KagakuFoods Corporation), an aminoalkyl methacrylate copolymer E (e.g., trade name: EUDRAGITE, manufactured by EVONIC), and a mixture thereof. Enteric polymers include enteric cellulose esters, such as cellulose acetate propionate, hydroxypropyl methyl cellulose acetate succinate, hydroxypropyl methyl cellulose phthalate (hypromellose phthalate), hydroxymethyl ethyl cellulose phthalate, carboxymethylethyl cellulose, cellulose acetate, and phthalate; and enteric acrylic acid copolymers, such as a methacrylic acid copolymer LD (e.g., trade name: EUDRAGITL30D-55, manufactured by EVONIC; trade name: POLYQUIDPA30, manufactured by Sanyo Chemical Industries, Ltd.; trade name: KollicoatMAE30DP, manufactured by BASF; trade name: Acryl-Eze, batch No.: 93018508, manufactured by Colorcon), a methacrylic acid copolymer L (e.g., trade name: EUDRAGITL, manufactured by EVONIC), and a methacrylic acid copolymer S (e.g., trade names: EUDRAGITS100 and EUDRAGITFS30D, manufactured by EVONIC).

In some embodiments, the coating agent is selected from one or more of sodium carboxymethylcellulose, calcium carboxymethylcellulose, hydroxypropyl cellulose, hydroxypropyl methyl cellulose, hydroxyethyl cellulose, hydroxymethyl cellulose, methyl cellulose, carboxymethyl cellulose, polyvinyl alcohol, methacrylic acid resin copolymer, and ethyl cellulose.

In some embodiments, the protease inhibitor includes, but is not limited to, phenylmethylsulfonyl fluoride (PSMF), soybean trypsin inhibitor, soybean trypsin-chymotrypsin inhibitor, colicin, sunflower trypsin inhibitor, leupeptin, citric acid, ethylenediaminetetraacetic acid, sodium glycocholate, and 4-(2-aminoethyl)benzenesulfonyl fluoride hydrochloride.

In some embodiments, the composition comprises, relative to the total mass of the composition, 10%-30% or 15%-45% of a filler, 1%-5% of a binder, 0.5%-5% of a glidant, 0.5%-5% of a lubricant, and/or 10%-30% of a protease inhibitor.

In some embodiments, the pharmaceutical composition of the present disclosure is selected from any one of the following compositions:
a composition (i) comprising the compound shown as 18# in FIG. 3 or a pharmaceutically acceptable salt, amide or ester thereof, *N*-(8-(2-hydroxybenzoyl)amino)caprylate, and one or more of a filler, a binder, and a lubricant;
a composition (ii) comprising the compound shown as 18# in FIG. 3 or a pharmaceutically acceptable salt, amide or ester thereof, *N*-(8-(2-hydroxybenzoyl)amino)caprylate, sodium caprylate, a glidant, and a lubricant;
a composition (iii) comprising the compound shown as 18# in FIG. 3 or a pharmaceutically acceptable salt, amide or ester thereof, *N*-(8-(2-hydroxybenzoyl)amino)caprylate, an amino acid (e.g., one or more of histidine, arginine, leucine and lysine or salts thereof, such as arginine or a salt thereof), a glidant, and a lubricant;
a composition (iv) comprising the compound shown as 18# in FIG. 3 or a pharmaceutically acceptable salt, amide or ester thereof, *N-*(8-(2-hydroxybenzoyl)amino)caprylate, β-cyclodextrin, a glidant, and a lubricant;
a composition (v) comprising the compound shown as 18# in FIG. 3 or a pharmaceutically acceptable salt, amide or ester thereof, sodium caprate, and a protease inhibitor;
a composition (vi) comprising the compound shown as 18# in FIG. 3 or a pharmaceutically acceptable salt, amide or ester thereof, *N-*(8-(2-hydroxybenzoyl)amino)caprylate, arginine or a salt thereof, sodium caprate, a glidant, and a lubricant;
a composition (vii) comprising the compound shown as 18# in FIG. 3 or a pharmaceutically acceptable salt, amide or ester thereof, *N-*(8-(2-hydroxybenzoyl)amino)caprylate, arginine or a salt thereof, leucine or a salt thereof, a glidant, and a lubricant;
a composition (viii) comprising the compound shown as 18# in FIG. 3 or a pharmaceutically acceptable salt, amide or ester thereof, *N-*(8-(2-hydroxybenzoyl)amino)caprylate, arginine or a salt thereof, lysine or a salt thereof, a glidant, and a lubricant;
a composition (ix) comprising the compound shown as 18# in FIG. 3 or a pharmaceutically acceptable salt, amide or ester thereof, *N-*(8-(2-hydroxybenzoyl)amino)caprylate, arginine or a salt thereof, a glidant, a lubricant, a filler, and/or a binder;
a composition (x) comprising the compound shown as 18# in FIG. 3 or a pharmaceutically acceptable salt, amide or ester thereof, *N-*(8-(2-hydroxybenzoyl)amino)caprylate, arginine or a salt thereof, sodium caprylate, a glidant, and a lubricant.

In some embodiments, the pharmaceutical composition of the present disclosure is selected from any one of the following compositions:
a composition (i-1) comprising the compound shown as 18# in FIG. 3 or a pharmaceutically acceptable salt, amide or ester thereof, *N-*(8-(2-hydroxybenzoyl)amino)caprylate, and one or more of microcrystalline cellulose, povidone, and magnesium stearate;
a composition (ii-1) comprising the compound shown as 18# in FIG. 3 or a pharmaceutically acceptable salt, amide or ester thereof, *N-*(8-(2-hydroxybenzoyl)amino)caprylate, sodium caprylate, colloidal silicon dioxide, and magnesium stearate;
a composition (iii-1) comprising the compound shown as 18# in FIG. 3 or a pharmaceutically acceptable salt, amide or ester thereof, *N-*(8-(2-hydroxybenzoyl)amino)caprylate, an amino acid (e.g., one or more of histidine, arginine, leucine and lysine or salts thereof, such as arginine or a salt thereof), colloidal silicon dioxide, and magnesium stearate;
a composition (iv-1) comprising the compound shown as 18# in FIG. 3 or a pharmaceutically acceptable salt, amide or ester thereof, *N-*(8-(2-hydroxybenzoyl)amino)caprylate, β-cyclodextrin, colloidal silicon dioxide, and magnesium stearate;
a composition (v-1) comprising the compound shown as 18# in FIG. 3 or a pharmaceutically acceptable salt, amide or ester thereof, sodium caprate, and PMSF;
a composition (vi-1) comprising the compound shown as 18# in FIG. 3 or a pharmaceutically acceptable salt, amide or ester thereof, *N-*(8-(2-hydroxybenzoyl)amino)caprylate, arginine or a salt thereof, sodium caprate, colloidal silicon dioxide, and magnesium stearate;
a composition (vii-1) comprising the compound shown as 18# in FIG. 3 or a pharmaceutically acceptable salt, amide or ester thereof, *N-*(8-(2-hydroxybenzoyl)amino)caprylate, arginine or a salt thereof, leucine or a salt thereof, colloidal silicon dioxide, and magnesium stearate;
a composition (viii-1) comprising the compound shown as 18# in FIG. 3 or a pharmaceutically acceptable salt, amide or ester thereof, *N-*(8-(2-hydroxybenzoyl)amino)caprylate, arginine or a salt thereof, lysine or a salt thereof, colloidal silicon dioxide, and magnesium stearate;
a composition (ix-1) comprising the compound shown as 18# in FIG. 3 or a pharmaceutically acceptable salt, amide or ester thereof, *N-*(8-(2-hydroxybenzoyl)amino)caprylate, arginine or a salt thereof, sorbitol, colloidal silicon dioxide, and magnesium stearate;
a composition (ix-2) comprising the compound shown as 18# in FIG. 3 or a pharmaceutically acceptable salt, amide or ester thereof, *N-*(8-(2-hydroxybenzoyl)amino)caprylate, arginine or a salt thereof, sorbitol, povidone, colloidal silicon dioxide, and magnesium stearate;
a composition (ix-3) comprising the compound shown as 18# in FIG. 3 or a pharmaceutically acceptable salt, amide or ester thereof, *N-*(8-(2-hydroxybenzoyl)amino)caprylate, arginine or a salt thereof, sorbitol, hydroxypropyl cellulose, colloidal silicon dioxide, and magnesium stearate; and
a composition (x-1) comprising the compound shown as 18# in FIG. 3 or a pharmaceutically acceptable salt, amide or ester thereof, *N-*(8-(2-hydroxybenzoyl)amino)caprylate, arginine or a salt thereof, sodium caprylate, colloidal silicon dioxide, and magnesium stearate.

In some embodiments, the pharmaceutical composition of the present disclosure is selected from any one of the following compositions:
a composition (i-2) comprising, relative to the total mass of the composition, 1%-5% of the compound shown as 18# in FIG. 3 or a pharmaceutically acceptable salt, amide or ester thereof, 70%-80% of *N-*(8-(2-hydroxybenzoyl)amino)caprylate, about 20% of a filler, about 2% of a binder, and 0.5%-5% of a lubricant;
a composition (i-3) comprising, relative to the total mass of the composition, 1%-5% of the compound shown as 18# in FIG. 3 or a pharmaceutically acceptable salt, amide or ester thereof, at least 90% of *N-*(8-(2-hydroxybenzoyl)amino)caprylate, and 0.5%-5% of a lubricant;
a composition (ii-2) comprising, relative to the total mass of the composition, 1%-20%, e.g., 1%-5%, of the compound shown as 18# in FIG. 3 or a pharmaceutically acceptable salt, amide or ester thereof, 70%-90% of N-(8-(2-hydroxybenzoyl)amino)caprylate, 5%-10% of sodium caprylate, 1%-1.5% of a glidant, and 0.5%-5% of a lubricant;
a composition (ii-3) comprising, relative to the total mass of the composition, 1%-20%, e.g., 1%-5%, of the compound shown as 18# in FIG. 3 or a pharmaceutically acceptable salt, amide or ester thereof, 50%-70% or 50%-85% of *N-*(8-(2-hydroxybenzoyl)amino)caprylate, 10%-45% of sodium caprylate, 1%-1.5% of a glidant, and 0.5%-5% of a lubricant;
a composition (ii-4) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-75% or 40%-75% of sodium caprylate, 1%-1.5% of a glidant, and 0.5%-5% of a lubricant; a composition (iii-2) comprising, relative to the total mass of the composition, 1%-20%, e.g., 1%-5%, of the compound shown as 18# in FIG. 3 or a pharmaceutically acceptable salt, amide or ester thereof, 70%-90% of *N-*(8-(2-hydroxybenzoyl)amino)caprylate, 5%-10% or 1%-20% of arginine, 1%-1.5% of a glidant, and 0.5%-5% of a lubricant;
a composition (iii-3) comprising, relative to the total mass of the composition, 1%-20%, e.g., 1%-5%, of the compound shown as 18# in FIG. 3 or a pharmaceutically acceptable salt, amide or ester thereof, 50%-70% or 50%-85% of *N-*(8-(2-hydroxybenzoyl)amino)caprylate, 10%-45% of arginine, 1%-1.5% of a glidant, and 0.5%-5% of a lubricant;
a composition (iii-4) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-75% or 40%-75% of arginine, 1%-1.5% of a glidant, and 0.5%-5% of a lubricant;
a composition (iii-5) comprising, relative to the total mass of the composition, 1%-20%, e.g., 1%-5%, of the compound shown as 18# in FIG. 3 or a pharmaceutically acceptable salt, amide or ester thereof, 70%-90% of *N-*(8-(2-hydroxybenzoyl)amino)caprylate, 5%-25% of arginine, 1%-1.5% of a glidant, and 0.5%-5% of a lubricant;
a composition (iv-2) comprising, relative to the total mass of the composition, 1%-20%, e.g., 1%-5%, of the compound shown as 18# in FIG. 3 or a pharmaceutically acceptable salt, amide or ester thereof, 70%-90% of *N-*(8-(2-hydroxybenzoyl)amino)caprylate, 5%-10% or 1%-20% of β-cyclodextrin, 1%-1.5% of a glidant, and 0.5%-5% of a lubricant;
a composition (iv-3) comprising, relative to the total mass of the composition, 1%-20%, e.g., 1%-5%, of the compound shown as 18# in FIG. 3 or a pharmaceutically acceptable salt, amide or ester thereof, 50%-70% or 50%-85% of *N-*(8-(2-hydroxybenzoyl)amino)caprylate, 10%-45% of β-cyclodextrin, 1%-1.5% of a glidant, and 0.5%-5% of a lubricant;
a composition (iv-4) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-75% or 40%-75% of β-cyclodextrin, 1%-1.5% of a glidant, and 0.5%-5% of a lubricant;
a composition (v-2) comprising, relative to the total mass of the composition, 1%-2% or 1%-5% of the compound shown as 18# in FIG. 3 or a pharmaceutically acceptable salt, amide or ester thereof, 70%-80% of sodium caprate, and 15%-25% of a protease inhibitor; a composition (v-3) comprising, relative to the total mass of the composition, 1%-5% of the compound shown as 18# in FIG. 3 or a pharmaceutically acceptable salt, amide or ester thereof, 65%-80% of sodium caprate, and 15%-30% of a protease inhibitor;
a composition (vi-2) comprising, relative to the total mass of the composition, 1%-20%, e.g., 1%-5%, of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, 1%-30% of sodium caprate, 1%-1.5% of a glidant, and 0.5%-5% of a lubricant;
a composition (vi-3) comprising, relative to the total mass of the composition, 1%-20%, e.g., 1%-5%, of the compound shown as 18# in FIG. 3 or a pharmaceutically acceptable salt, amide or ester thereof, 20%-50% of *N*-(8-(2-hydroxybenzoyl)amino)caprylate, 15%-40% of arginine or a salt thereof, 10%-35% of sodium caprate, 1%-1.5% of a glidant, and 0.5%-5% of a lubricant;
a composition (vi-4) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, 10%-35% of sodium caprate, 1%-1.5% of a glidant, and 0.5%-5% of a lubricant;
a composition (vii-2) comprising, relative to the total mass of the composition, 1%-20%, e.g., 1%-5%, of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, 1%-30% of leucine or a salt thereof, 1%-1.5% of a glidant, and 0.5%-5% of a lubricant;
a composition (vii-3) comprising, relative to the total mass of the composition, 1%-20%, e.g., 1%-5%, of the compound shown as 18# in FIG. 3 or a pharmaceutically acceptable salt, amide or ester thereof, 20%-50% of N (8-(2-hydroxybenzoyl)amino)caprylate, 10%-35% of arginine or a salt thereof, 15%-40% of leucine or a salt thereof, 1%-1.5% of a glidant, and 0.5%-5% of a lubricant;
a composition (vii-4) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, 10%-35% of leucine or a salt thereof, 1%-1.5% of a glidant, and 0.5%-5% of a lubricant;
a composition (viii-2) comprising, relative to the total mass of the composition, 1%-20%, e.g., 1%-5%, of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, 1%-30% of lysine or a salt thereof, 1%-1.5% of a glidant, and 0.5%-5% of a lubricant;
a composition (viii-3) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-35% of arginine or a salt thereof, 15%-40% of lysine or a salt thereof, 1%-1.5% of a glidant, and 0.5%-5% of a lubricant;
a composition (viii-4) comprising, relative to the total mass of the composition, 1%-20%, e.g., 1%-5%, of the compound shown as 18# in FIG. 3 or a pharmaceutically acceptable salt, amide or ester thereof, 50%-70% of N (8-(2-hydroxybenzoyl)amino)caprylate, 10%-35% of arginine or a salt thereof, 10%-35% of lysine or a salt thereof, 1%-1.5% of a glidant, and 0.5%-5% of a lubricant;
a composition (ix-4) comprising, relative to the total mass of the composition, 1%-20%, e.g., 1%-5%, of the compound shown as 18# in FIG. 3 or a pharmaceutically acceptable salt, amide or ester thereof, 70%-90% of *N-*(8-(2-hydroxybenzoyl)amino)caprylate, 5%-25% of arginine or a salt thereof, 1%-1.5% of a glidant, 0.5%-5% of a lubricant, 1%-20% of a filler, and/or 0.5%-5% of a binder;
a composition (ix-5) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-40% of arginine, 1%-1.5% of a glidant and 0.5%-5% of a lubricant, 5%-35% of a filler, and/or 0.5%-5% of a binder;
a composition (ix-6) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-75% of arginine, 1%-1.5% of a glidant and 0.5%-5% of a lubricant, 1%-40% of a filler, and/or 0.5%-5% of a binder;
a composition (x-2) comprising, relative to the total mass of the composition, 1%-20%, e.g., 1%-5%, of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, 1%-30% of sodium caprylate, 1%-1.5% of a glidant, and 0.5%-5% of a lubricant;
a composition (x-3) comprising, relative to the total mass of the composition, 1%-20%, e.g., 1%-5%, of the compound shown as 18# in FIG. 3 or a pharmaceutically acceptable salt, amide or ester thereof, 20%-50% of *N*-(8-(2-hydroxybenzoyl)amino)caprylate, 15%-40% of arginine or a salt thereof, 5%-30% of sodium caprylate, 1%-1.5% of a glidant, and 0.5%-5% of a lubricant; and
a composition (x-4) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, 10%-35% of sodium caprylate, 1%-1.5% of a glidant, and 0.5%-5% of a lubricant.

In some embodiments, the pharmaceutical composition of the present disclosure is selected from any one of the following compositions:
a composition (i-4) comprising, relative to the total mass of the composition, 1%-5% of the compound shown as 18# in FIG. 3 or a pharmaceutically acceptable salt, amide or ester thereof, 70%-80% of *N-*(8-(2-hydroxybenzoyl)amino)caprylate, about 20% of microcrystalline cellulose, 2% of povidone, and 0.5%-5% of magnesium stearate;
a composition (i-5) comprising, relative to the total mass of the composition, 1%-5% of the compound shown as 18# in FIG. 3 or a pharmaceutically acceptable salt, amide or ester thereof, at least 90% of *N-*(8-(2-hydroxybenzoyl)amino)caprylate, and 0.5%-5% of magnesium stearate;
a composition (ii-5) comprising, relative to the total mass of the composition, 1%-20%, e.g., 1%-5%, of the compound shown as 18# in FIG. 3 or a pharmaceutically acceptable salt, amide or ester thereof, 70%-90% of *N-*(8-(2-hydroxybenzoyl)amino)caprylate, 5%-10% of sodium caprylate, 1%-1.5% of colloidal silicon dioxide, and 0.5%-5% of magnesium stearate;
a composition (ii-6) comprising, relative to the total mass of the composition, 1%-20%, e.g., 1%-5%, of the compound shown as 18# in FIG. 3 or a pharmaceutically acceptable salt, amide or ester thereof, 50%-70% or 50%-85% of *N-*(8-(2-hydroxybenzoyl)amino)caprylate, 10%-45% of sodium caprylate, 1%-1.5% of colloidal silicon dioxide, and 0.5%-5% of magnesium stearate;
a composition (ii-7) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-75% or 40%-75% of sodium caprylate, 1%-1.5% of colloidal silicon dioxide, and 0.5%-5% of magnesium stearate;
a composition (iii-6) comprising, relative to the total mass of the composition, 1%-20%, e.g., 1%-5%, of the compound shown as 18# in FIG. 3 or a pharmaceutically acceptable salt, amide or ester thereof, 70%-90% of *N-*(8-(2-hydroxybenzoyl)amino)caprylate, 5%-10% or 1%-20% of arginine, 1%-1.5% of colloidal silicon dioxide, and 0.5%-5% of magnesium stearate;
a composition (iii-7) comprising, relative to the total mass of the composition, 1%-20%, e.g., 1%-5%, of the compound shown as 18# in FIG. 3 or a pharmaceutically acceptable salt, amide or ester thereof, 50%-70% or 50%-85% of N (8-(2-hydroxybenzoyl)amino)caprylate, 10%-45% of arginine, 1%-1.5% of colloidal silicon dioxide, and 0.5%-5% of magnesium stearate;
a composition (iii-8) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-75% or 40%-75% of arginine, 1%-1.5% of colloidal silicon dioxide, and 0.5%-5% of magnesium stearate;
a composition (iii-9) comprising, relative to the total mass of the composition, 1%-20%, e.g., 1%-5%, of the compound shown as 18# in FIG. 3 or a pharmaceutically acceptable salt, amide or ester thereof, 70%-90% of *N*-(8-(2-hydroxybenzoyl)amino)caprylate, 5%-25% of arginine, 1%-1.5% of colloidal silicon dioxide, and 0.5%-5% of magnesium stearate;
a composition (iv-5) comprising, relative to the total mass of the composition, 1%-20%, e.g., 1%-5%, of the compound shown as 18# in FIG. 3 or a pharmaceutically acceptable salt, amide or ester thereof, 70%-90% of *N-*(8-(2-hydroxybenzoyl)amino)caprylate, 5%-10% or 1%-20% of β-cyclodextrin, 1%-1.5% of colloidal silicon dioxide, and 0.5%-5% of magnesium stearate;
a composition (iv-6) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-45% or 50%-85% of β-cyclodextrin, 1%-1.5% of colloidal silicon dioxide, and 0.5%-5% of magnesium stearate;
a composition (iv-7) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-75% or 40%-75% of β-cyclodextrin, 1%-1.5% of colloidal silicon dioxide, and 0.5%-5% of magnesium stearate; and
a composition (v-4) comprising, relative to the total mass of the composition, 1%-2% of the compound shown as 18# in FIG. 3 or a pharmaceutically acceptable salt, amide or ester thereof, 70%-80% of sodium caprate, and 15%-25% of PMSF;
a composition (v-5) comprising, relative to the total mass of the composition, 1%-5% of the compound shown as 18# in FIG. 3 or a pharmaceutically acceptable salt, amide or ester thereof, 65%-80% of sodium caprate, and 15%-30% of PMSF;
a composition (vi-5) comprising, relative to the total mass of the composition, 1%-20%, e.g., 1%-5%, of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, 1%-30% of sodium caprate, 1%-1.5% of colloidal silicon dioxide, and 0.5%-5% of magnesium stearate;
a composition (vi-6) comprising, relative to the total mass of the composition, 1%-20%, e.g., 1%-5%, of the compound shown as 18# in FIG. 3 or a pharmaceutically acceptable salt, amide or ester thereof, 20%-50% of *N*-(8-(2-hydroxybenzoyl)amino)caprylate, 15%-40% of arginine or a salt thereof, 10%-35% of sodium caprate, 1%-1.5% of colloidal silicon dioxide, and 0.5%-5% of magnesium stearate;
a composition (vi-7) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, 10%-35% of sodium caprate, 1%-1.5% of colloidal silicon dioxide, and 0.5%-5% of magnesium stearate;
a composition (vii-5) comprising, relative to the total mass of the composition, 1%-20%, e.g., 1%-5%, of the compound shown as 18# in FIG. 3 or a pharmaceutically acceptable salt, amide or ester thereof, 50%-90% of N-(8-(2-hydroxybenzoyl)amino)caprylate, 1%-30% of arginine or a salt thereof, 1%-30% of leucine or a salt thereof, 1%-1.5% of colloidal silicon dioxide, and 0.5%-5% of magnesium stearate;
a composition (vii-6) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-35% of arginine or a salt thereof, 15%-40% of leucine or a salt thereof, 1%-1.5% of colloidal silicon dioxide, and 0.5%-5% of magnesium stearate;
a composition (vii-7) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, 10%-35% of leucine or a salt thereof, 1%-1.5% of colloidal silicon dioxide, and 0.5%-5% of magnesium stearate;
a composition (viii-5) comprising, relative to the total mass of the composition, 1%-20%, e.g., 1%-5%, of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, 1%-30% of lysine or a salt thereof, 1%-1.5% of colloidal silicon dioxide, and 0.5%-5% of magnesium stearate;
a composition (viii-6) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-35% of arginine or a salt thereof, 15%-40% of lysine or a salt thereof, 1%-1.5% of colloidal silicon dioxide, and 0.5%-5% of magnesium stearate;
a composition (viii-7) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, 10%-35% of lysine or a salt thereof, 1%-1.5% of colloidal silicon dioxide, and 0.5%-5% of magnesium stearate;
a composition (ix-7) comprising, relative to the total mass of the composition, 1%-20%, e.g., 1%-5%, of the compound shown as 18# in FIG. 3 or a pharmaceutically acceptable salt, amide or ester thereof, 70%-90% of *N-*(8-(2-hydroxybenzoyl)amino)caprylate, 5%-25% of arginine or a salt thereof, 1%-1.5% of colloidal silicon dioxide, 0.5%-5% of magnesium stearate, and 1%-20% of sorbitol;
a composition (ix-8) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-40% of arginine, 1%-1.5% of colloidal silicon dioxide, 0.5%-5% of magnesium stearate, and 5%-35% of sorbitol;
a composition (ix-9) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-75% of arginine, 1%-1.5% of colloidal silicon dioxide, 0.5%-5% of magnesium stearate, and 1%-40% of sorbitol;
a composition (ix-10) comprising, relative to the total mass of the composition, 1%-20%, e.g., 1%-5%, of the compound shown as 18# in FIG. 3 or a pharmaceutically acceptable salt, amide or ester thereof, 70%-90% of *N-*(8-(2-hydroxybenzoyl)amino)caprylate, 5%-25% of arginine or a salt thereof, 1%-1.5% of colloidal silicon dioxide, 0.5%-5% of magnesium stearate, 1%-20% of sorbitol, and 0.5%-5% of povidone or hydroxypropyl cellulose;
a composition (ix-11) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-40% of arginine, 1%-1.5% of colloidal silicon dioxide, 0.5%-5% of magnesium stearate, 5%-35% of sorbitol, and 0.5%-5% of povidone or hydroxypropyl cellulose;
a composition (ix-12) comprising, relative to the total mass of the composition, 1%-20%, e.g., 1%-5%, of the compound shown as 18# in FIG. 3 or a pharmaceutically acceptable salt, amide or ester thereof, 20%-50% of *N-*(8-(2-hydroxybenzoyl)amino)caprylate, 10%-75% of arginine, 1%-1.5% of colloidal silicon dioxide, 0.5%-5% of magnesium stearate, 1%-40% of sorbitol, and 0.5%-5% of povidone or hydroxypropyl cellulose; and
a composition (x-5) comprising, relative to the total mass of the composition, 1%-20%, e.g., 1%-5%, of the compound shown as 18# in FIG. 3 or a pharmaceutically acceptable salt, amide or ester thereof, 50%-90% of *N-*(8-(2-hydroxybenzoyl)amino)caprylate, 1%-30% of arginine or a salt thereof, 1%-30% of sodium caprylate, 1%-1.5% of colloidal silicon dioxide, and 0.5%-5% of magnesium stearate;
a composition (x-6) comprising, relative to the total mass of the composition, 1%-20%, e.g., 1%-5%, of the compound shown as 18# in FIG. 3 or a pharmaceutically acceptable salt, amide or ester thereof, 20%-50% of *N*-(8-(2-hydroxybenzoyl)amino)caprylate,, 15%-40% of arginine or a salt thereof, 10%-35% of sodium caprylate, 1%-1.5% of colloidal silicon dioxide, and 0.5%-5% of magnesium stearate;
a composition (x-7) comprising, relative to the total mass of the composition, 1%-20%, e.g., 1%-5%, of the compound shown as 18# in FIG. 3 or a pharmaceutically acceptable salt, amide or ester thereof, 50%-70% of *N-*(8-(2-hydroxybenzoyl)amino)caprylate, 10%-35% of arginine or a salt thereof, 10%-35% of sodium caprylate, 1%-1.5% of colloidal silicon dioxide, and 0.5%-5% of magnesium stearate.

In some embodiments, the pharmaceutical composition of the present disclosure is selected from any one of the following compositions:
a composition (1) comprising, relative to the total mass of the composition, 1%-2% of the compound shown as 18# in FIG. 3 or a pharmaceutically acceptable salt, amide or ester thereof, about 75% of *N-*(8-(2-hydroxybenzoyl)amino)caprylate, about 20% of microcrystalline cellulose, about 2% of povidone, and about 0.5% or about 2.5% of magnesium stearate;
a composition (2) comprising, relative to the total mass of the composition, 3%-3.5% of the compound shown as 18# in FIG. 3 or a pharmaceutically acceptable salt, amide or ester thereof, 94%-95% of *N*-(8-(2-hydroxybenzoyl)amino)caprylate, and about 2.5% of magnesium stearate;
a composition (3) comprising, relative to the total mass of the composition, about 8% of the compound shown as 18# in FIG. 3 or a pharmaceutically acceptable salt, amide or ester thereof, about 80% of *N-*(8-(2-hydroxybenzoyl)amino)caprylate, about 9% of sodium caprylate, about 1% of colloidal silicon dioxide, and about 2.5% of magnesium stearate;
a composition (4) comprising, relative to the total mass of the composition, about 3% of the compound shown as 18# in FIG. 3 or a pharmaceutically acceptable salt, amide or ester thereof, about 85% of *N-*(8-(2-hydroxybenzoyl)amino)caprylate, about 10% of sodium caprylate, about 1% of colloidal silicon dioxide, and about 2.5% of magnesium stearate;
a composition (5) comprising, relative to the total mass of the composition, about 1.5% of the compound shown as 18# in FIG. 3 or a pharmaceutically acceptable salt, amide or ester thereof, about 85% of *N-*(8-(2-hydroxybenzoyl)amino)caprylate, about 10% of sodium caprylate, about 1% of colloidal silicon dioxide, and about 2.5% of magnesium stearate;
a composition (6) comprising, relative to the total mass of the composition, about 3% of the compound shown as 18# in FIG. 3 or a pharmaceutically acceptable salt, amide or ester thereof, about 85% of *N-*(8-(2-hydroxybenzoyl)amino)caprylate, about 10% of sodium caprylate, about 1% of colloidal silicon dioxide, and about 2.5% of magnesium stearate;
a composition (7) comprising, relative to the total mass of the composition, about 5% of the compound shown as 18# in FIG. 3 or a pharmaceutically acceptable salt, amide or ester thereof, about 85% of *N-*(8-(2-hydroxybenzoyl)amino)caprylate, about 10% of sodium caprylate, about 1% of colloidal silicon dioxide, and about 2.5% of magnesium stearate;
a composition (8) comprising, relative to the total mass of the composition, about 5.5% of the compound shown as 18# in FIG. 3 or a pharmaceutically acceptable salt, amide or ester thereof, 80%-85% of *N*- (8-(2-hydroxybenzoyl)amino)caprylate, about 10% of sodium caprylate, about 1% of colloidal silicon dioxide, and about 2.5% of magnesium stearate;
a composition (9) comprising, relative to the total mass of the composition, about 15% of the compound shown as 18# in FIG. 3 or a pharmaceutically acceptable salt, amide or ester thereof, 70%-75% of *N*-(8-(2-hydroxybenzoyl)amino)caprylate, 8%-10% of sodium caprylate, about 1% of colloidal silicon dioxide, and about 2.5% of magnesium stearate; a composition (10) comprising, relative to the total mass of the composition, about 3% of the compound shown as 18# in FIG. 3 or a pharmaceutically acceptable salt, amide or ester thereof, about 85% of *N-*(8-(2-hydroxybenzoyl)amino)caprylate, about 10% of arginine or a salt thereof, about 1.5% of colloidal silicon dioxide, and about 1.5% of magnesium stearate;
a composition (11) comprising, relative to the total mass of the composition, about 3% of the compound shown as 18# in FIG. 3 or a pharmaceutically acceptable salt, amide or ester thereof, about 60% of *N-*(8-(2-hydroxybenzoyl)amino)caprylate, about 35% of arginine or a salt thereof, about 1.5% of colloidal silicon dioxide, and about 1% of magnesium stearate;
a composition (12) comprising, relative to the total mass of the composition, about 2.5% of the compound shown as 18# in FIG. 3 or a pharmaceutically acceptable salt, amide or ester thereof, about 45% of *N-*(8-(2-hydroxybenzoyl)amino)caprylate, about 50% of arginine or a salt thereof, about 1.5% of colloidal silicon dioxide, and about 1% of magnesium stearate;
a composition (13) comprising, relative to the total mass of the composition, about 4% of the compound shown as 18# in FIG. 3 or a pharmaceutically acceptable salt, amide or ester thereof, about 75% of *N-*(8-(2-hydroxybenzoyl)amino)caprylate, about 18% of arginine or a salt thereof, about 1% of colloidal silicon dioxide, and about 2% of magnesium stearate;
a composition (14) comprising, relative to the total mass of the composition, 2%-5% or 5% of the compound shown as 18# in FIG. 3 or a pharmaceutically acceptable salt, amide or ester thereof, about 58% of *N-*(8-(2-hydroxybenzoyl)amino)caprylate, about 35% of arginine or a salt thereof, about 1% of colloidal silicon dioxide, and about 2% of magnesium stearate;
a composition (15) comprising, relative to the total mass of the composition, about 3% of the compound shown as 18# in FIG. 3 or a pharmaceutically acceptable salt, amide or ester thereof, about 85% of *N-*(8-(2-hydroxybenzoyl)amino)caprylate, about 10% of histidine or a salt thereof, about 1.5% of colloidal silicon dioxide, and about 1.5% of magnesium stearate;
a composition (16) comprising, relative to the total mass of the composition, about 3% of the compound shown as 18# in FIG. 3 or a pharmaceutically acceptable salt, amide or ester thereof, about 85% of *N-*(8-(2-hydroxybenzoyl)amino)caprylate, about 10% of β-cyclodextrin, about 0.5% of colloidal silicon dioxide, and about 2.5% of magnesium stearate;
a composition (17) comprising, relative to the total mass of the composition, about 1.5% of the compound shown as 18# in FIG. 3 or a pharmaceutically acceptable salt, amide or ester thereof, about 80% of sodium caprate, and about 20% of PMSF;
a composition (18) comprising, relative to the total mass of the composition, about 2.5% of the compound shown as 18# in FIG. 3 or a pharmaceutically acceptable salt, amide or ester thereof, about 47% of *N-*(8-(2-hydroxybenzoyl)amino)caprylate, about 28% of arginine or a salt thereof, about 20% of sodium caprate, about 1% of colloidal silicon dioxide, and about 2% of magnesium stearate;
a composition (19) comprising, relative to the total mass of the composition, about 4% of the compound shown as 18# in FIG. 3 or a pharmaceutically acceptable salt, amide or ester thereof, about 47% of *N-*(8-(2-hydroxybenzoyl)amino)caprylate, about 19% of arginine or a salt thereof, about 28% of sodium caprate, about 1% of colloidal silicon dioxide, and about 2% of magnesium stearate;
a composition (20) comprising, relative to the total mass of the composition, about 5% of the compound shown as 18# in FIG. 3 or a pharmaceutically acceptable salt, amide or ester thereof, about 58% of *N-*(8-(2-hydroxybenzoyl)amino)caprylate, about 23% of arginine or a salt thereof, about 12% of sodium caprate, about 1% of colloidal silicon dioxide, and about 2% of magnesium stearate;
a composition (21) comprising, relative to the total mass of the composition, about 4% of the compound shown as 18# in FIG. 3 or a pharmaceutically acceptable salt, amide or ester thereof, about 47% of *N-*(8-(2-hydroxybenzoyl)amino)caprylate, about 19% of arginine or a salt thereof, about 28% of leucine, about 1% of colloidal silicon dioxide, and about 2% of magnesium stearate;
a composition (22) comprising, relative to the total mass of the composition, about 4% of the compound shown as 18# in FIG. 3 or a pharmaceutically acceptable salt, amide or ester thereof, about 47% of *N-*(8-(2-hydroxybenzoyl)amino)caprylate, about 19% of arginine or a salt thereof, about 28% of lysine or a salt thereof, about 1% of colloidal silicon dioxide, and about 2% of magnesium stearate;
a composition (23) comprising, relative to the total mass of the composition, about 2%-4% of the compound shown as 18# in FIG. 3 or a pharmaceutically acceptable salt, amide or ester thereof, about 47% of *N-*(8-(2-hydroxybenzoyl)amino)caprylate, about 28% of arginine or a salt thereof, about 20% of sorbitol, about 1% of colloidal silicon dioxide, and about 2% of magnesium stearate;
a composition (24) comprising, relative to the total mass of the composition, about 4% of the compound shown as 18# in FIG. 3 or a pharmaceutically acceptable salt, amide or ester thereof, about 45% of *N-*(8-(2-hydroxybenzoyl)amino)caprylate, about 20% of arginine or a salt thereof, about 28% of sorbitol, about 1% of colloidal silicon dioxide, and about 2% of magnesium stearate;
a composition (25) comprising, relative to the total mass of the composition, about 4% of the compound shown as 18# in FIG. 3 or a pharmaceutically acceptable salt, amide or ester thereof, about 47% of *N-*(8-(2-hydroxybenzoyl)amino)caprylate, about 20% of arginine or a salt thereof, about 25% of sorbitol, about 1.5% of povidone or hydroxypropyl cellulose, about 1% of colloidal silicon dioxide, and about 2% of magnesium stearate; and
a composition (26) comprising, relative to the total mass of the composition, about 2.5% of the compound shown as 18# in FIG. 3 or a pharmaceutically acceptable salt, amide or ester thereof, about 47% of *N-*(8-(2-hydroxybenzoyl)amino)caprylate, about 28% of arginine or a salt thereof, about 20% of sodium caprylate, about 1% of colloidal silicon dioxide, and about 2% of magnesium stearate.

In some embodiments, the pharmaceutical composition of the present disclosure is administered orally.

In some embodiments, the pharmaceutical composition of the present disclosure is a powder, a granule, a tablet, a capsule, or a lyophilized formulation.

In some embodiments, the pharmaceutical composition of the present disclosure is a tablet or a capsule.

In some embodiments, the capsule is an immediate release formulation or an enteric-coated formulation.

In some embodiments, the pharmaceutical composition of the present disclosure is in the form of a tablet.

In some embodiments, the weight of the tablet is 100 mg to 1000 mg, such as 100 mg to 800 mg, 100 mg to 600 mg, or 100 mg to 300 mg. In some embodiments, the weight of the tablet is 100 mg, 150 mg, 200 mg, 250 mg, 300 mg, 350 mg, 400 mg, 450 mg, 500 mg, 550 mg, or 600 mg.

In some embodiments, the aforementioned pharmaceutical composition of the present disclosure has good dissolution characteristics.

In some embodiments, the dissolution test is performed at 50 rpm using phosphate buffer (pH 6.8) containing 0.1% Tween 80 as a dissolution medium, and the dissolution of the GLP-1 agonist and/or absorption enhancer in the pharmaceutical composition of the present disclosure at 15 min is ≥ 80%, ≥ 85%, ≥ 90%, ≥ 95%, ≥.98%, or ≥99%.

In some embodiments, the dissolution test is performed at 50 rpm using phosphate buffer (pH 6.8) containing 0.1% Tween 80 as a dissolution medium, and the dissolution of the GLP-1 agonist and/or absorption enhancer in the pharmaceutical composition of the present disclosure at 30 min is ≥ 80%, ≥ 85%, ≥ 90%, ≥ 95%, ≥.98%, or ≥ 99%.

In some embodiments, in the aforementioned pharmaceutical composition of the present disclosure, the dissolution of the active ingredient is substantially consistent with the dissolution of the absorption enhancer SNAC.

In some embodiments, the aforementioned pharmaceutical composition of the present disclosure has good *in vivo* exposure and/or oral bioavailability. For example, the *in vivo* exposure and/or oral bioavailability are better than or equivalent to those of LY3298176. In some embodiments, the aforementioned pharmaceutical composition of the present disclosure has good stability.

In some embodiments, in the pharmaceutical composition of the present disclosure, the total impurity content is no more than 10.0%, e.g., no more than 9.0%, 8.0%, 7.0%, 6.0%, 5.0%, 4.0%, 3.0%, or 2.0%, after the pharmaceutical composition is stored at 40 °C/RH75% for 2 months.

In some embodiments, in the pharmaceutical composition of the present disclosure, the total impurity content is no more than 6.0%, e.g., no more than 5.5%, 5.0%, 4.5%, 4.0%, 3.5%, 3.0%, 2.5%, 2.0%, 1.5%, or 1.0%, after the pharmaceutical composition is stored at 25 °C/RH60% for 2 months.

In some embodiments, in the pharmaceutical composition of the present disclosure, the total impurity content is no more than 6.0%, e.g., no more than 5.5%, 5.0%, 4.5%, 4.0%, 3.5%, 3.0%, 2.5%, 2.0%, 1.5%, or 1.0%, after the pharmaceutical composition is stored at 5±3 °C% for 1 month.

The present disclosure further provides a method for preparing any one of the aforementioned pharmaceutical compositions, which comprises the step of mixing the GLP-1 analog or the pharmaceutically acceptable salt, amide or ester thereof with the absorption enhancer.

In some embodiments, the method comprises the step of tabletting, granulating, or capsule filling.

In some embodiments, the method comprises the step of direct powder compression, wet granulation, dry granulation, or filling enteric capsule shells.

The present disclosure further provides use of any one of the aforementioned pharmaceutical compositions in the preparation of a medicament for treating non-insulin-dependent diabetes, insulin-dependent diabetes, obesity, non-alcoholic fatty liver, hepatic steatosis, diabetic retinopathy, diabetic neuropathy, diabetic nephropathy, insulin resistance, dyslipidemia associated with insulin resistance, and/or dyslipidemia associated with diabetes.

The GLP-1 analog provided by the present disclosure is synthesized by a solid-phase synthesis method. As an example, the synthetic vector is Rink-amide MBHA (Xi'an sunresin Tech Ltd.) resin. During the synthesis, the α-amino of the amino acid derivative used is protected by the Fmoc (fluorenylmethoxycarbonyl) group. As an example, for the side chain of an amino acid, the following protecting groups are selected according to the difference of functional groups: the sulfhydryl of the cysteine side chain, the amino of the asparagine and glutamine side chains, and the imidazolyl of the histidine side chain are protected by Trt (trityl); the guanidyl of the arginine side chain is protected by Pbf (2,2,4,6,7-pentamethyldihydrobenzofuran-5-sulfonyl); the indolyl of the tryptophan side chain and the amino of the lysine side chain are protected by Boc (*tert*-butyloxycarbonyl); and the carboxyl of the aspartic acid and glutamic acid side chains, the hydroxyl of the threonine side chain, the phenolic group of the tyrosine side chain, and the hydroxyl of the serine side chain are protected by *t*-Bu (*tert*-butyl). As an example, during the synthesis, the carboxyl of the C-terminal amino acid residue of the polypeptide is firstly condensed to the insoluble Rink-amide MBHA polymer resin in the form of an amide bond; then the Fmoc protecting group on the α-amino is removed using an *N,N-*dimethylformamide (DMF) solution containing 20% 4-methylpiperidine; and then the solid-phase support is condensed in excess with the next amino acid derivative in the polypeptide sequence to form an amide bond to extend the peptide chain. The procedures of "condensation → washing → deprotection → washing → the next round of amino acid condensation" were repeated to enable the desired length of the polypeptide chain to be synthesized; finally, a mixed solution of trifluoroacetic acid:water:triisopropylsilane (as an example, 90:5:5, v:v:v) is reacted with the resin to cleave the polypeptide from the solid-phase support, and the polypeptide is precipitated using frozen methyl *tert*-butyl ether (5 volume) to obtain a crude solid product of the GLP-1 analog. The crude solid product of the polypeptide is dissolved in an acetonitrile/water mixed solution containing 0.1% trifluoroacetic acid, and purified and separated using a C-18 reversed-phase preparative chromatographic column to obtain a pure product of the GLP-1 analog.

According to some embodiments, the present disclosure further provides a kit-of-parts, which comprises:
- the GLP-1 analog or the pharmaceutically acceptable salt, amide or ester thereof according to the present disclosure; and
- an additional therapeutic agent selected from any one of or a combination of: an anti-obesity agent, an antidiabetic agent, an antihypertensive agent, and a lipid-lowering agent; wherein the GLP-1 analog or the pharmaceutically acceptable salt, amide or ester thereof and the additional therapeutic agent are each placed in a separate container. In some embodiments, the GLP-1 analog or the pharmaceutically acceptable salt, amide or ester thereof and the additional therapeutic agent are administered to a subject separately or in combination (e.g., simultaneously or sequentially).

### BRIEF DESCRIPTION OF THE DRAWINGS

FIG. 1 shows the effect of the compound of the present disclosure on the rate of change in body weight of diet-induced-obesity mice.
FIG. 2 shows the effect of the compound of the present disclosure on daily food intake of diet-induced-obesity mice.
FIG. 3 shows the structures of exemplary compounds of the present disclosure.

### DETAILED DESCRIPTION

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. The amino acid sequences of the present disclosure contain the standard single-letter or three-letter codes for twenty amino acids, and all amino acid residues in the present disclosure are preferably in the L-configuration unless specifically stated. In addition, Aib refers to α-aminoisobutyric acid, D-Ala refers to D-alanine, Orn refers to ornithine, Dap refers to 2,3-diaminopropionic acid, and Dab refers to 2,4-diaminobutyric acid.

The term "agonist" is defined as a substance having an activating effect on the GLP-1 receptor or on the GIP receptor.

The term "GLP-1/GIP dual-agonist" refers to a substance or ligand that can activate the GLP-1 receptor and the GIP receptor.

The term "treat", "treating", or "treatment" includes inhibiting, alleviating, stopping, or reversing the progression or severity of an existing symptom or condition.

The term "natural amino acids" refers to 20 conventional amino acids, i.e., alanine (A), cysteine (C), aspartic acid (D), glutamic acid (E), phenylalanine (F), glycine (G), histidine (H), isoleucine (I), lysine (K), leucine (L), methionine (M), asparagine (N), proline (P), glutamine (Q), arginine (R), serine (S), threonine (T), valine (V), tryptophan (W), and tyrosine (Y).

The term "unnatural amino acids" refers to amino acids that are not naturally encoded or are not found in the genetic code of any organism. For example, the unnatural amino acids may be completely synthetic compounds. Examples of unnatural amino acids include, but are not limited to, hydroxyproline, γ-carboxyglutamic acid, O-phosphoserine, azetidinecarboxylic acid, 2-aminoadipic acid, 3-aminoadipic acid, β-alanine, aminopropionic acid, 2-aminobutyric acid, 4-aminobutyric acid, 6-aminohexanoic acid, 2-aminoheptanoic acid, 2-aminoisobutyric acid, 3-aminoisobutyric acid, 2-aminopimelic acid, *tert*-butylglycine, 2,4-diaminoisobutyric acid (Dap), desmosine, 2,2'-diaminopimelic acid, 2,3-diaminopropionic acid (Dab), *N*-ethylglycine, *N*-methylglycine, *N*-ethylasparagine, homoproline, hydroxylysine, allo-hydroxylysine, 3-hydroxyproline, 4-hydroxyproline, isodesmosine, allo-isoleucine, *N*-methylalanine, *N*-methylglycine, *N-*methylisoleucine, *N*-methylpentylglycine, *N*-methylvaline, naphthalanine, norvaline, norleucine, ornithine (Orn), D-omithine, D-arginine, *p*-aminophenylalanine, pentylglycine, pipecolic acid, and thioproline. In addition, the term also includes derivatives obtained by chemical modification of the C-terminal carboxyl (or N-terminal amino and/or side chain functional group) of a natural amino acid (or unnatural amino acid).

The term "alkyl" refers to a saturated aliphatic hydrocarbon group which is a linear or branched group containing 1 to 20 carbon atoms, e.g., alkyl containing 1 to 8 carbon atoms, e.g., alkyl containing 1 to 6 carbon atoms, e.g., alkyl containing 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof, and the like. Alkyl may be, for example, a lower alkyl containing 1 to 6 carbon atoms, and non-limiting examples include methyl, ethyl, *n-*propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n-*hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted. When it is substituted, the substituent may be substituted at any accessible point of attachment, and the substituent may be one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and a carboxylate group. The substituted alkyl of the present disclosure may be methyl, ethyl, isopropyl, *tert*-butyl, haloalkyl, deuterated alkyl, alkoxy-substituted alkyl, or hydroxy-substituted alkyl.

The expressions "X is selected from the group consisting of A, B or C", "X is selected from the group consisting of A, B and C", "X is A, B or C", "X is A, B and C", and the like all carry the same meaning, i.e., X may be any one or more of A, B and C.

The "modification" of the amino acid as described in the present disclosure refers to substitution, addition, or deletion of an amino acid, including substitution or addition of any one or more of the 20 natural amino acids.

The term "natural GLP-1" refers to a naturally occurring molecule of the glucagon or exendin family of peptides, wherein the glucagon family of peptides is encoded by the pre-proglucagon gene and includes three small peptides with high homology, i.e., glucagon (1-29), GLP-1 (1-37), and GLP-2 (1-33); and exendins are peptides expressed in lizards and, like GLP-1, are insulinotropic. In some embodiments, the term "natural GLP-1" also refers to human GLP-1 (7-37) and human GLP-1 (7-36).

The term "GLP-1 analog" refers to a substance having up to 25, up to 24, up to 23, up to 22, up to 21, up to 20, up to 19, up to 18, up to 17, up to 16, up to 15, up to 14, up to 13, up to 12, up to 11, up to 10, up to 9, up to 8, up to 7, up to 6, up to 5, up to 4, up to 3, up to 2, or 1 amino acid modification or chemical modification compared with natural GLP-1 (in particular with human GLP-1 (7-37) and human GLP-1 (7-36)), wherein the amino acid modification may be an amino acid substitution, addition, and/or deletion, and the chemical modification may be a chemical modification with a group selected from the group consisting of the following groups: amide, carbohydrate, alkyl, acyl, ester, a polyethylene glycol (PEG) group, a sialylation group, a glycosylation group, and the like. The term amino acid "substitution" as described in the present disclosure refers to the substitution of one amino acid residue with a different amino acid residue.

The term "polyethylene glycol" or "PEG" refers to a mixture of polycondensates of ethylene oxide and water and is present in a linear or branched form and represented by the general formula H(OCH₂CH₂)ₙOH, where n is at least equal to 9. Unless further stated, this term includes polymers of polyethylene glycol having an average total molecular weight selected from the group consisting of 5,000 to 40,000 daltons.

The term "fatty acid" refers to carboxylic acid with an aliphatic long tail (chain) and may be saturated or unsaturated. The fatty acids in the present disclosure are carboxylic acids having a C4-C30 linear or branched aliphatic group.

The term "peptide" encompasses peptides having modified amino and carboxyl termini. For example, an amino acid chain containing a terminal carboxylic acid substituted with an amide group is also included within the amino acid sequence designated as a natural amino acid.

All of the hydrogen atoms described in the present disclosure may be substituted with their isotopes (protium, deuterium, and tritium), and any hydrogen atom in the compound of the present disclosure to which the present disclosure relates may also be substituted with an isotope atom.

The term "optional" or "optionally" means that the event or circumstance subsequently described may, but does not necessarily, occur and that the description includes instances where the event or circumstance occurs or does not occur. For example, "a heterocyclyl group optionally substituted with alkyl" means that the alkyl may, but does not necessarily, exist and that the description includes instances where the heterocyclyl group is or is not substituted with the alkyl.

The term "substituted" means that one or more, preferably up to 5, more preferably 1 to 3 hydrogen atoms in the group are independently substituted with a substituent. A substituent is only in its possible chemical position, and those skilled in the art will be able to determine (experimentally or theoretically) possible or impossible substitution without undue efforts. For example, it may be unstable when amino or hydroxy having a free hydrogen is bound to a carbon atom having an unsaturated (e.g., olefinic) bond. The term "agonist activity" refers to the ability of the compound according to the present disclosure to activate the human GIP receptor and the human GLP-1 receptor. In some examples, "agonist activity" is embodied in a relatively active form and specifically refers to the ratio of the activation ability of the compound of the present disclosure against GLP-1R to that against the GIP receptor.

The term "pharmaceutically acceptable salt" refers to the salts of the compound of the present disclosure, which are safe and effective for use in the body of a mammal and possess the requisite biological activities. Acids commonly employed to form acid addition salts are: hydrochloric acid, hydrobromic acid, hydroiodic acid, sulfuric acid, phosphoric acid, *p*-toluenesulfonic acid, methanesulfonic acid, oxalic acid, *p-*bromophenylsulfonic acid, carbonic acid, succinic acid, citric acid, benzoic acid, and acetic acid; the salts include sulfate, pyrosulfate, trifluoroacetate, sulfite, bisulfite, phosphate, hydrogen phosphate, dihydrogen phosphate, metaphosphate, pyrophosphate, hydrochloride, bromide, iodide, acetate, propionate, caprylate, acrylate, formate, isobutyrate, hexanoate, heptanoate, propiolate, oxalate, malonate, succinate, suberate, fumarate, maleate, butyne-1,4-dioate, hexyne-1,6-dioate, benzoate, chlorobenzoate, methylbenzoate, dinitrobenzoate, hydroxybenzoate, methoxybenzoate, phenyl acetate, phenylpropionate, phenylbutyrate, citrate, lactate, γ-hydroxybutyrate, glycolate, tartrate, mesylate, propanesulfonate, naphthalene-1-sulfonate, naphthalene-2-sulfonate, mandelate, and the like, such as trifluoroacetate. Alkaline substances may also form salts with GLP-1 analogs, and these alkaline substances include ammonium, alkali or alkaline earth metal hydroxides, carbonate, and bicarbonate, such as sodium hydroxide, potassium hydroxide, ammonium hydroxide, sodium carbonate, potassium carbonate, and the like. Semaglutide refers to a once-a-week GLP-1 receptor single agonist polypeptide drug developed by Novo Nordisk in Denmark, which is currently approved and marketed in the United States, Japan, and the European Union.

LY3298176 refers to a once-a-week GIP receptor/GLP-1 receptor dual-agonist polypeptide drug developed by Eli Lilly, which is currently in phase III clinical trials in several countries. The structure is as follows:
YAibEGTFTSDYSIAibLDKIAQ**K**AFVQWLIAGGPSSGAPPPS-NH₂, wherein the K at position 20 is modified with a fatty acid shown below as

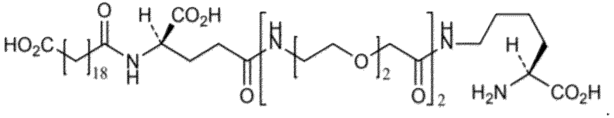

"Pharmaceutical composition" refers to a mixture containing one or more of the compounds described herein or physiologically/pharmaceutically acceptable salts or prodrugs thereof, and other chemical components, wherein the other components are, for example, physiologically/pharmaceutically acceptable carriers and excipients. The pharmaceutical composition is intended to promote administration to an organism and facilitate the absorption of the active ingredient so that it can exert its biological activity. As used herein, "pharmaceutical composition" and "formulation" may be used interchangeably.

The term "excipient" as used herein generally refers to any component other than the active therapeutic ingredient. Excipients may be inert substances, inactive substances, and/or substances without pharmaceutical activity. Excipients can be used for various purposes, e.g., as carriers, fillers, binders, lubricants, glidants, disintegrants, flow control agents, crystallization retarders, solubilizers, stabilizers, colorants, flavoring agents, surfactants, and emulsifiers, and/or for improving the administration and/or absorption of active substances. Those skilled in the art may select one or more of the above excipients according to the particular desired properties of the solid oral dosage form by routine experimentation and without any undue burden. The amount of each excipient used may vary within ranges conventional in the art. Techniques and excipients that may be used to formulate oral dosage forms are described in Handbook of Pharmaceutical Excipients, 6th edition, Rowe et al., Eds., American Pharmaceuticals Association and the Pharmaceutical Press, publishing department of Royal Pharmaceutical Society of Great Britain (2009); and Remington: the Science and Practice of Pharmacy, the 21st edition, Gennaro, Ed., Lippincott Williams & Wilkins (2005).

The term "about" or "approximately" as used herein means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprise" may mean a range of up to ±20%; for example, a pH of about 5.5 means a pH of 5.5±1.1. Furthermore, particularly for biological systems or processes, the term may mean up to an order of magnitude or up to 5-fold of a numerical value. Unless otherwise stated, when a specific value is provided in the present application and claims, the meaning of "about" or "substantially comprise" should be assumed to be within an acceptable error range for that specific value.

The pharmaceutical composition described in the present disclosure can achieve the effect of being stable: the GLP-1 analog in the pharmaceutical composition substantially retains its physical and/or chemical stability and/or biological activity after storage; for example, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical formulation is one in which no significant change is observed under the following conditions: storage at refrigeration temperature (2-8 °C) for at least 3 months, at least 6 months, at least 1 year, or at most 2 years. In addition, stable pharmaceutical formulations include formulations that exhibit desirable features after storage at 25 °C for periods including 1 month, 3 months, and 6 months, or storage at 40 °C for periods including 1 month. Typical acceptable criteria for stability are as follows: typically, no more than about 10%, for example, no more than about 5% of the GLP-1 analog monomer, is degraded as measured by SEC-HPLC. Typically, clippings of no more than about 10%, for example, no more than about 5% are observed. Typically, aggregation of no more than about 10%, for example, no more than about 5%, is formed. A GLP-1 analog "retains its physical stability" in a pharmaceutical formulation if it shows no significant increase in aggregation, precipitation, and/or denaturation upon visual inspection of color and/or clarity, or as determined by UV light scattering, size exclusion chromatography (SEC), and dynamic light scattering (DLS). Changes in protein conformation can be assessed by fluorescence spectroscopy (which determines the protein tertiary structure) and by FTIR spectroscopy (which determines the protein secondary structure).

A GLP-1 analog "retains its chemical stability" in a pharmaceutical formulation if it shows no significant chemical change. Chemical stability can be evaluated by detecting and quantifying chemically changed protein. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.). A GLP-1 analog "retains its biological activity" in a pharmaceutical formulation if its biological activity at a given time is within a predetermined range of the biological activity exhibited during the preparation of the pharmaceutical formulation. The biological activity of a GLP-1 analog can be determined, for example, by an antigen binding assay.

The three-letter and single-letter codes for amino acids used in the present disclosure are as described in J. biol. chem, 243, p3558 (1968).

### Examples

The following specific embodiments are provided herein only for illustrating the present disclosure in more detail, rather than limiting the present disclosure. Experimental procedures without specific conditions indicated in the examples of the present disclosure are generally conducted according to conventional conditions or according to conditions recommended by the manufacturers of the starting materials or commercial products. Reagents without specific sources indicated are commercially available conventional reagents.

**Table 1. Part of experimental reagents and sources**

| Reagent | Source |
|---|---|
| Rink-amide MBHA resin | Xi'an sunresin Tech Ltd. |
| HCTU (O-(6-chloro-1-benzotriazol-1-yl)-*N*,*N*,*N'*,*N'-*tetramethyluronium hexafluorophosphate) | Highfine Tech Ltd., Sunzhou |
| Fmoc-Aib-OH | GL Biochem |
| Fmoc-L-Lys(Mtt)-OH | GL Biochem |
| *N,N*-dimethylformamide | SinoPharm |
| Dichloromethane | SinoPharm |
| Trifluoroacetic acid | SinoPharm |
| Triisopropylsilane | Sigma-Aldrich |
| Hexafluoroisopropanol | Sigma-Aldrich |
| Acetonitrile | Merck-Millipore |
| Diisopropylethylamine | Sigma-Aldrich |
| 4-methylpiperidine | TCI Chemicals |
| Methyl *tert*-butyl ether | TCI Chemicals |
| Boc-L-Tyr(tBu)-OH | GL Biochem |
| Fmoc-NH-PEG₂-COOH | GL Biochem |
| Fmoc-L-Glu-OtBu | GL Biochem |
| HOOC-(CH₂)₁₈-COOtBu | ChinaPeptides Co., Ltd, Suzhou |
| 4-methylmorpholine | TCI Chemicals |
| DMEM/F12 | Gibco 11330032 |
| Casein | Sigma C3400-500G |
| 3-Isobutyl-1-methylxanthine | Sigma I7018-250 MG |
| cAMP - Gs Dynamic kit - 20,000 tests | Cisbio 62AM4PEC |
| Coming^{®} 384 well microplate, low volume | Sigma CLS4514-50EA |
| 96-well V bottom plate (PS) | Axygen WIPP02280 |
| Countess^{®} Cell Counting Chamber Slides | Invitrogen C10228 |
| puromycin | ThermoFisher A1113803 |
| Hygromycin B | Sigma A1720 |
| PBS | Gibco 10010023 |
| 0.25%Trypsin-EDTA(1X), Phenol Red | ThermoFisher 25200-114 |
| Gibco^{™} Fetal Bovine Serum, Qualified, Australia Origin | ThermoFisher 10099-141 |
| Glucose | Sigma G8270-100G |

**Table 2. Part of experimental instruments and sources**

| Instrument | Source |
|---|---|
| H-CLASS analytical ultra-high performance liquid chromatograph | WATERS |
| Agilent 1290-6530 ultra-high performance liquid chromatograph/mass spectrometer combination | Agilent |
| Labconco multifunctional freeze dryer | Thermo-Fisher Scientific |
| Prep150 preparative high performance liquid chromatograph | WATERS |
| Prelude-X automatic polypeptide synthesizer | Protein Technology Inc |
| Multichannel high-speed centrifuge | Sigma |
| Refrigerated centrifuge 5810R | Eppendorf 5810R |
| Active glucometer | Roche |
| Microplate reader | BioTek HIMFD |

### Example 1. Chemical Synthesis of Compound 18#

### 1. Synthesis of polypeptide skeleton

Rink-amide MBHA resin (degree of substitution: 0.48 mMole/g, 0.1 mMol) was taken and placed in a polypropylene reaction tube for solid phase synthesis of the polypeptide; *N,N*-dimethylformamide (DMF, 10 mL) was added to swell the resin for 10 min under nitrogen-blowing; DMF was removed *in vacuum,* and fresh DMF (10 mL) was added to wash the resin; after repeated washing of the resin twice, the solid phase synthesis of the polypeptide was performed on a Prelude-X automatic polypeptide synthesizer using Fmoc/tBu strategy, in which 10 equivalents of amino acid residues activated by HCTU and 4-methylmorpholine (molar ratio of HCTU to 4-methylmorpholine to amino acid residues was 1:2:1) were reacted in DMF at room temperature for 25 min for amide bond condensation, so as to achieve coupling. Deprotection of the N-terminal Fmoc protecting group was performed by 2 reactions (10 min each) at room temperature using a DMF solution containing 20% 4-methylpiperidine. In the synthesis of a polypeptide skeleton, the N-terminal amino acid residue was constructed using Boc-L-Tyr (tBu)-OH and subjected to secondary condensation, which was necessary for improving the quality of a crude peptide.

### 2. Selective deprotection of resin-peptide protecting group Mtt and fatty acid modification of side chain

After the extension of the polypeptide skeleton (or called resin-peptide) was completed, a mixed solution (10 mL) of dichloromethane containing 30% hexafluoroisopropanol was added, and the mixture was shaken at room temperature for 45 min, and then the mixed solution was removed *in vacuum*; a mixed solution (10 mL) of dichloromethane containing 30% hexafluoroisopropanol was added, and the mixture was shaken at room temperature for 45 min, and then the mixed solution was removed *in vacuum.* After the reaction was completed, the resin was washed 6 times with DMF. The lysine side chain at position 14 was extended using a Prelude-X automatic polypeptide synthesizer, with an additional coupling/deprotection cycle involving the amino acid components Fmoc-NH-PEG₂-COOH and Fmoc-L-Glu-OtBu. All couplings were performed in DMF at room temperature for 25 min using 10 equivalents of amino acid residues activated by HCTU and 4-methylmorpholine (molar ratio of HCTU to 4-methylmorpholine to amino acid residues was 1:2:1). Deprotection of the N-terminal Fmoc protecting group was performed by 2 reactions (10 min each) at room temperature using a DMF solution containing 20% 4-methylpiperidine. After the finally obtained resin was washed three times with DCM and DMF separately, a mixed solution (8 mL) of DMF containing 10 equivalents of HOOC-(CH₂)₁₈-COOtBu, 10 equivalents of HCTU, and 20 equivalents of diisopropylethylamine (DIEA) was added, and the mixture was reacted at room temperature for 4 h to complete the fatty acid modification of the side chain.

### 3. Product cleavage

The resin-peptide obtained in the previous step was washed 3 times with DMF and DCM sequentially and dried in vacuum, followed by the addition of a freshly prepared cleavage buffer (trifluoroacetic acid:triisopropylsilane:water = 90:5:5, v:v:v), and the mixture was shaken at room temperature for 3-4 h. After the reaction was completed, the mixture was filtered and the resin was washed twice with trifluoroacetic acid. The filtrates were combined before a large amount of frozen methyl *tert*-butyl ether was added to precipitate a solid. The mixture was centrifuged and the supernatant was discarded to obtain a crude polypeptide of **compound 18#.**

### 4. Purification by reversed-phase liquid chromatography

The crude polypeptide of **compound 18#** was dissolved in a mixed solvent containing 0.1% trifluoroacetic acid, 20% acetonitrile, and 20% acetic acid/water, and the solution was filtered through a 0.22 µm membrane; the filtrate was separated using a WATERS Prep 150 LC reversed-phase high performance liquid chromatography system with buffers A (0.1% trifluoroacetic acid, 10% acetonitrile, and water) and B (0.1% trifluoroacetic acid, 90% acetonitrile, and water). The chromatographic column was an X-SELECT OBD C-18 reversed-phase chromatographic column, and in the purification process, the detection wavelength of the chromatograph was set as 220 nm, and the flow rate was 15 mL/min. The related fractions of the product were collected and lyophilized to obtain a pure polypeptide product of compound 1#, with the yield of 18%. The purity of the pure polypeptide product was determined by a combination of analytical high performance liquid chromatography and ultra-high performance liquid chromatography/mass spectrometry, with the purity of 92.81%. The molecular structure of compound 18# is: H-YAibEGTFTSDYSIYK(OEG-OEG-γGlu-C20-OH)EKIAAQEFVNWLLAGGPSSGAPPPS-NH₂, with the structural formula shown as the structure of 18# in FIG. 3.

### Example 2. Chemical Synthesis of Other Compounds

The compounds in Table 3 were synthesized using the experimental protocol of Example 1.

**Table 3. Compounds of the present disclosure**

| Compound No. and molecular structure thereof | |
|---|---|
| 1# | H-YAibEGTFTSDYSIYK(OEG-OEG-γGlu-C20-OH)DKIAAQEFVNWLIAGGPSSGAPPPS- NH2 |
| 2# | H-YAibEGTFTSDYSIYK(OEG-OEG-γGlu-C20-OH)DRIAAQEFVNWLIAGGPSSGAPPPS- NH2 |
| 3# | H-YAibEGTFTSDYSIYK(OEG-OEG-γGlu-C20-OH)DKIAAQEFINWLIAGGPSSGAPPPS- NH2 |
| 4# | H-YAibEGTFTSDYSIYK(OEG-OEG-γGlu-C20-OH)DRIAAQEFINWLIAGGPSSGAPPPS- NH2 |
| 5# | H-YAibEGTFTSDYSIYK(OEG-OEG-γGlu-C20-OH)DKIAAQEFINWLLAGGPSSGAPPPS- NH2 |
| 6# | H-YAibEGTFTSDYSIYK(OEG-OEG-γGlu-C20-OH)DRIAAQEFVNWLLAGGPSSGAPPPS- NH2 |
| 7# | H-YAibEGTFTSDYSIYK(OEG-OEG-γGlu-C20-OH)DKIAAQEFVNWLLAGGPSSGAPPPS- NH2 |
| 8# | H-YAibEGTFTSDYSIYLEK(OEG-OEG-γGlu-C20-OH)IAAQEFVNWLLAGGPSSGAPPPS- NH2 |
| 9# | H-YAibEGTFTSDYSIYLEK(OEG-OEG-γGlu-C20-OH)IAAQEFVNWLIAGGPSSGAPPPS- NH2 |
| 10# | H-YAibEGTFTSDYSIYLEK(OEG-OEG-γGlu-C20-OH)IAAQEFINWLIAGGPSSGAPPPS- NH2 |
| 11# | H-YAibEGTFTSDYSIYLEK(OEG-OEG-γGlu-C20-OH)IAAQEFINWLLAGGPSSGAPPPS- NH2 |
| 12# | H-YAibEGTFTSDYSIYK(OEG-OEG-γGlu-C20-OH)EKIAAQEFVNWLIAGGPSSGAPPPS- NH2 |
| 13# | H-YAibEGTFTSDYSIYK(OEG-OEG-γGlu-C20-OH)ERIAAQEFVNWLIAGGPSSGAPPPS- NH2 |
| 14# | H-YAibEGTFTSDYSIYK(OEG-OEG-γGlu-C20-OH)EKIAAQEFINWLIAGGPSSGAPPPS- NH2 |
| 15# | H-YAibEGTFTSDYSIYK(OEG-OEG-γGlu-C20-OH)ERIAAQEFINWLIAGGPSSGAPPPS- NH2 |
| 16# | H-YAibEGTFTSDYSIYK(OEG-OEG-γGlu-C20-OH)EKIAAQEFINWLLAGGPSSGAPPPS- NH2 |
| 17# | H-YAibEGTFTSDYSIYK(OEG-OEG-γGlu-C20-OH)ERIAAQEFVNWLLAGGPSSGAPPPS- NH2 |

The purity of the compounds was determined by a combination of analytical high performance liquid chromatography and ultra-high performance liquid chromatography/mass spectrometry, with the purity of some of the compounds shown in Table 4 below:

**Table 4. Purity and molecular weight of compounds 8# to 11# determined by combination of analytical high performance liquid chromatography and liquid chromatography/mass spectrometry**

| Compound No. | Purity |
|---|---|
| 8# | 96.30% |
| 9# | 93.28% |
| 10# | 94.56% |
| 11# | 92.18% |

### Biological Evaluation

The present disclosure is further described and explained below with reference to test examples, but these examples are not intended to limit the scope of the present disclosure.

### Example 3. Evaluation of Agonist Activity of Compounds of the Present Disclosure Against Glucagon-Like Peptide-1 Receptor (GLP-1R)

### 1. Experimental objective:

This test example was intended to determine agonist activity of the compounds of the present disclosure against the glucagon-like peptide-1 receptor (GLP-1R).

### 2. Experimental procedures:

Cryopreserved CHO-K1/GLP-1R/CRE-luc stable cell strains (which can be prepared by conventional methods in the art) were taken out of a liquid nitrogen tank, rapidly thawed in a water bath at 37 °C, resuspended in a DMEM/F12 medium, and centrifuged, and the cells were washed once, resuspended in an assay buffer, i.e., DMEM/F12 medium containing 0.1% casein, subjected to the adjustment of cell density with the assay buffer, and seeded in a 384-well plate (Sigma Cat# CLS4514) at a density of 2500 cells/5 µL/well. Then 2.5 µL of an IBMX working solution (Sigma Cat# I7018) prepared in a buffer (the final concentration of IBMX was 0.5 mM) and 2.5 µL of polypeptide samples diluted in a gradient were added to each well, and the plate was centrifuged at 1000 rpm for 1 min, shaken for 30 s for mixing well, and left to stand for incubation at room temperature for 30 min. Detection was performed using the Cisbio cAMP-Gs Dynamic kit (Cisbio Cat# 62AM4PEC), and cAMP-d2 and Anti-cAMP-Eu³⁺-Cryptate were each subjected to a 20-fold dilution using cAMP Lysis & Detection Buffer and each mixed well. 5 µL of diluted cAMP-d2 solution was added to each well, followed by the addition of 5 µL of diluted Anti-cAMP-Eu³⁺-Cryptate solution, and the mixture was shaken for 30 s for mixing well and then incubated at room temperature for 1 h in the dark.

### 3. Data processing:

HTRF signal reading was performed using a Biotek Synergy H1 microplate reader at an excitation wavelength of 320 nm and emission wavelengths of 620 nm and 665 nm. The signal ratios (665 nm/620 nm* 10,000) were calculated and fitted non-linearly to sample concentrations in GraphPad Prism 6 using a four-parameter equation to obtain EC₅₀ values, with the specific data shown in Table 5 below.

### Example 4. Evaluation of Agonist Activity of Compounds of the Present Disclosure Against Glucose-dependent Insulinotropic Polypeptide Receptor (GIPR)

### 1. Experimental objective:

This example was intended to determine agonist activity of the compounds of the present disclosure against the glucose-dependent insulinotropic polypeptide receptor (GIPR).

### 2. Experimental procedures:

Wild-type CHO-K1 cells were collected, and the cell suspension was adjusted to an appropriate density, seeded in a 6-well plate at 2 mL/well, and placed in an incubator at 37 °C with 5% CO₂ for adherence culture overnight. The transfection mixture (hGIP receptor plasmid, Fugene HD (Promega Cat# E2311), and OptiMEM (Gibco Cat# 31985070)) was mixed well, left to stand at room temperature for 15 min, and added to the corresponding cell wells in a volume of 100 µL, followed by transfection for 24 h to enable the overexpression of the hGIP receptor on the surface of CHO-K1 cells. After the transient transfection was completed, the cells in the 6-well plate were collected, washed once with an assay buffer, i.e., DMEM/F12 medium (Gibco Cat# 11330032) containing 0.1% casein (Sigma Cat# C3400), subjected to the adjustment of cell density with the assay buffer, and seeded in a 384-well plate (Sigma Cat# CLS4514) at a density of 5000 cells/5 µL/well. Then 2.5 µL of an IBMX working solution (Sigma Cat# I7018) prepared in a buffer (the final concentration of IBMX was 0.5 mM) and 2.5 µL of polypeptide samples diluted in a gradient were added to each well, and the plate was centrifuged at 1000 rpm for 1 min, shaken for 30 s for mixing well, and left to stand for incubation at room temperature for 30 min. Detection was performed using the Cisbio cAMP-Gs Dynamic kit (Cisbio Cat# 62 AM4PEC), and cAMP-d2 and Anti-cAMP-Eu3+-Cryptate were each subjected to a 20-fold dilution using cAMP Lysis & Detection Buffer and each mixed well. 5 µL of diluted cAMP-d2 solution was added to each well, followed by the addition of 5 µL of diluted Anti-cAMP-Eu3+-Cryptate solution, and the mixture was shaken for 30 s for mixing well and then incubated at room temperature for 1 h in the dark.

### 3. Data processing:

HTRF signal reading was performed using a Biotek Synergy H1 microplate reader at an excitation wavelength of 320 nm and emission wavelengths of 620 nm and 665 nm. The signal ratios (665 nm/620 nm* 10,000) were calculated and fitted non-linearly to sample concentrations in GraphPad Prism 6 using a four-parameter equation to obtain EC₅₀ values, with the specific data shown in Tables 5 and 6 below.

**Table 5. Determination results of agonist activity against human GLP-1R and human GIPR**

| Compound | Activity against human GLP-1R (EC₅₀ nM) | Activity against human GIPR (EC₅₀ nM) |
|---|---|---|
| Natural GLP-1 | 0.010 | N/A |
| Natural GIP | N/A | 0.011 |
| Semaglutide | 0.024 | >10 |
| LY3298176 | 0.13 | 0.056 |
| 7# | 0.021 | 0.11 |

**Table 6. Determination results of agonist activity against human GLP-1R and human GIPR**

| Compound | Activity against human GLP-1R (EC₅₀ nM) | Activity against human GIPR (EC₅₀ nM) |
|---|---|---|
| Natural GLP-1 | 0.006 | N/A |
| Natural GIP | N/A | 0.006 |
| Semaglutide | 0.014 | >10.0 |
| LY3298176 | 0.078 | 0.031 |
| 9# | 0.049 | 0.040 |
| 10# | 0.065 | 0.056 |
| 12# | 0.030 | 0.170 |
| 13# | 0.017 | 0.130 |
| 14# | 0.013 | 0.130 |
| 15# | 0.015 | 0.230 |
| 16# | 0.029 | 0.095 |
| 17# | 0.022 | 0.110 |
| 18# | 0.013 | 0.060 |

### 4. Experimental conclusion:

Through the design of the polypeptide skeleton and the subsequent site-directed fatty acid modification, the compounds of the present disclosure have stronger agonist activity against GLP-1/GIPR than many GLP-1/GIPR dual-agonist polypeptides in the art and thus have greater potential for treating metabolic diseases. In addition, LY3298176 shows preferential activity against GIPR, while the compounds 12#-18# of the present disclosure show preferential activity against GLP-1R.

### Example 5. Stability Test of Some of Compounds of the Present Disclosure

Stability in plasma is important for therapeutic polypeptide drugs, since the polypeptide drugs are likely to be sensitive to polypeptide hydrolases and protein hydrolases in plasma. The half-life and efficacy of polypeptides that are unstable in plasma will be affected.

### 1. Experimental objective:

This experiment was intended to test the stability of some of the compounds of the present disclosure in human plasma.

### 2. Experimental procedures:

5 µL of each of samples at concentrations of 20 ng/mL, 50 ng/mL, 100 ng/mL, 200 ng/mL, 500 ng/mL, 1000 ng/mL, 2000 ng/mL, 5000 ng/mL, and 10000 ng/mL was added to 45 µL of human plasma. The content of the compounds in the samples was determined by the LC-MS method and a standard curve was formed. 5 µL of a 1 mg/mL polypeptide solution was added to 45 µL of human plasma. Five samples were prepared for each test compound, and the samples were taken at 0 min, 30 min, 60 min, 120 min, and 240 min, respectively, and determined for the content of the retained compound by the LC-MS method. With the content at 0 min as the standard (100%), the relative content of the retained compounds in the samples at other time points was calculated. The LC-MS method for detecting the compounds was as follows: a 5% acetonitrile solution was prepared as solution A, a 95% acetonitrile solution was prepared as solution B, solution gradients were formed at a flow rate of 0.6 mL/min according to the time points and solution proportions shown in Table 9, and 15 µL of the sample was injected, and the content of the compounds was determined using a Raptor Biphenyl 2.7 µm detection column; see Table 7.

**Table 7. Test time points and solution proportions**

| Time (min) | A (%) | B (%) |
|---|---|---|
| 0.20 | 95.0 | 5.00 |
| 1.70 | 5.00 | 95.0 |
| 2.00 | 5.00 | 95.0 |
| 2.01 | 95.0 | 5.00 |
| 2.50 | 95.0 | 5.00 |

### 3. Experimental results:

The data for the stability of some of the compounds of the present disclosure in plasma are shown in Table 8 below.

**Table 8. Experimental results of the stability of the compounds in plasma**

| Compound | Relative content of compounds retained in plasma (%) | | | | |
|---|---|---|---|---|---|
| | 0 min | 30 min | 60 min | 120 min | 240 min |
| LY3298176 | 100.00 | 102.89 | 114.76 | 117.59 | 113.35 |
| 7# | 100.00 | 101.45 | 101.66 | 103.28 | 102.15 |

| | | | | | |
|---|---|---|---|---|---|
| Conclusion: | | | | | |

It was found by study that compound 7# of the present disclosure has similar stability (relative content > 90%) in human plasma compared with compound LY3298176 at the 4-h time point.

### Example 6. Pharmacokinetic Properties of Some of Compounds of the Present Disclosure in Mice

Plasma stability is one of the factors that affect the pharmacokinetics of polypeptide drugs. The pharmacokinetics of polypeptide drugs *in vivo* is also affected by factors such as absorption and clearance of the polypeptide drugs *in vivo.*

### 1. Experimental objective:

This experiment was intended to study the pharmacokinetic behavior of the compounds of the present disclosure in Balb/c mice (plasma) after a single intravenous injection by taking the mice as test animals.

### 2. Experimental procedures:

Male Balb/c mice weighing 18-30 g and aged 7-9 weeks were purchased from Shanghai Jiesijie Laboratory Animal Co., Ltd. After compound 7# was prepared in a buffer containing 20 mM citric acid (pH = 7.0), compound 7# was intravenously injected into mice at a dose of 30 nmol/kg body weight via tail vein, and 0.2 mL of blood was separately collected at time points of 0 h, 0.083 h, 0.25 h, 0.5 h, 1 h, 2 h, 4 h, 6 h, 8 h, 24 h, and 32 h. The collected blood of mice was centrifuged at 6000 rpm for 6 min at 4 °C to separate the plasma. The content of compound 7# in plasma of mice was assayed by the experimental procedures of Example 3.3.

### 3. Experimental results:

Through the above experimental procedures, the specific data are shown in Table 9 below.

**Table 9. Pharmacokinetic behavior of a single intravenous injection in mice (plasma)**

| PK parameters | Unit | Compound 7# |
|---|---|---|
| T_{1/2} | h | 13.0 |
| AUC_{Inf} | h*ng/mL | 16133 |

### 4. Experimental conclusion:

It was found by study that compound 7# of the present disclosure has good pharmacokinetic properties after intravenous injection into mice, indicating that this compound is advantageous in treating diseases, for example, being able to support subcutaneous injection once a week in humans.

### Example 7. Pharmacokinetic Properties of Some of Compounds of the Present Disclosure in Mice

### 1. Experimental objective:

This experiment was intended to study the pharmacokinetic behavior of the compounds of the present disclosure in Balb/c mice (plasma) after a single subcutaneous injection by taking the mice as test animals.

### 2. Experimental procedures:

Male Balb/c mice weighing 18-30 g and aged 7-9 weeks were purchased from Shanghai Jiesijie Laboratory Animal Co., Ltd. After compound 7# was prepared in a buffer containing 20 mM citric acid (pH = 7.0), compound 7# was subcutaneously injected into mice at a dose of 30 nmol/kg body weight via left side of abdomen, and 0.2 mL of blood was separately collected at time points of 0 h, 0.083 h, 0.25 h, 0.5 h, 1 h, 2 h, 4 h, 6 h, 8 h, 24 h, and 32 h. The collected blood of mice was centrifuged at 6000 rpm for 6 min at 4 °C to separate the plasma. The content of compound 7# in plasma of mice was assayed by the experimental procedures of Example 5.2.

### 3. Experimental results:

Through the above experimental procedures, the specific data are shown in Table 10 below.

**Table 10. Pharmacokinetic results for compound 7# in mice**

| PK parameters | Unit | Compound 7# |
|---|---|---|
| T_{1/2} | h | 10.1 |
| AUC_{Inf} | h*ng/mL | 14488 |

### 4. Experimental conclusion:

It was found by study that the compound of the present disclosure has good pharmacokinetic properties after subcutaneous injection into mice, indicating that this compound is advantageous in treating diseases, for example, being able to support subcutaneous injection once a week in humans.

### Example 8. In Vivo Efficacy of Some of Compounds of the Present Disclosure

### 1. Experimental objective:

This experiment was intended to test the regulatory effect of some of the compounds of the present disclosure and compound LY3298176 on blood glucose in normal mice after a single subcutaneous administration.

### 2. Experimental procedures:

Male C57BL/6 mice aged 10-12 weeks were purchased from Shanghai Jiesijie Laboratory Animal Co., Ltd. The C57BL/6 mice were subcutaneously injected with compound 7# or compound LY3298176 (dose: 10 nmol/kg body weight) and a control buffer, and then fasted without water deprivation. 18 h later, a glucose solution at a concentration of 0.2 g/mL was intraperitoneally injected. Blood glucose values were measured by collecting blood from the tail of mice at time points of 0 min, 15 min, 30 min, 60 min, and 120 min according to the experimental design. The specific procedures were as follows: the mouse was physically immobilized with the tail exposed, a little part was cut off at the tail end, then the tail was squeezed to bleed, and blood glucose was determined using a Roche active glucometer after the 1st drop of blood was discarded. The area under the blood glucose curve (AUC) was calculated from the results of all time points.

### 3. Experimental results:

Through the above experimental procedures, the specific data are shown in Table 11 below.

**Table 11. Change in blood glucose values of mice after a single subcutaneous administration**

| Test compounds | Dose | Blood glucose (mMol/L, mean±SD) | | | | | AUC (mMol/L.hr) |
|---|---|---|---|---|---|---|---|
| | | 0 min | 15 min | 30 min | 60 min | 120 min | |
| Placebo | - | 5.3±0.6 | 20.5±2.0 | 24.0±1.4 | 19±1.3 | 10.9±1.2 | 34.5±2.4 |
| 7# | 10nmol/kg | 4.4±0.8 | 6.7±0.8 | 6.2±1.3 | 5.7±1.2 | 3.8±1.1 | 10.7±1.8 |
| LY3298176 | 10nmol/kg | 3.2±0.2 | 9.1±1.3 | 8±1.4 | 6.4±1.0 | 4.5±0.7 | 12.7±1.6 |

### 4. Experimental conclusion:

In this experiment, compound 7# of the present disclosure shows significant blood glucose-lowering effect on normal mice at a dose of 10 nmol/kg body weight, with the area under the blood glucose curve of compound 7# group reduced by more than 60% compared with that of placebo (i.e., blank vehicle).

### Example 9. Body Weight-Reducing Efficacy of Some of Compounds of the Present Disclosure

### 1. Experimental objective:

This experiment was intended to test the regulatory effect of the numbered compounds on the body weight of diet-induced-obesity mice after subcutaneous administration.

### 2. Experimental procedures:

High-fat food-induced-obesity male C57BL/6 mice (weighing 35-55 g, aged 10-12 weeks, purchased from Shanghai Jiesijie Laboratory Animal Co., Ltd.) were tested. The diet-induced-obesity C57BL/6 mice were subcutaneously injected with compound LY3298176 (10 nmol/kg body weight), compound 7# (10 nmol/kg body weight), and compound 18# (three doses of 3 nmol/kg, 10 nmol/kg, and 100 nmol/kg body weight, administered once every 3 days). According to the experimental design, the body weight of each mouse was measured and recorded on day 0, day 3, day 6, and so on to day 27, the average body weight of each group of mice was calculated, and body weight change curves were plotted by taking the body weight on the first day as the standard. At the end, the fat and other visceral organs of each part of the mice were taken out and weighed, and the viscera/brain ratio for fat in each part of each mouse was calculated. The effect of the drug on the fat was determined by comparing the change in the viscera/brain ratio for fat of different parts of each group of mice.

### 3. Experimental results:

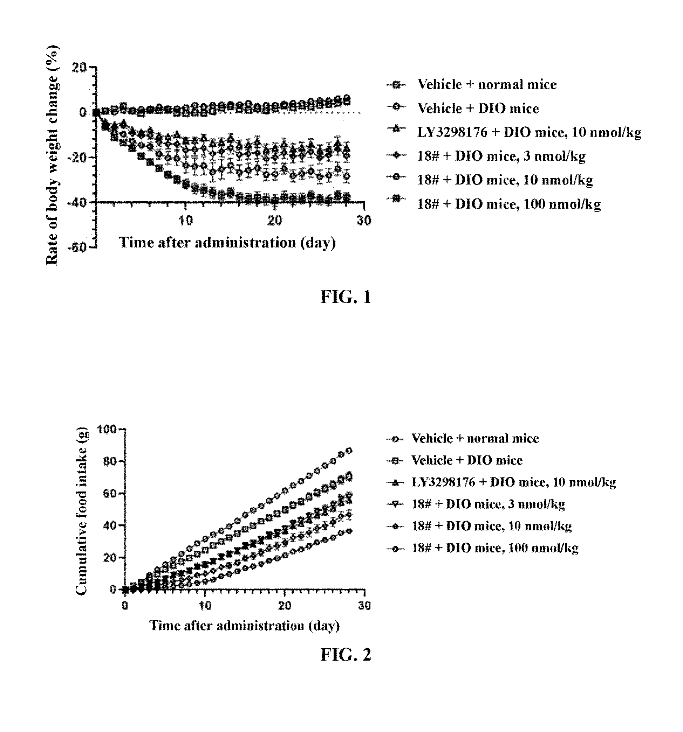

Through the above experimental procedures, the specific data are shown in Tables 12 to 14 below and FIG. 1.

**Table 12. Body weight-reducing effect of compounds on induced-obesity mice**

| | Days | Test compound (dose) | |
|---|---|---|---|
| | | Placebo (-) | 7# (10 nmol/kg) |
| Body weight change (%, mean±SD) | Day 1 | 0 | 0 |
| | Day 4 | -0.9±1.6 | -11.0±1.0 |
| | Day 7 | -2.6±1.5 | -17.6±2.6 |
| | Day 10 | -3.0±2.4 | -22.6±5.9 |
| | Day 13 | -3.5±3.7 | -22.3±6.3 |
| | Day 16 | -2.7±4.8 | -22.7±6.9 |
| | Day 19 | -2.8±6.7 | -25.9±6.2 |
| | Day 22 | -2.5±8.4 | -23.9±5.6 |
| | Day 25 | -1.4±8.9 | -23.9±5.6 |
| | Day 28 | -2.1±9.6 | -25.5±5.3 |

### 4. Experimental conclusion:

In this experiment, at the doses of 3 nmol/kg, 10 nmol/kg, and 100 nmol/kg, the compounds 7# and 18# of the present disclosure show significant body weight-reducing effect on high-fat food-induced-obesity mice and exhibit significant dose dependence. The body weight of mice in 10 nmol/kg dose test group of the compound 18# was reduced by more than 20.0% on day 27, while the body weight of mice in the same dose test group of the control compound LY3298176 was reduced by about 13.4%. In addition, the content of fat of each part (except scapular fat) of mice in all dose test groups of the compound 18# was significantly reduced relative to that of the placebo (i.e., blank vehicle) group.

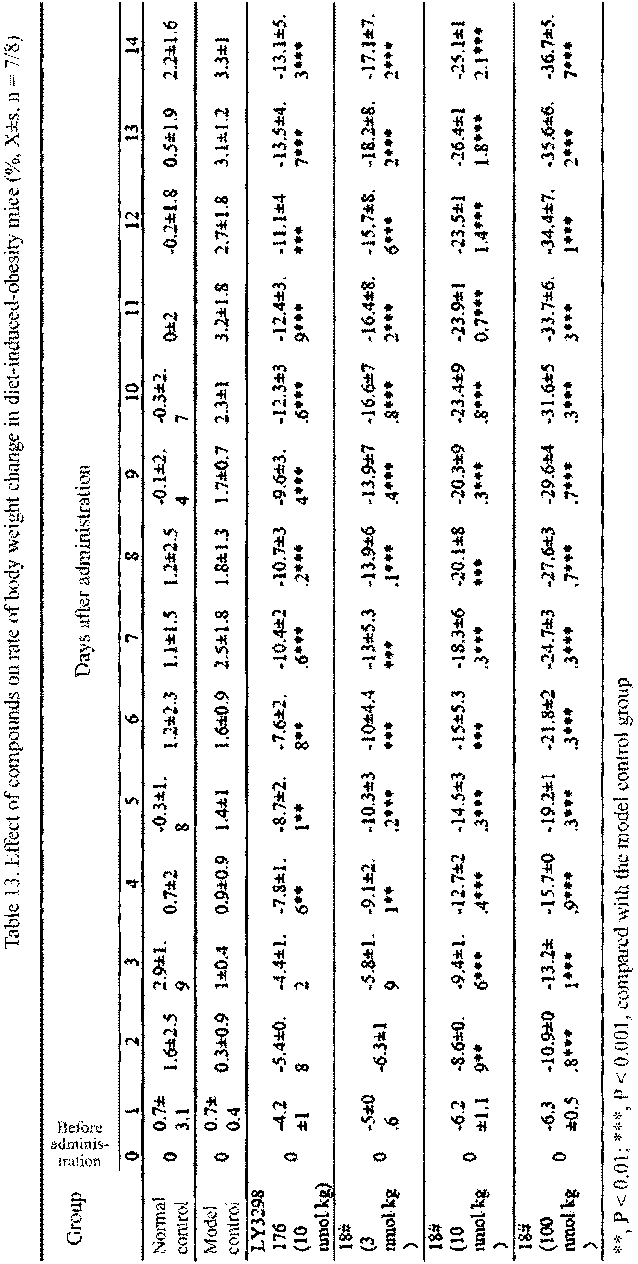

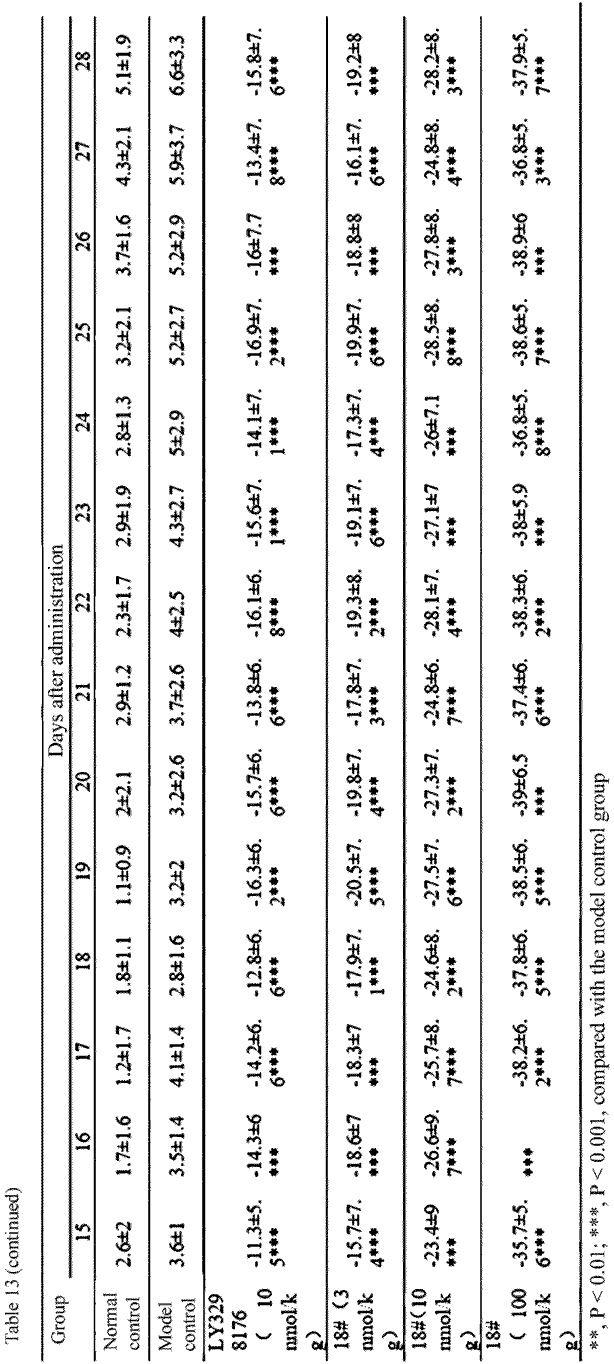

**Table 14. Compounds on the change of the viscera/brain ratio for fat mass in different parts of diet-induced-obesity mice (%, X±s, n = 7-8)**

| Group | Scapular fat | Subcutaneous fat | Inguinal fat | Mesenteric fat | Perirenal fat | Epididymal fat |
|---|---|---|---|---|---|---|
| Model control | **51.6±31.8** | **320.2±54.2** | **510.2±104.3** | **220.3±68.6** | **306.2±67.7** | **384.1±61.1** |
| **LY3298176 (10nmol/kg)** | **52±26** | **144.6±75.8***** | **289.1±169.2**** | **105.3±93**** | **163.2±57.9***** | **316.9±75.6** |
| **18# (3 nmol/kg)** | **48±15.3** | **129±62***** | **281.5±140.1**** | **84.8±41.3***** | **159.2±87.6***** | **254.6±113.4*** |
| **18# (10 nmol/kg)** | **42.5±17** | **122±73.2***** | **194.4±86.2***** | **53.3±14.6***** | **109.4±57.1***** | **211.7±80.7**** |
| **18# (100 nmol/kg)** | **32.3±7** | **57.1±21.2***** | **92±34.5***** | **27.4±11.1***** | **46.6±18.2***** | **102.9±32.2***** |

| | | | | | | |
|---|---|---|---|---|---|---|
| *, P < 0.05; **, P < 0.01; ***, P < 0.001, compared with the model control group | | | | | | |

### Example 10. Effect of Compounds of the Present Disclosure on Food Intake of Mice

The food intake of mice in each group was measured daily during the experiment. The results are shown in Table 1 and FIG. 2.

The average daily food intake of DIO (diet-induced obesity) mice in the model control group was 2.5 g throughout the experiment. After subcutaneous injection of the compound 18# or compound LY3298176 at different doses, the food intake of mice in all groups was reduced to different extents.

On the first day after the administration, the food intake of mice in each administration group was significantly reduced, with the food intake of mice in 3 nmol/kg, 10 nmol/kg, and 100 nmol/kg dose groups of the compound 18# being 0.6 g, 0.3 g, and 0.2 g, respectively, which was significantly different from that of the model control group (2.5 g) and showed a better dose-effect relationship.

The cumulative food intake of the mice in the model control group within 5 days after the administration was 12.8 g, while the cumulative food intake of the mice in the 3 nmol/kg, 10 nmol/kg, and 100 nmol/kg dose groups of the compound 18# within 5 days after the administration was 7.2 g, 3.9 g, and 1.8 g, respectively, which was significantly lower than that of the model control group and showed a better dose-effect relationship.

Daily food intake of mice in each administration group began to decrease on day 1 and began to restore on days 2 and 3 after each administration. Daily food intake showed an overall upward recovery trend during the administration. 28 days after the administration, the cumulative food intake of three dose groups of the compound 18# was 58.2 g, 46.8 g, and 36.7 g, respectively, which was significantly lower than that of the model control group (70.8 g) and showed a better dose dependence. Therefore, the compound 18# can significantly reduce food intake of DIO mice.

**Table 15. Effect of long-term administration of compound 18# on daily food intake of DIO mice (g, X±s, n = 7-8)**

| Group | Days after administration | | | | | | | | | | | | | |
|---|---|---|---|---|---|---|---|---|---|---|---|---|---|---|
| | 1 | 2 | 3 | 4 | 5 | 6 | 7 | 8 | 9 | 10 | 11 | 12 | 13 | 14 |
| Normal control | 1±1.4 | 4±0.5 | 4.1±0. 5 | 3.6±0. 8 | 3.2±1 | 3.3±0. 5 | 3.4±0. 4 | 3.3±0. 4 | 3.3±0. 7 | 2.8±0. 4 | 2.9±1. 1 | 2.2± 1.1 | 2.8±0. 3 | 3.4± 0.5 |
| Model control | 2.5±0. 4 | 2.4±0. 4 | 2.6±0. 3 | 2.3±0. 3 | 2.8±0. 3 | 2.4±0. 5 | 2.4±0. 3 | 2.3±0. 3 | 2.5±0. 4 | 2.5±0. 4 | 2.8±0. 4 | 2.4± 0.3 | 2.6±0. 5 | 2.5± 0.3 |
| LY3298176(10 nmol/kg) | 0.8±0. 2*** | 1.2±0. 2*** | 2.3±0. 3 | 0.9±0. 3*** | 1.8±0. 3*** | 2.3±0. 3 | 1.2±0. 3*** | 1.7±0. 4 | 2.7±0. 3 | 1.2±0. 4*** | 2±0.3* | 2.8± 0.4 | 1.4±0. 6*** | 2±0. 5 |
| Compound 18# (3 nmol/kg) | 0.6±0. 1*** | 1.3±0. 5*** | 2±0.5 | 1.1±0. 4*** | 2.2±1. 3 | 2.1f1. 1 | 1.1±0. 6*** | 1.6±0. 6* | 2.5±0. 9 | 1.3±0. 6*** | 2±0.3* | 3±1 | 1.8±1* | 2.3± 1 |
| Compound 18# (10 nmol/kg) | 0.3±0. 2*** | 0.6±0. 6*** | 1.1±0. 7*** | 0.7±0. 5*** | 1.1±0. 5*** | 1.5±0. 8** | 0.8±0. 4*** | 1.1±0. 7*** | 2±0.6 | 0.9±0. 4*** | 1.8±0. 8** | 2.4± 0.6 | 0.9±0. 4*** | 1.7± 0.7* |
| Compound 18# (100 nmol/kg) | 0.2±0. 1*** | 0.1±0. | 0.4±0. 1*** | 0.4±0. 2*** | 0.6±0. 2*** | 0.7±0. 3*** | 0.5±0. 2*** | 0.7±0. 5*** | 0.9±0. 6*** | 0.9±0. 6*** | 1.1±0. 5*** | 2±1 | 1.3±0. 7*** | 1.7± 0.5* |
| *, P < 0.05; **, P < 0.01; ***, P < 0.001, compared with the model control group | | | | | | | | | | | | | | |

**Table 15 (continued)**

| Group | Days after administration | | | | | | | | | | | | | |
|---|---|---|---|---|---|---|---|---|---|---|---|---|---|---|
| | 15 | 16 | 17 | 18 | 19 | 20 | 21 | 22 | 23 | 24 | 25 | 26 | 27 | 28 |
| Normal control | 3.7± 0.7 | 3±0.4 | 2.6±0. 2 | 3.3± 0.3 | 3.1±0.3 | 3.3± 0.5 | 3.3± 0.2 | 2.7±0. 4 | 3.3±0. 3 | 3.1± 0.3 | 3.3±0.8 | 2.8±0. 8 | 4.1±0. 3 | 2.5±0. 3 |
| Model control | 2.7± 0.3 | 2.1±0. 2 | 2.7±0. 2 | 2.3± 0.4 | 2.5±0.5 | 2.4± 0.3 | 2.9± 0.3 | 2.3±0. 2 | 2.9±0. 4 | 2.6± 0.5 | 2.6±0.3 | 2.4±0. 4 | 3±0.5 | 2.1±0. 3 |
| LY3298176(10n mol/kg) | 3±0. 6 | 1.2±0. 4** | 2.1±0. 4 | 2.9± 1.1 | 1.2±0.8 *** | 2±0. 4 | 3.3± 0.3 | 1.4±0. 4** | 2.5±0. 5 | 2.8± 0.4 | 1.8±0.6 | 2.4±0. 4 | 3.9±0. 5 | 1.4±0. 3 |
| Compound 18# (3 nmol/kg) | 2.9± 0.7 | 1.5±0. 7 | 2.2±0. 5 | 2.7± 0.3 | 1.7±0.5 * | 2.2± 0.5 | 3.4± 0.4 | 1.8±0. 5 | 2.6±0. 4 | 2.9± 0.5 | 1.8±0.8 * | 2.5±0. 9 | 3.9±0. 5** | 1.3±0. 6* |
| Compound 18# (10 nmol/kg) | 3±0. 9 | 1.2±1* * | 2.1±0. 9 | 2.9± 0.8 | 1.3±0.5 *** | 2±0. 5 | 3.2± 0.6 | 1±0.4* ** | 2.3±0. 5 | 2.5± 0.6 | 1.6±1.2 ** | 2.2±0. 8 | 3.6±1 | 1±0.5 *** |
| Compound 18# (100 nmol/kg) | 2±0. 4 | 1±0.3* * | 1.6±0. 3** | 2±0. 4 | 1.4±0.2 ** | 1.8± 0.3 | 2.4± 0.3 | 1.4±0. 5** | 2.1±0. 5* | 2.3± 0.4 | 1.5±0.3 *** | 1.6±0. 4* | 2.8±0. 3 | 1.2±0. 4* |
| *, P < 0.05; **, P < 0.01; ***, P < 0.001, compared with the model control group | | | | | | | | | | | | | | |

### Example 11. Improvement Effect of Some of Compounds of the Present Disclosure on Glucose Metabolism Level of db/db Mice

### 1. Experimental objective:

This experiment was intended to test the improvement effect of the numbered compound on the glucose metabolism level of db/db mice after subcutaneous administration.

### 2. Experimental procedures:

C57BL/KsJ-db/db mice were subcutaneously injected with blank vehicle (20 mM sodium citrate + 0.05% Tween-80, pH 7.5), compound LY3298176 (100 nmol/kg body weight), and compound 18# (three doses of 10 nmol/kg body weight, 30 nmol/kg body weight, and 100 nmol/kg body weight) on days 0, 3, 7, 10, 14, 17, 21, 24, and 27. Each administration group had 10 db/db mice. According to the experimental design, tail vein blood was collected by needle pricking on days 0, 7, 14, 21, and 28 and determined for fasting blood glucose levels with a glucometer and glucose dipsticks, and the mice were fasted 6 h prior to the blood collection at each time point. Tail vein blood was collected by needle pricking on days 3, 10, 17, 24, and 27 and randomly determined for blood glucose levels with a glucometer. Finally, at the end of the experiment on day 28, all the animals in the administration groups were subjected to 2-5% isoflurane inhalation anesthesia, and 100 µL of whole blood was collected through the orbit of each mouse using an EDTA-K2 anticoagulation tube and used for the determination of glycated hemoglobin.

### 3. Experimental results:

Through the above experimental procedures, the specific data are shown in Tables 16 to 18 below.

**Table 16. Effect of long-term administration of compound 18# on fasting blood glucose of db/db mice**

| Administration group | Concentration of fasting blood glucose (mMol/L, mean±SD) | | | | |
|---|---|---|---|---|---|
| | Day 0 | Day 7 | Day 14 | Day 21 | Day 28 |
| Blank control | 14.25±1.27 | 17.92±1.33 | 22.89±1.88 | 24.95±1.52 | 25.94±1.32 |
| LY3298176 (100 nmol/kg) | 14.35±1.41 | 7.44±0.76 | 7.56±0.88 | 9.42±1.67 | 9.89±1.28*** |
| #18 (10 nmol/kg) | 14.77±1.30 | 6.05±0.42 | 6.30±0.46 | 7.89±0.81 | 9.41±0.97*** |
| #18 (30 nmol/kg) | 14.13±1.32 | 6.21±0.26 | 6.40±0.57 | 7.03±0.52 | 9.68±1.03*** |
| #18 (100 nmol/kg) | 14.67±1.46 | 5.85±0.33 | 6.25±0.32 | 6.13±0.19 | 7.89±0.41*** |

| | | | | | |
|---|---|---|---|---|---|
| ***: p < 0.001 vs. blank control group. | | | | | |

**Table 17. Effect of long-term administration of compound 18# on random blood glucose of db/db mice**

| Administration group | Concentration of random blood glucose (mMol/L, mean±SD) | | | |
|---|---|---|---|---|
| | Day 0 | Day 10 | Day 17 | Day 24 |
| Blank control | 23.06±0.97 | 26.40±0.90 | 27.64±1.15 | 30.22±0.74 |
| LY3298176 (100 nmol/kg) | 18.60±1.52 | 17.10±1.96 | 17.98±1.37 | 20.70±1.27*** |
| #18 (10 nmol/kg) | 20.42±1.56 | 20.66±1.48 | 18.91±1.33 | 21.17±2.07** |
| #18 (30 nmol/kg) | 16.73±1.59 | 15.88±1.86 | 17.30±1.17 | 17.43±1.92*** |
| #18 (100 nmol/kg) | 9.11±1.25 | 12.34±1.12 | 11.89±1.15 | 11.51±0.95*** |

| | | | | |
|---|---|---|---|---|
| **: p < 0.01 vs. blank control group; ***: p < 0.001 vs. blank control group. | | | | |

**Table 18. Effect of long-term administration of compound 18# on glycated hemoglobin level of db/db mice**

| Administration group | Glycated hemoglobin (%, mean±SD) |
|---|---|
| Blank control | 6.54±0.17 |
| LY3298176 (100 nmol/kg) | 4.58±0.23** |
| #18 (10 nmol/kg) | 4.71±0.23*** |
| #18 (30 nmol/kg) | 4.53±0.17*** |
| #18 (100 nmol/kg) | 3.78±0.13*** |

| | |
|---|---|
| **: p < 0.01 vs. blank control group; ***: p < 0.001 vs. blank control group. | |

### 4. Experimental conclusion:

In this experiment, at the doses of 10 nmol/kg, 30 nmol/kg, and 100 nmol/kg, the compound 18# of the present disclosure shows excellent improvement effect on the glucose metabolism level of db/db mice and shows significant dose dependence. The glycated hemoglobin level of the 100 nmol/kg dose group of the compound 18# was 3.78% at the end of the experiment, while the glycated hemoglobin level of the same dose group of the control compound LY3298176 was 4.58%. Therefore, the efficacy of the compound 18# in improving the glucose metabolism level of db/db mice is significantly better than that of the control compound LY3298176 at the same dose.

GLP-1 analogs or APIs used in the following examples are all compound **18#.**

### Example 12. Preparation and Test of Pharmaceutical Compositions

The pharmaceutical compositions of the present disclosure were prepared by dry granulation according to the following process: API (compound **18#** shown in FIG. 3) and other ingredients each at an amount specified in the formulation were mixed well, subjected to dry granulation together, and compressed into tablets.

Dissolution test: based on the dissolution test method, taking 500 mL of phosphate buffer solution (pH 6.8) containing 0.1% Tween 80 as dissolution medium, and setting the rotating speed to 50rpm, the operations were performed as required. At each of the time points of 5 min, 10 min, 15 min, 30 min, and 45 min, 5 mL of solution was collected and 5 mL of fresh solution was added for supplementation. Each dissolution liquid was filtered through a 10 µm polyethylene filter head and then filtered through a 0.22 µm Millipore PVDF filter membrane, and the subsequent filtrate was filled into a liquid phase vial and sent for HPLC detection of the dissolution amount of API and SANC of each tablet at different time points.

**12.1** SNAC, API, microcrystalline cellulose, povidone, and magnesium stearate (intragranular) each at an amount specified in the formulation were mixed well and subjected to dry granulation, and then magnesium stearate (extragranular) was added, and the resulting mixture was mixed well and compressed into tablets. The tablets were subjected to the dissolution test in phosphate buffer (pH 6.8) containing 0.1% tween 80, and the specific formulations and dissolution results are shown in Table 19.

**Table 19. Ingredients and dissolution of compositions**

| Batch | Formulation 1 | Formulation 2 | | |
|---|---|---|---|---|
| Component | Amount mg/tablet | | | |
| API | 5 | 5 | | |
| SNAC | 300 | 150 | | |
| Microcrystalline cellulose | 80 | / | | |
| 101 | | | | |
| Povidone K90 | 8 | | / | |
| Magnesium stearate (intragranular) | 7.7 | | 3.858 | |
| Magnesium stearate (extragranular) | 2 | | / | |

| Time/min | Dissolution/% | | | |
|---|---|---|---|---|
| | API | SNAC | API | SNAC |
| 5 | 9 | 26 | 59 | 57 |
| 10 | / | / | 91 | 88 |
| 15 | 43 | 60 | 104 | 100 |
| 30 | 70 | 83 | 108 | 102 |
| 45 | 82 | 89 | 107 | 103 |
| 60 | 89 | 93 | 108 | 103 |

In the formulations, "/" means not added.

**12.2** SNAC, API, sodium caprylate, colloidal silicon dioxide (intragranular) and magnesium stearate (intragranular) each at an amount specified in the formulation were mixed well and subjected to dry granulation, and then colloidal silicon dioxide and magnesium stearate (extragranular) were added, and the resulting mixture was mixed well and compressed into tablets. The tablets were subjected to the dissolution test in phosphate buffer (pH 6.8) containing 0.1% tween 80, and the specific formulations and dissolution results are shown in Table 20.

**Table 20. Ingredients and dissolution of compositions**

| Batch | Formulation 3 | | Formulation 4 | | Formulation 5 | | Formulation 6 | | Formulation 7 | | Formulation 8 | | Formulation 9 | | Formulation 10 | |
|---|---|---|---|---|---|---|---|---|---|---|---|---|---|---|---|---|
| Component | Amount mg/tablet | | | | | | | | | | | | | | | |
| API | 2.5 | | 2.5 | | 2.5 | | 5 | | 5 | | 10 | | 30 | | 10 | |
| SNAC | 25 | | 75 | | 150 | | 133.856 | | 150 | | 150 | | 150 | | 300 | |
| Sodium caprylate | 2.8 | | 8.5 | | 17 | | 15.35 | | 17 | | 17 | | 17 | | 34 | |
| Colloidal silicon dioxide (intragranular) | 0.165 | | 0.5 | | 1 | | 0.794 | | 1 | | 1 | | 1 | | 2 | |
| Magnesium stearate (intragranular) | 0.42 | | 1.25 | | 2.5 | | 2.268 | | 2.5 | | 2.5 | | 2.5 | | 5 | |
| Colloidal silicon dioxide (extragranular) | 0.165 | | 0.5 | | 1 | | 1.794 | | 1 | | 1 | | 1 | | 2 | |
| Magnesium stearate (extragranular) | 0.33 | | 1 | | 2 | | 1.59 | | 2 | | 2 | | 2 | | 4 | |

| Time/min | Dissolution/% | | | | | | | | | | | | | | | |
|---|---|---|---|---|---|---|---|---|---|---|---|---|---|---|---|---|
| | API | SNA C | API | SNA C | API | SNA C | API | SNA C | API | SNA C | API | SNA C | API | SNA C | API | SNA C |
| 5 | 61 | 69 | 45 | 53 | 31 | 54 | 43 | 48 | 44 | 54 | 18 | 59 | 14 | 34 | 22 | 34 |
| 10 | 96 | 106 | 82 | 91 | 72 | 87 | 78 | 83 | 77 | 87 | 70 | 86 | 52 | 65 | 53 | 63 |
| 15 | 102 | 110 | 94 | 102 | 85 | 101 | 93 | 96 | 91 | 100 | 86 | 99 | 72 | 83 | 70 | 83 |
| 30 | 103 | 110 | 98 | 106 | 92 | 106 | 98 | 99 | 97 | 106 | 94 | 106 | 92 | 105 | 86 | 103 |

**12.3** SNAC, API, amino acids, colloidal silicon dioxide (intragranular), and magnesium stearate (intragranular) each at an amount specified in the formulation were mixed well and subjected to dry granulation, and then magnesium stearate (extragranular) was added, and the resulting mixture was compressed into tablets. The tablets were subjected to the dissolution test in phosphate buffer (pH 6.8) containing 0.1% tween 80, and the specific formulations and dissolution results are shown in Table 21.

**Table 21. Ingredients and dissolution of compositions**

| Batch | Formulation 11 | | Formulation 12 | | Formulation 13 | |
|---|---|---|---|---|---|---|
| Component | Amount mg/tablet | | | | | |
| API | 5 | | 5 | | 5 | |
| SNAC | 133.856 | | 100 | | 133.856 | |
| Arginine | 15.35 | | 60 | | / | |
| Histidine | / | | / | | 15.35 | |
| Colloidal silicon dioxide (intragranular) | 0.794 | | 0.845 | | 0.794 | |
| Magnesium stearate (intragranular) | 2.268 | | 1.6 | | 2.268 | |
| Silicon magnesium stearate (extragranular) | 1.59 | | 1.6 | | 1.59 | |

| Time/min | Dissolution/% | | | | | |
|---|---|---|---|---|---|---|
| | API | SNAC | API | SNAC | API | SNAC |
| 5 | 39 | 43 | 38 | 43 | 43 | 52 |
| 10 | 72 | 74 | 73 | 75 | 80 | 85 |
| 15 | 89 | 89 | 94 | 94 | 95 | 97 |
| 30 | 99 | 97 | 101 | 101 | 99 | 99 |

In the formulations, "/" means not added.

**12.4** SNAC, API, β-cyclodextrin, colloidal silicon dioxide (intragranular), and magnesium stearate (intragranular) each at an amount specified in the formulation were mixed well and subjected to dry granulation, and then magnesium stearate (extragranular) was added, and the resulting mixture was compressed into tablets. The tablets were subjected to the dissolution test in phosphate buffer (pH 6.8) containing 0.1% tween 80, and the specific formulations and dissolution results are shown in Table 22.

**Table 22. Ingredients and dissolution of compositions**

| Batch | Formulation 14 | |
|---|---|---|
| Component | Amount mg/tablet | |
| API | 5 | |
| SNAC | 133.856 | |
| β-cyclodextrin | 15.35 | |
| Colloidal silicon dioxide (intragranular) | 0.794 | |
| Magnesium stearate (intragranular) | 2.268 | |
| Magnesium stearate (extragranular) | 1.59 | |

| Time/min | Dissolution/% | |
|---|---|---|
| | API | SNAC |
| 5 | 46 | 55 |
| 10 | 77 | 82 |
| 15 | 90 | 93 |
| 30 | 98 | 98 |

**12.5** Uncoated tablets prepared according to formulation 6, formulation 11, and formulation 12 were coated with coating powders made from hypromellose film-forming material and then stored under the conditions of accelerated test (40 °C, RH75%), long-term test (25 °C, RH60%), and refrigerated test (5 °C) for stability examination, and the results are shown in Table 23.

**Table 23. Ingredients and dissolution of compositions**

| Formulatio n | Sample storage conditions | | Main peak purity (%) | Maximum single impurity (%) | Total impurity (%) |
|---|---|---|---|---|---|
| Formulatio n 6 | Initial | | 98.2 | 0.64 | 1.80 |
| | 40°C/RH75% | 2 weeks | 96.9 | 0.59 | 3.10 |
| | | 1M | 96.2 | 0.95 | 3.80 |
| | | 2M | 93.5 | 1.39 | 6.50 |
| | 25°C/RH60% | 1M | 97.8 | 0.55 | 2.20 |
| | | 2M | 96.6 | 0.54 | 3.40 |
| | 5°C±3°C | 2.5M | 97.2 | 0.57 | 2.8 |
| Formulatio n 11 | Initial | | 97.9 | 0.67 | 2.10 |
| | 40°C/RH75% | 2 weeks | 97.0 | 0.60 | 3.00 |
| | | 1M | 96.2 | 1.03 | 3.80 |
| | | 2M | 94.0 | 1.38 | 5.99 |
| | 25°C/RH60% | 1M | 97.8 | 0.56 | 2.20 |
| | | 2M | 96.9 | 0.55 | 3.10 |
| | 5°C±3°C | 2.5M | 97.0 | 0.58 | 3.0 |
| Formulatio n 12 | Initial | | 98.3 | 0.57 | 1.70 |
| | 25°C/RH60% | 1M | 98.1 | 0.416 | 1.90 |
| | | 2M | 97.1 | 0.59 | 2.9 |
| | 5°C±3°C | 2M | 97.3 | 0.59 | 2.7 |

**12.6** API, sodium caprate, and a PMSF protease inhibitor each at an amount specified in the formulation were mixed well and then filled into corresponding capsule shells. The capsules were placed in a basket sinker for the dissolution test in phosphate buffer (pH 6.8) containing 0.1% tween 80, and the specific formulations and dissolution results are shown in Table 24.

**Table 24. Ingredients and dissolution of compositions**

| Batch | Formulation 15 | Formulation 16 |
|---|---|---|
| Component | Amount mg/capsule | |
| API | 5 | 5 |
| Sodium caprate | 250 | 250 |
| PMSF protease inhibitor | 62.50 | 62.50 |
| Capsule shell | Enteric capsule shell | Gastric soluble capsule shell |

| Time/min | Dissolution/% | |
|---|---|---|
| 15 | 89 | 97 |
| 30 | 93 | 100 |
| 45 | 93 | 99 |
| 60 | 94 | 101 |

**12.7** SNAC, API, amino acids, sodium caprate or sodium caprylate, colloidal silicon dioxide (intragranular), and magnesium stearate (intragranular) each at an amount specified in the formulation were mixed well and subjected to dry granulation, and then magnesium stearate (extragranular) was added, and the resulting mixture was compressed into tablets. The tablets were subjected to the dissolution test in phosphate buffer (pH 6.8) containing 0.1% tween 80, and the specific formulations and dissolution results are shown in Table 25.

**Table 25. Ingredients and dissolution of compositions**

| Batch | Formulation 17 | | Formulation 18 | | Formulation 19 | | Formulation 20 | |
|---|---|---|---|---|---|---|---|---|
| Component | Amount mg/tablet | | | | | | | |
| API | 25 | | 25 | | 15 | | 15 | |
| SNAC | 300 | | 300 | | 300 | | 300 | |
| Arginine | 120 | | 120 | | 180 | | 180 | |
| Sodium caprate | 60 | | 180 | | 130 | | / | |
| Sodium caprylate | / | | / | | / | | 130 | |
| Colloidal silicon dioxide (intragranular) | 5 | | 6 | | 6 | | 6 | |
| Magnesium stearate (intragranular) | 5 | | 6 | | 6 | | 6 | |
| Magnesium stearate (extragranular) | 5 | | 6 | | 6 | | 6 | |

| Time/min | Dissolution/% | | | | | | | |
|---|---|---|---|---|---|---|---|---|
| | API | SNAC | API | SNAC | API | SNAC | API | SNAC |
| 5 | 33 | 37 | / | / | / | / | / | / |
| 10 | 63 | 66 | / | / | / | / | / | / |
| 15 | 79 | 82 | / | / | / | / | / | / |
| 30 | 93 | 95 | / | / | / | / | / | / |

In the formulations, "/" means not added.

12.8 SNAC, API, amino acids, sorbitol, colloidal silicon dioxide (intragranular), magnesium stearate (intragranular), and/or a binder (povidone or hydroxypropyl cellulose) each at an amount specified in the formulation were mixed well and subjected to dry granulation, and then magnesium stearate (extragranular) was added, and the resulting mixture was compressed into tablets. The tablets were subjected to the dissolution test in phosphate buffer (pH 6.8) containing 0.1% tween 80, and the specific formulations and dissolution results are shown in Table 26.

**Table 26. Ingredients and dissolution of compositions**

| Batch | Formulation 21 | Formulation 22 | Formulation 23 | Formulation 24 | Formulation 25 | | | | | |
|---|---|---|---|---|---|---|---|---|---|---|
| API | 25 | 15 | 25 | 25 | 25 | | | | | |
| SNAC | 300 | 300 | 300 | 300 | 300 | | | | | |
| Arginine | 120 | 180 | 180 | 120 | 120 | | | | | |
| Sorbitol | 180 | 130 | 130 | 170 | 170 | | | | | |
| PVP K30 | / | / | / | 10 | / | | | | | |
| HPC-SSL | / | / | / | / | 10 | | | | | |
| Colloidal silicon dioxide (intragranular) | 6 | 6 | 6.5 | 5 | 5 | | | | | |
| Magnesium stearate (intragranular) | 6 | 6 | 6 | 5 | 5 | | | | | |
| Magnesium stearate (extragranular) | 6 | | 6 | | 6.5 | | 5 | | 5 | |

| Time/min | Dissolution/% | | | | | | | | | |
|---|---|---|---|---|---|---|---|---|---|---|
| | API | SNAC | API | SNAC | API | SNAC | API | SNAC | API | SNAC |
| 5 | 32 | 36 | / | / | / | / | 31 | 35 | 29 | 35 |
| 10 | 59 | 63 | / | / | / | / | 59 | 63 | 59 | 63 |
| 15 | 78 | 81 | / | / | / | / | 78 | 81 | 77 | 81 |
| 30 | 91 | 94 | / | / | / | / | 93 | 95 | 90 | 94 |
| 45 | 93 | 96 | / | / | / | / | 94 | 95 | 90 | 94 |

In the formulations, "/" means not added.

12.9 SNAC, API, amino acids, colloidal silicon dioxide (intragranular), and magnesium stearate (intragranular) each at an amount specified in the formulation were mixed well and subjected to dry granulation, and then magnesium stearate (extragranular) was added, and the resulting mixture was compressed into tablets. The tablets were subjected to the dissolution test in phosphate buffer (pH 6.8) containing 0.1% tween 80, the specific formulations are shown in Table 27, and the formulations and dissolution results are shown in Table 28.

**Table 27. Ingredients of compositions**

| Batch | Formulation 26 | Formulation 27 |
|---|---|---|
| Component | Amount mg/tablet | |
| API | 5 | 5 |
| SNAC | 100 | 100 |
| Arginine | 60 | 114.25 |
| Colloidal silicon dioxide (intragranular) | 1.65 | 2.25 |
| Magnesium stearate (intragranular) | 1.65 | 2.25 |
| Magnesium stearate (extragranular) | 1.7 | 2.25 |

**Table 28. Ingredients and dissolution of compositions**

| Batch | Formulation 28 | | Formulation 29 | | Formulation 30 | |
|---|---|---|---|---|---|---|
| Component | Amount mg/tablet | | | | | |
| API | 25.0 | | 25.0 | | 25.0 | |
| SNAC | 500.0 | | 500.0 | | 300.0 | |
| Arginine | 120.0 | | 300.0 | | 180.0 | |
| Colloidal silicon dioxide (intragranular) | 6.0 | | 8.0 | | 5.0 | |
| Magnesium stearate (intragranular) | 6.0 | | 8.5 | | 5.0 | |
| Magnesium stearate (extragranular) | 6.0 | | 8.5 | | 5.0 | |

| Time/min | Dissolution/% | | | | | |
|---|---|---|---|---|---|---|
| | API | SNAC | API | SNAC | API | SNAC |
| 5 | 30 | 32 | 31 | 33 | 26 | 31 |
| 10 | 53 | 56 | 57 | 60 | 50 | 55 |
| 15 | 74 | 76 | 75 | 78 | 66 | 72 |
| 30 | 94 | 95 | 90 | 93 | 82 | 87 |
| 45 | 96 | 97 | 91 | 93 | 82 | 88 |

12.10 SNAC, API, amino acids, colloidal silicon dioxide (intragranular), and magnesium stearate (intragranular) each at an amount specified in the formulation were mixed well and subjected to dry granulation, and then magnesium stearate (extragranular) was added, and the resulting mixture was compressed into tablets. The tablets were subjected to the dissolution test in phosphate buffer (pH 6.8) containing 0.1% tween 80, and the specific formulations and dissolution results are shown in Table 29.

**Table 29. Ingredients and dissolution of compositions**

| Batch | Formulation 31 | | Formulation 32 | |
|---|---|---|---|---|
| API | 25 | | 25 | |
| SNAC | 300 | | 300 | |
| Arginine | 120 | | 120 | |
| Leucine | 180 | | / | |
| Lysine hydrochloride | / | | 180 | |
| Colloidal silicon dioxide (intragranular) | 6 | | 6 | |
| Magnesium stearate (intragranular) | 6 | | 6 | |
| Magnesium stearate (extragranular) | 6 | | 6 | |

| Time/min | Dissolution/% | | | |
|---|---|---|---|---|
| | API | SNAC | API | SNAC |
| 5 | / | / | 32 | 33 |
| 10 | / | / | 58 | 59 |
| 15 | / | / | 78 | 78 |
| 30 | / | / | 94 | 94 |
| 45 | / | / | 96 | 95 |

In the formulations, "/" means not added.

Although the foregoing invention has been described in detail by way of drawings and examples for purposes of clarity of understanding, the description and examples should not be construed as limiting the scope of the present disclosure. The disclosures of all the patents and scientific literature cited herein are clearly incorporated by reference in their entireties.

## Claims

1. A pharmaceutical composition, comprising:
(a) a GLP-1 analog of general formula (I) or a pharmaceutically acceptable salt, amide or ester thereof, 
R1-X1-X2-Glu-Gly-Thr-Phe-Thr-Ser-Asp-X10-Ser-X12-X13-X14-X15-X16-X17-X18-X19-
X20-Glu-Phe-X23-X24-Trp-Leu-X27-X28-X29-X30-Pro-Ser-Ser-Gly-Ala-Pro-Pro-Pro-Ser-
R2
(I)
 wherein, R₁ is H, alkyl, acetyl, formyl, benzoyl, trifluoroacetyl, pGlu, or absent; R₂ is - NH₂, -OH, or absent; X₁, X₂, X₁₀, X₁₂, X₁₃, X₁₄, X₁₅, X₁₆, X₁₇, X₁₈, X₁₉, X₂₀, X₂₃, X₂₄, X₂₇, X₂₈, X₂₉, and X₃₀ are independently selected from the group consisting of any natural amino acid residues and any non-natural amino acid residues; and
(b) an absorption enhancer, wherein preferably, the absorption enhancer is selected from one or more of N (8-(2-hydroxybenzoyl)amino)caprylate, sodium caprylate, sodium caprate, sodium taurodeoxycholate, lauroyl carnitine, dodecyl phosphatidylcholine, an amino acid, cyclodextrin and a derivative thereof, a surfactant, laurocapram and a homolog thereof, an alcohol-based organic solvent, an organic acid, and fatty alcohol.

2. The pharmaceutical composition according to claim 1, wherein:
the amino acid is selected from one or more of isoleucine, leucine, lysine, methionine, phenylalanine, threonine, tryptophan, valine, arginine, histidine, alanine, glycine, proline, serine, and salts thereof, and preferably selected from one or more of arginine, histidine, leucine and lysine and salts thereof;
the cyclodextrin and the derivative thereof are selected from one or more of α-cyclodextrin, β-cyclodextrin, γ-cyclodextrin, sulfobutyl-β-cyclodextrin (SBE-β-CD), 2,6-dimethyl-β-cyclodextrin, hydroxypropyl-β-cyclodextrin, hydroxyethyl-β-cyclodextrin, methylated-β-cyclodextrin, glucosyl-β-cyclodextrin, diglucosyl-β-cyclodextrin, maltosyl-β-cyclodextrin, dimaltosy-β-cyclodextrin, and carboxymethyl-β-cyclodextrin, and are preferably β-cyclodextrin;
the surfactant is selected from the group consisting of sodium dodecyl sulfate, polysorbate, betaine, quaternary ammonium salt, sorbitan ester, poloxamer, and any mixtures thereof;
the alcohol-based organic solvent is selected from the group consisting of ethanol, glycerol, polyethylene glycol, propylene glycol, and any mixtures thereof;
the organic acid is selected from the group consisting of stearic acid, palmitic acid, oleic acid, lauric acid, and any mixtures thereof; and/or
the fatty alcohol is selected from the group consisting of lauryl alcohol, cetyl alcohol, stearyl alcohol, and any mixtures thereof.

3. The pharmaceutical composition according to claim 1 or 2, wherein the absorption enhancer is selected from any one of the following:
(1) N (8-(2-hydroxybenzoyl)amino)caprylate;
(2) sodium caprate;
(3) N (8-(2-hydroxybenzoyl)amino)caprylate and sodium caprylate;
(4) N (8-(2-hydroxybenzoyl)amino)caprylate and an amino acid or a salt thereof;
(5) N (8-(2-hydroxybenzoyl)amino)caprylate and β-cyclodextrin;
(6) N-(8-(2-hydroxybenzoyl)amino)caprylate, an amino acid or a salt thereof, and sodium caprate; and
(7) N-(8-(2-hydroxybenzoyl)amino)caprylate, an amino acid or a salt thereof, and sodium caprylate;
preferably, the N (8-(2-hydroxybenzoyl)amino)caprylate is sodium N (8-(2-hydroxybenzoyl)amino)caprylate.

4. The pharmaceutical composition according to any one of claims 1 to 3, wherein:
X₁ is Tyr or His; X₂ is Aib or D-Ala; X₁₀ is Val, Tyr, or Y1; X₁₂ is Ser, Ile, or Y1; X₁₃ is Tyr, Ala, or Y1; X₁₄ is Leu, Nle, or Y1; X₁₅ is Asp or Glu; X₁₆ is Arg, Glu, Gly, Lys, Aib, or Y1; X₁₇ is Glu, Ile, Gln, or Y1; Xis is Ala, Aib, or His; X₁₉ is Ala, Aib, or Gin; X₂₀ is Gln, Glu, or Lys; X₂₃ is Ile or Val; X₂₄ is Ala, Asn, or Gln; X₂₇ is Val or Leu; X₂₈ is Arg or Ala; X₂₉ is Gly or Gln; X₃₀ is Gly or Lys;
Y1 is a Lys, Orn, Dap, Dab, or Cys residue comprising a substituent on a side chain, the substituent having a formula {[2-(2-amino-ethoxy)-ethoxy]-acetyl}ₐ-(γ-Glu)_{b}-CO-(CH₂)_{c}-COOH;
a is an integer of 1-3;
b is 1 or 2;
c is an integer of 10-30.

5. The pharmaceutical composition according to any one of claims 1 to 4, wherein:
X₁ is Tyr; X₂ is Aib; X₁₀ is Tyr; X₁₂ is Ile; X₁₃ is Tyr; X₁₄ is Y1; X₁₅ is Asp or Glu; X₁₆ is Arg or Lys; X₁₇ is Ile; Xis is Ala; X₁₉ is Ala; X₂₀ is Gln; X₂₃ is Ile or Val; X₂₄ is Asn; X₂₇ is Ile or Leu; X₂₈ is Ala; X₂₉ is Gly; X₃₀ is Gly;
Y1 is as defined in claim 4.

6. The pharmaceutical composition according to claim 5, wherein:
X₁₆ is Lys; X₂₃ is Val; X₂₇ is Leu.

7. The pharmaceutical composition according to any one of claims 1 to 3, wherein:
X₁ is Tyr; X₂ is Aib or D-Ala; X₁₀ is Y1; X₁₂ is Ile; X₁₃ is Tyr; X₁₄ is Leu or Nle; X₁₅ is Glu; X₁₆ is Arg or Lys; X₁₇ is Ile; Xis is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly;
Y1 is as defined in claim 4.

8. The pharmaceutical composition according to any one of claims 1 to 3, wherein:
X₁ is Tyr; X₂ is Aib or D-Ala; X₁₀ is Tyr; X₁₂ is Y1; X₁₃ is Tyr; X₁₄ is Leu or Nle; X₁₅ is Glu; X₁₆ is Arg or Lys; X₁₇ is Ile; Xis is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly;
Y1 is as defined in claim 4.

9. The pharmaceutical composition according to any one of claims 1 to 3, wherein:
X₁ is Tyr; X₂ is Aib or D-Ala; X₁₀ is Tyr; X₁₂ is Ile; X₁₃ is Y1; X₁₄ is Leu or Nle; X₁₅ is Glu; X₁₆ is Arg or Lys; X₁₇ is Ile; Xis is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly;
Y1 is as defined in claim 4.

10. The pharmaceutical composition according to any one of claims 1 to 3, wherein:
X₁ is Tyr; X₂ is Aib or D-Ala; X₁₀ is Tyr; X₁₂ is Ile; X₁₃ is Tyr; X₁₄ is Y1; X₁₅ is Glu; X₁₆ is Arg or Lys; X₁₇ is Ile; Xis is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly;
Y1 is as defined in claim 4.

11. The pharmaceutical composition according to claim 10, wherein:
X₂ is Aib; X₂₀ is Gin; X₂₄ is Asn.

12. The pharmaceutical composition according to any one of claims 1 to 3, wherein:
X₁ is Tyr; X₂ is Aib or D-Ala; X₁₀ is Tyr; X₁₂ is Ile; X₁₃ is Tyr; X₁₄ is Leu or Nle; X₁₅ is Glu; X₁₆ is Y1; X₁₇ is Ile; Xis is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly;
Y1 is as defined in claim 4.

13. The pharmaceutical composition according to claim 12, wherein:
X₂ is Aib; X₁₄ is Leu; X₂₀ is Gln; X₂₄ is Asn.

14. The pharmaceutical composition according to any one of claims 1 to 3, wherein:
X₁ is Tyr; X₂ is Aib or D-Ala; X₁₀ is Tyr; X₁₂ is Ile; X₁₃ is Tyr; X₁₄ is Leu or Nle; X₁₅ is Glu; X₁₆ is Arg or Lys; X₁₇ is Y1; Xis is Ala; X₁₉ is Ala; X₂₀ is Gln or Lys; X₂₃ is Ile or Val; X₂₄ is Asn or Gln; X₂₇ is Ile or Leu; X₂₈ is Ala; X₂₉ is Gly; X₃₀ is Gly;
Y1 is as defined in claim 4.

15. The pharmaceutical composition according to any one of claims 4 to 14, wherein a is 2, b is 1 or 2, and c is an integer of 16-20.

16. The pharmaceutical composition according to claim 15, wherein c is 16, 18, or 20.

17. The pharmaceutical composition according to any one of claims 4 to 14, wherein Y1 is a Lys residue comprising a substituent on a side chain, the substituent having a formula {[2-(2-amino-ethoxy)-ethoxy]-acetyl}ₐ-(γ-Glu)_{b}-CO-(CH₂)_{c}-COOH;
a is 2;
b is 1 or 2;
c is 16 or 18.

18. The pharmaceutical composition according to any one of claims 4 to 17, wherein the substituent is covalently connected to amino on the side chain via an amide bond.

19. The pharmaceutical composition according to any one of claims 4 to 18, wherein Y1 is K(-OEG-OEG-γGlu-C18-OH) or K(-OEG-OEG-γGlu-C20-OH),
wherein K(-OEG-OEG-yGlu-C18-OH) has a structure shown below: 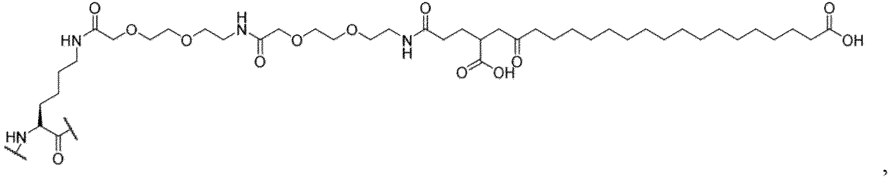 and
preferably has a structure shown below: 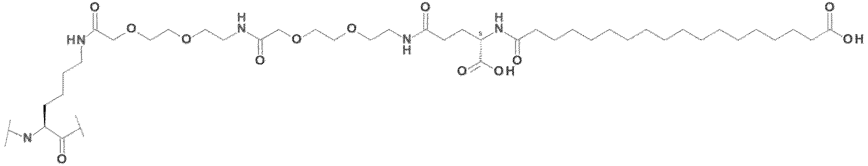
and K(-OEG-OEG-yGlu-C20-OH) has a structure shown below: 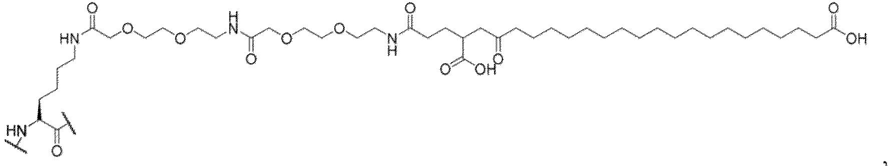
and preferably has a structure shown below:

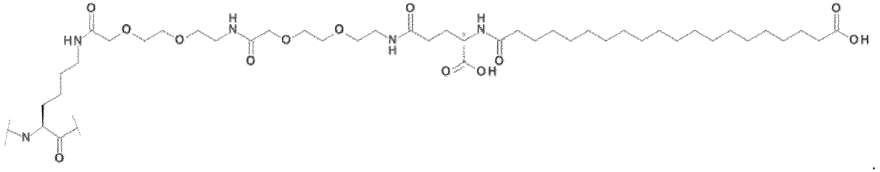

20. The pharmaceutical composition according to any one of claims 4 to 19, wherein the substituent is covalently connected to ε amino on the side chain via an amide bond.

21. The pharmaceutical composition according to any one of claims 1 to 20, wherein the GLP-1 analog has the sequence set forth in SEQ ID NO: 20;
preferably, the GLP-1 analog is selected from the group consisting of the following compounds numbered 1 to 18:
| | |
|---|---|
| 1 | H-YAibEGTFTSDYSIYKDKIAAQEFVNWLIAGGPSSGAPPPS-NH₂ |
| 2 | H-YAibEGTFTSDYSIYKDRIAAQEFVNWLIAGGPSSGAPPPS-NH₂ |
| 3 | H-YAibEGTFTSDYSIYKDKIAAQEFINWLIAGGPSSGAPPPS-NH₂ |
| 4 | H-YAibEGTFTSDYSIYKDRIAAQEFINWLIAGGPSSGAPPPS-NH₂ |
| 5 | H-YAibEGTFTSDYSIYKDKIAAQEFINWLLAGGPSSGAPPPS-NH₂ |
| 6 | H-YAibEGTFTSDYSIYKDRIAAQEFVNWLLAGGPSSGAPPPS-NH₂ |
| 7 | H-YAibEGTFTSDYSIYKDKIAAQEFVNWLLAGGPSSGAPPPS-NH₂ |
| 8 | H-YAibEGTFTSDYSIYLEKIAAQEFVNWLLAGGPSSGAPPPS-NH₂ |
| 9 | H-YAibEGTFTSDYSIYLEKIAAQEFVNWLIAGGPSSGAPPPS-NH₂ |
| 10 | H-YAibEGTFTSDYSIYLEKIAAQEFINWLIAGGPSSGAPPPS-NH₂ |
| 11 | H-YAibEGTFTSDYSIYLEKIAAQEFINWLLAGGPSSGAPPPS-NH₂ |
| 12 | H-YAibEGTFTSDYSIYKEKIAAQEFVNWLIAGGPSSGAPPPS-NH₂ |
| 13 | H-YAibEGTFTSDYSIYKERIAAQEFVNWLIAGGPSSGAPPPS-NH₂ |
| 14 | H-YAibEGTFTSDYSIYKEKIAAQEFINWLIAGGPSSGAPPPS-NH₂ |
| 15 | H-YAibEGTFTSDYSIYKERIAAQEFINWLIAGGPSSGAPPPS-NH₂ |
| 16 | H-YAibEGTFTSDYSIYKEKIAAQEFINWLLAGGPSSGAPPPS-NH₂ |
| 17 | H-YAibEGTFTSDYSIYKERIAAQEFVNWLLAGGPSSGAPPPS-NH₂ |
| 18 | H-YAibEGTFTSDYSIYKEKIAAQEFVNWLLAGGPSSGAPPPS-NH₂. |

22. The pharmaceutical composition according to any one of claims 1 to 21, wherein the GLP-1 analog is selected from the group consisting of the following compounds numbered 1# to 18#:
| | |
|---|---|
| 1# | H-YAibEGTFTSDYSIYK(OEG-OEG-γGlu-C20-OH)DKIAAQEFVNWLIAGGPSSGAPPPS- NH2 |
| 2# | H-YAibEGTFTSDYSIYK(OEG-OEG-γGlu-C20-OH)DRIAAQEFVNWLIAGGPSSGAPPPS- NH2 |
| 3# | H-YAibEGTFTSDYSIYK(OEG-OEG-γGlu-C20-OH)DKIAAQEFINWLIAGGPSSGAPPPS- NH2 |
| 4# | H-YAibEGTFTSDYSIYK(OEG-OEG-γGlu-C20-OH)DRIAAQEFINWLIAGGPSSGAPPPS- NH2 |
| 5# | H-YAibEGTFTSDYSIYK(OEG-OEG-γGlu-C20-OH)DKIAAQEFINWLLAGGPSSGAPPPS- NH2 |
| 6# | H-YAibEGTFTSDYSIYK(OEG-OEG-γGlu-C20-OH)DRIAAQEFVNWLLAGGPSSGAPPPS- NH2 |
| 7# | H-YAibEGTFTSDYSIYK(OEG-OEG-γGlu-C20-OH)DKIAAQEFVNWLLAGGPSSGAPPPS- NH2 |
| 8# | H-YAibEGTFTSDYSIYLEK(OEG-OEG-γGlu-C20-OH)IAAQEFVNWLLAGGPSSGAPPPS- |
| | NH2 |
| 9# | H-YAibEGTFTSDYSIYLEK(OEG-OEG-γGlu-C20-OH)IAAQEFVNWLIAGGPSSGAPPPS- NH2 |
| 10# | H-YAibEGTFTSDYSIYLEK(OEG-OEG-γGlu-C20-OH)IAAQEFINWLIAGGPSSGAPPPS- NH2 |
| 11# | H-YAibEGTFTSDYSIYLEK(OEG-OEG-γGlu-C20-OH)IAAQEFINWLLAGGPSSGAPPPS- NH2 |
| 12# | H-YAibEGTFTSDYSIYK(OEG-OEG-γGlu-C20-OH)EKIAAQEFVNWLIAGGPSSGAPPPS- NH2 |
| 13# | H-YAibEGTFTSDYSIYK(OEG-OEG-γGlu-C20-OH)ERIAAQEFVNWLIAGGPSSGAPPPS- NH2 |
| 14# | H-YAibEGTFTSDYSIYK(OEG-OEG-γGlu-C20-OH)EKIAAQEFINWLIAGGPSSGAPPPS- NH2 |
| 15# | H-YAibEGTFTSDYSIYK(OEG-OEG-γGlu-C20-OH)ERIAAQEFINWLIAGGPSSGAPPPS- NH2 |
| 16# | H-YAibEGTFTSDYSIYK(OEG-OEG-γGlu-C20-OH)EKIAAQEFINWLLAGGPSSGAPPPS- NH2 |
| 17# | H-YAibEGTFTSDYSIYK(OEG-OEG-γGlu-C20-OH)ERIAAQEFVNWLLAGGPSSGAPPPS- NH2 |
| 18# | H-YAibEGTFTSDYSIYK(OEG-OEG-γGlu-C20-OH)EKIAAQEFVNWLLAGGPSSGAPPPS- NH2. |

23. The pharmaceutical composition according to any one of claims 1 to 22, wherein the GLP-1 analog is selected from the group consisting of compounds shown as 7#, 12#, 13#, 14#, 15#, 16#, 17#, and 18# in FIG. 3.

24. The pharmaceutical composition according to any one of claims 1 to 23, wherein the GLP-1 analog or the pharmaceutically acceptable salt, amide or ester thereof has higher agonist activity against GLP-1R than against GIP receptor;
preferably, the GLP-1 analog has a ratio of the agonist activity against GLP-1R to the agonist activity against GIP receptor of 1.1:1 to 10:1, more preferably 3:1 to 6.5:1, and most preferably 4.5:1 to 6:1.

25. The pharmaceutical composition according to any one of claims 1 to 24, further comprising one or more of a filler, a binder, a disintegrant, a lubricant, a glidant, a coating agent, and a protease inhibitor.

26. The pharmaceutical composition according to any one of claims 1 to 25, wherein:
the filler is selected from the group consisting of microcrystalline cellulose, mannitol, lactose, pregelatinized starch, calcium hydrogen phosphate, sorbitol, and any mixtures thereof;
the binder is selected from the group consisting of povidone and a homolog thereof, hydroxypropyl cellulose, hydroxypropyl methyl cellulose and a homolog thereof, and any mixtures thereof;
the disintegrant is selected from the group consisting of microcrystalline cellulose, alginic acid, alginate, polacrilin potassium, calcium hydrogen phosphate, corn starch, pregelatinized starch, sodium carboxymethyl starch, sodium starch glycolate, crospovidone, cellulose powder, hydroxymethyl cellulose, hydroxyethyl cellulose, hydroxypropyl cellulose, hydroxypropyl methylcellulose, croscarmellose sodium, and any mixtures thereof;
the lubricant is selected from the group consisting of magnesium stearate, glyceryl behenate, sodium stearyl fumarate, stearic acid, palmitic acid, calcium stearate, talc, carnauba wax, and any mixtures thereof;
the glidant is selected from the group consisting of colloidal silicon dioxide and talc;
the coating agent is selected from one or more of sodium carboxymethylcellulose, calcium carboxymethylcellulose, hydroxypropyl cellulose, hydroxypropyl methyl cellulose, hydroxyethyl cellulose, hydroxymethyl cellulose, methylcellulose, carboxymethyl cellulose, polyvinyl alcohol, methacrylic acid resin copolymer, and ethyl cellulose; and/or
the protease inhibitor is selected from the group consisting of phenylmethylsulfonyl fluoride (PSMF), soybean trypsin inhibitor, soybean trypsin-chymotrypsin inhibitor, colicin, sunflower trypsin inhibitor, leupeptin, citric acid, ethylenediaminetetraacetic acid, sodium glycocholate, and 4-(2-aminoethyl)benzenesulfonyl fluoride hydrochloride.

27. The pharmaceutical composition according to any one of claims 1 to 26, wherein the absorption enhancer accounts for at least 50% of the total mass of the composition;
preferably, the absorption enhancer accounts for at least 70% of the total mass of the composition;
more preferably, the absorption enhancer accounts for at least 80% or at least 90% of the total mass of the composition.

28. The pharmaceutical composition according to any one of claims 1 to 27, wherein:
the absorption enhancer is SNAC, and the SNAC accounts for at least 20%, at least 50%, at least 70%, or at least 90% of the total mass of the composition;
the absorption enhancer is sodium caprate, and the sodium caprate accounts for at least 50%, at least 60%, or at least 70% of the total mass of the composition;
the absorption enhancer is SNAC and sodium caprylate; the SNAC accounts for 20%-90% of the total mass of the composition, and the sodium caprylate accounts for 5%-75% of the total mass of the composition;
the absorption enhancer is SNAC and arginine or a salt thereof; the SNAC accounts for 20%-90% of the total mass of the composition, and the arginine or the salt thereof accounts for 5%-75% of the total mass of the composition;
the absorption enhancer is SNAC and β-cyclodextrin; the SNAC accounts for 20%-90% of the total mass of the composition, and the β-cyclodextrin accounts for 5%-75% of the total mass of the composition;
the absorption enhancer is SNAC, arginine or a salt thereof, and sodium caprate; the SNAC accounts for 20%-90% of the total mass of the composition, the arginine or the salt thereof accounts for 5%-60% of the total mass of the composition, and the sodium caprate accounts for 1%-50% of the total mass of the composition;
the absorption enhancer is SNAC, arginine or a salt thereof, and leucine or a salt thereof;
the SNAC accounts for 20%-90% of the total mass of the composition, the arginine or the salt thereof accounts for 5%-60% of the total mass of the composition, and the leucine or the salt thereof accounts for 1%-50% of the total mass of the composition;
the absorption enhancer is SNAC, arginine or a salt thereof, and lysine or a salt thereof; the SNAC accounts for 20%-90% of the total mass of the composition, the arginine or the salt thereof accounts for 5%-60% of the total mass of the composition, and the lysine or the salt thereof accounts for 1%-50% of the total mass of the composition; or
the absorption enhancer is SNAC, arginine or a salt thereof, and sodium caprylate; the SNAC accounts for 20%-90% of the total mass of the composition, the arginine or the salt thereof accounts for 5%-60% of the total mass of the composition, and the sodium caprylate accounts for 1%-50% of the total mass of the composition.

29. The pharmaceutical composition according to any one of claims 1 to 28, wherein the GLP-1 analog or the pharmaceutically acceptable salt, amide or ester thereof accounts for 0.1%-30% of the total mass of the composition;
preferably, the GLP-1 analog or the pharmaceutically acceptable salt, amide or ester thereof accounts for 1%-10% of the total mass of the composition;
more preferably, the GLP-1 analog or the pharmaceutically acceptable salt, amide or ester thereof accounts for 1%-5% of the total mass of the composition.

30. The pharmaceutical composition according to any one of claims 1 to 29, selected from any one of the following compositions:
a composition (a) comprising the compound shown as 18# in FIG. 3 or a pharmaceutically acceptable salt, amide or ester thereof and N-(8-(2-hydroxybenzoyl)amino)caprylate;
a composition (b) comprising the compound shown as 18# in FIG. 3 or a pharmaceutically acceptable salt, amide or ester thereof, N (8-(2-hydroxybenzoyl)amino)caprylate, and sodium caprylate;
a composition (c) comprising the compound shown as 18# in FIG. 3 or a pharmaceutically acceptable salt, amide or ester thereof, N (8-(2-hydroxybenzoyl)amino)caprylate, and an amino acid or a salt thereof, the amino acid being preferably one or more of histidine, arginine, leucine and lysine or salts thereof;
a composition (d) comprising the compound shown as 18# in FIG. 3 or a pharmaceutically acceptable salt, amide or ester thereof, N (8-(2-hydroxybenzoyl)amino)caprylate, and β-cyclodextrin;
a composition (e) comprising the compound shown as 18# in FIG. 3 or a pharmaceutically acceptable salt, amide or ester thereof, and sodium caprate;
a composition (f) comprising the compound shown as 18# in FIG. 3 or a pharmaceutically acceptable salt, amide or ester thereof, N (8-(2-hydroxybenzoyl)amino)caprylate, arginine or a salt thereof, and sodium caprate;
a composition (g) comprising the compound shown as 18# in FIG. 3 or a pharmaceutically acceptable salt, amide or ester thereof, N (8-(2-hydroxybenzoyl)amino)caprylate, and histidine or a salt thereof;
a composition (h) comprising the compound shown as 18# in FIG. 3 or a pharmaceutically acceptable salt, amide or ester thereof, N (8-(2-hydroxybenzoyl)amino)caprylate, and arginine or a salt thereof;
a composition (i) comprising the compound shown as 18# in FIG. 3 or a pharmaceutically acceptable salt, amide or ester thereof, N (8-(2-hydroxybenzoyl)amino)caprylate, arginine or a salt thereof, and leucine or a salt thereof;
a composition (j) comprising the compound shown as 18# in FIG. 3 or a pharmaceutically acceptable salt, amide or ester thereof, N (8-(2-hydroxybenzoyl)amino)caprylate, arginine or a salt thereof, and lysine or a salt thereof; and
a composition (k) comprising the compound shown as 18# in FIG. 3 or a pharmaceutically acceptable salt, amide or ester thereof, N (8-(2-hydroxybenzoyl)amino)caprylate, arginine or a salt thereof, and sodium caprylate.

31. The pharmaceutical composition according to any one of claims 1 to 30, selected from any one of the following compositions:
a composition (a1) comprising, relative to the total mass of the composition, 1%-20% of the compound shown as 18# in FIG. 3 or a pharmaceutically acceptable salt, amide or ester thereof and at least 20% or at least 50% of N-(8-(2-hydroxybenzoyl)amino)caprylate;
a composition (b1) comprising, relative to the total mass of the composition, 1%-20% of the compound shown as 18# in FIG. 3 or a pharmaceutically acceptable salt, amide or ester thereof, 20%-90% of *N*-(8-(2-hydroxybenzoyl)amino)caprylate, and 5%-75% of sodium caprylate;
a composition (c1) comprising, relative to the total mass of the composition, 1%-20% of the compound shown as 18# in FIG. 3 or a pharmaceutically acceptable salt, amide or ester thereof, 20%-90% of *N*-(8-(2-hydroxybenzoyl)amino)caprylate, and 5%-75% of an amino acid (e.g., one or more of histidine, arginine, leucine and lysine or salts thereof);
a composition (d1) comprising, relative to the total mass of the composition, 1%-20% of the compound shown as 18# in FIG. 3 or a pharmaceutically acceptable salt, amide or ester thereof, 20%-90% of *N*-(8-(2-hydroxybenzoyl)amino)caprylate, and 5%-75% of β-cyclodextrin;
a composition (e1) comprising, relative to the total mass of the composition, 1%-20% of the compound shown as 18# in FIG. 3 or a pharmaceutically acceptable salt, amide or ester thereof and at least 50% of sodium caprate;
a composition (f1) comprising, relative to the total mass of the composition, 1%-20% of the compound shown as 18# in FIG. 3 or a pharmaceutically acceptable salt, amide or ester thereof, 20%-90% of *N*-(8-(2-hydroxybenzoyl)amino)caprylate, 5%-60% of arginine or a salt thereof, and 1%-50% of sodium caprate;
a composition (g1) comprising, relative to the total mass of the composition, 1%-20% of the compound shown as 18# in FIG. 3 or a pharmaceutically acceptable salt, amide or ester thereof, 20%-90% of N (8-(2-hydroxybenzoyl)amino)caprylate, and 5%-75% of arginine or a salt thereof;
a composition (h1) comprising, relative to the total mass of the composition, 1%-20% of the compound shown as 18# in FIG. 3 or a pharmaceutically acceptable salt, amide or ester thereof, 20%-90% of N (8-(2-hydroxybenzoyl)amino)caprylate, 1%-50% of arginine or a salt thereof, and 1%-50% of leucine or a salt thereof;
a composition (i1) comprising, relative to the total mass of the composition, 1%-20% of the compound shown as 18# in FIG. 3 or a pharmaceutically acceptable salt, amide or ester thereof, 20%-90% of N (8-(2-hydroxybenzoyl)amino)caprylate, 1%-50% of arginine or a salt thereof, and 1%-50% of lysine or a salt thereof; and
a composition (j 1) comprising, relative to the total mass of the composition, 1%-20% of the compound shown as 18# in FIG. 3 or a pharmaceutically acceptable salt, amide or ester thereof, 20%-90% of N (8-(2-hydroxybenzoyl)amino)caprylate, 1%-50% of arginine or a salt thereof, and 1%-50% of sodium caprylate;
wherein preferably, the pharmaceutical composition is selected from any one of the following compositions:
a composition (a2) comprising, relative to the total mass of the composition, 1%-5% of the compound shown as 18# in FIG. 3 or a pharmaceutically acceptable salt, amide or ester thereof and at least 70%, e.g., at least 90%, of N-(8-(2-hydroxybenzoyl)amino)caprylate;
a composition (b2) comprising, relative to the total mass of the composition, 1%-20% of the compound shown as 18# in FIG. 3 or a pharmaceutically acceptable salt, amide or ester thereof, 70%-90% of N (8-(2-hydroxybenzoyl)amino)caprylate, and 5%-10% of sodium caprylate;
a composition (b3) comprising, relative to the total mass of the composition, 1%-20% of the compound shown as 18# in FIG. 3 or a pharmaceutically acceptable salt, amide or ester thereof, 50%-70% or 50%-85% of *N*-(8-(2-hydroxybenzoyl)amino)caprylate, and 10%-45% of sodium caprylate;
a composition (b4) comprising, relative to the total mass of the composition, 1%-20% of the compound shown as 18# in FIG. 3 or a pharmaceutically acceptable salt, amide or ester thereof, 20%-50% of *N*-(8-(2-hydroxybenzoyl)amino)caprylate, and 10%-75% or 40%-75% of sodium caprylate;
a composition (c2) comprising, relative to the total mass of the composition, 1%-20% of the compound shown as 18# in FIG. 3 or a pharmaceutically acceptable salt, amide or ester thereof, 70%-90% of N (8-(2-hydroxybenzoyl)amino)caprylate, and 5%-10% or 1%-20% of arginine or a salt thereof;
a composition (c3) comprising, relative to the total mass of the composition, 1%-20% of the compound shown as 18# in FIG. 3 or a pharmaceutically acceptable salt, amide or ester thereof, 50%-70% or 50%-85% of *N*-(8-(2-hydroxybenzoyl)amino)caprylate, and 10%-45% of arginine or a salt thereof;
a composition (c4) comprising, relative to the total mass of the composition, 1%-20% of the compound shown as 18# in FIG. 3 or a pharmaceutically acceptable salt, amide or ester thereof, 20%-50% of *N*-(8-(2-hydroxybenzoyl)amino)caprylate, and 10%-75% or 40%-75% of arginine or a salt thereof;
a composition (c5) comprising, relative to the total mass of the composition, 1%-20% of the compound shown as 18# in FIG. 3 or a pharmaceutically acceptable salt, amide or ester thereof, 70%-90% of N (8-(2-hydroxybenzoyl)amino)caprylate, and 5%-25% of arginine or a salt thereof;
a composition (d2) comprising, relative to the total mass of the composition, 1%-20% of the compound shown as 18# in FIG. 3 or a pharmaceutically acceptable salt, amide or ester thereof, 70%-90% of *N-*(8-(2-hydroxybenzoyl)amino)caprylate, and 5%-10% or 1%-20% of β-cyclodextrin;
a composition (d3) comprising, relative to the total mass of the composition, 1%-20% of the compound shown as 18# in FIG. 3 or a pharmaceutically acceptable salt, amide or ester thereof, 50%-70% or 50%-85% of *N*-(8-(2-hydroxybenzoyl)amino)caprylate, and 10%-45% of β-cyclodextrin;
a composition (d4) comprising, relative to the total mass of the composition, 1%-20% of the compound shown as 18# in FIG. 3 or a pharmaceutically acceptable salt, amide or ester thereof, 20%-50% of *N*-(8-(2-hydroxybenzoyl)amino)caprylate, and 10%-75% or 40%-75% of β-cyclodextrin; and
a composition (e2) comprising, relative to the total mass of the composition, 1%-5% of the compound shown as 18# in FIG. 3 or a pharmaceutically acceptable salt, amide or ester thereof and 70%-80% of sodium caprate;
a composition (f1) comprising, relative to the total mass of the composition, 1%-20% of the compound shown as 18# in FIG. 3 or a pharmaceutically acceptable salt, amide or ester thereof, 20%-90% of *N*-(8-(2-hydroxybenzoyl)amino)caprylate, 5%-60% of arginine or a salt thereof, and 1%-50% of sodium caprate;
a composition (g1) comprising, relative to the total mass of the composition, 1%-20% of the compound shown as 18# in FIG. 3 or a pharmaceutically acceptable salt, amide or ester thereof, 20%-90% of N (8-(2-hydroxybenzoyl)amino)caprylate, and 5%-75% of arginine or a salt thereof;
a composition (h1) comprising, relative to the total mass of the composition, 1%-20% of the compound shown as 18# in FIG. 3 or a pharmaceutically acceptable salt, amide or ester thereof, 20%-90% of N (8-(2-hydroxybenzoyl)amino)caprylate, 1%-50% of arginine or a salt thereof, and 1%-50% of leucine or a salt thereof;
a composition (i1) comprising, relative to the total mass of the composition, 1%-20% of the compound shown as 18# in FIG. 3 or a pharmaceutically acceptable salt, amide or ester thereof, 20%-90% of N (8-(2-hydroxybenzoyl)amino)caprylate, 1%-50% of arginine or a salt thereof, and 1%-50% of lysine or a salt thereof; and
a composition (j 1) comprising, relative to the total mass of the composition, 1%-20% of the compound shown as 18# in FIG. 3 or a pharmaceutically acceptable salt, amide or ester thereof, 20%-90% of N (8-(2-hydroxybenzoyl)amino)caprylate, 1%-50% of arginine or a salt thereof, and 1%-50% of sodium caprylate;

32. The pharmaceutical composition according to any one of claims 25 to 31, selected from any one of the following compositions:
a composition (i) comprising the compound shown as 18# in FIG. 3 or a pharmaceutically acceptable salt, amide or ester thereof, *N-*(8-(2-hydroxybenzoyl)amino)caprylate, and one or more of a filler, a binder, and a lubricant;
a composition (ii) comprising the compound shown as 18# in FIG. 3 or a pharmaceutically acceptable salt, amide or ester thereof, *N*-(8-(2-hydroxybenzoyl)amino)caprylate, sodium caprylate, a glidant, and a lubricant;
a composition (iii) comprising the compound shown as 18# in FIG. 3 or a pharmaceutically acceptable salt, amide or ester thereof, *N*-(8-(2-hydroxybenzoyl)amino)caprylate, an amino acid (e.g., one or more of histidine, arginine, leucine and lysine or salts thereof, such as arginine or a salt thereof), a glidant, and a lubricant;
a composition (iv) comprising the compound shown as 18# in FIG. 3 or a pharmaceutically acceptable salt, amide or ester thereof, *N*-(8-(2-hydroxybenzoyl)amino)caprylate, β-cyclodextrin, a glidant, and a lubricant;
a composition (v) comprising the compound shown as 18# in FIG. 3 or a pharmaceutically acceptable salt, amide or ester thereof, sodium caprate, and a protease inhibitor;
a composition (vi) comprising the compound shown as 18# in FIG. 3 or a pharmaceutically acceptable salt, amide or ester thereof, *N*-(8-(2-hydroxybenzoyl)amino)caprylate, arginine or a salt thereof, sodium caprate, a glidant, and a lubricant;
a composition (vii) comprising the compound shown as 18# in FIG. 3 or a pharmaceutically acceptable salt, amide or ester thereof, *N*-(8-(2-hydroxybenzoyl)amino)caprylate, arginine or a salt thereof, leucine or a salt thereof, a glidant, and a lubricant;
a composition (viii) comprising the compound shown as 18# in FIG. 3 or a pharmaceutically acceptable salt, amide or ester thereof, *N*-(8-(2-hydroxybenzoyl)amino)caprylate, arginine or a salt thereof, lysine or a salt thereof, a glidant, and a lubricant;
a composition (ix) comprising the compound shown as 18# in FIG. 3 or a pharmaceutically acceptable salt, amide or ester thereof, *N*-(8-(2-hydroxybenzoyl)amino)caprylate, arginine or a salt thereof, a glidant, a lubricant, a filler, and/or a binder; and
a composition (x) comprising the compound shown as 18# in FIG. 3 or a pharmaceutically acceptable salt, amide or ester thereof, *N-*(8-(2-hydroxybenzoyl)amino)caprylate, arginine or a salt thereof, sodium caprylate, a glidant, and a lubricant;
wherein preferably, the pharmaceutical composition is selected from any one of the following compositions:
a composition (i-1) comprising the compound shown as 18# in FIG. 3 or a pharmaceutically acceptable salt, amide or ester thereof, *N*-(8-(2-hydroxybenzoyl)amino)caprylate, and one or more of microcrystalline cellulose, povidone, and magnesium stearate;
a composition (ii-1) comprising the compound shown as 18# in FIG. 3 or a pharmaceutically acceptable salt, amide or ester thereof, *N*-(8-(2-hydroxybenzoyl)amino)caprylate, sodium caprylate, colloidal silicon dioxide, and magnesium stearate;
a composition (iii-1) comprising the compound shown as 18# in FIG. 3 or a pharmaceutically acceptable salt, amide or ester thereof, *N*-(8-(2-hydroxybenzoyl)amino)caprylate, an amino acid (e.g., one or more of histidine, arginine, leucine and lysine or salts thereof, such as arginine or a salt thereof), colloidal silicon dioxide, and magnesium stearate;
a composition (iv-1) comprising the compound shown as 18# in FIG. 3 or a pharmaceutically acceptable salt, amide or ester thereof, *N*-(8-(2-hydroxybenzoyl)amino)caprylate, β-cyclodextrin, colloidal silicon dioxide, and magnesium stearate;
a composition (v-1) comprising the compound shown as 18# in FIG. 3 or a pharmaceutically acceptable salt, amide or ester thereof, sodium caprate, and PMSF;
a composition (vi-1) comprising the compound shown as 18# in FIG. 3 or a pharmaceutically acceptable salt, amide or ester thereof, *N*-(8-(2-hydroxybenzoyl)amino)caprylate, arginine or a salt thereof, sodium caprate, colloidal silicon dioxide, and magnesium stearate;
a composition (vii-1) comprising the compound shown as 18# in FIG. 3 or a pharmaceutically acceptable salt, amide or ester thereof, *N*-(8-(2-hydroxybenzoyl)amino)caprylate, arginine or a salt thereof, leucine or a salt thereof, colloidal silicon dioxide, and magnesium stearate;
a composition (viii-1) comprising the compound shown as 18# in FIG. 3 or a pharmaceutically acceptable salt, amide or ester thereof, *N*-(8-(2-hydroxybenzoyl)amino)caprylate, arginine or a salt thereof, lysine or a salt thereof, colloidal silicon dioxide, and magnesium stearate;
a composition (ix-1) comprising the compound shown as 18# in FIG. 3 or a pharmaceutically acceptable salt, amide or ester thereof, *N*-(8-(2-hydroxybenzoyl)amino)caprylate, arginine or a salt thereof, sorbitol, colloidal silicon dioxide, and magnesium stearate;
a composition (ix-2) comprising the compound shown as 18# in FIG. 3 or a pharmaceutically acceptable salt, amide or ester thereof, *N*-(8-(2-hydroxybenzoyl)amino)caprylate, arginine or a salt thereof, sorbitol, povidone, colloidal silicon dioxide, and magnesium stearate;
a composition (ix-3) comprising the compound shown as 18# in FIG. 3 or a pharmaceutically acceptable salt, amide or ester thereof, *N*-(8-(2-hydroxybenzoyl)amino)caprylate, arginine or a salt thereof, sorbitol, hydroxypropyl cellulose, colloidal silicon dioxide, and magnesium stearate; and
a composition (x-1) comprising the compound shown as 18# in FIG. 3 or a pharmaceutically acceptable salt, amide or ester thereof, *N*-(8-(2-hydroxybenzoyl)amino)caprylate, arginine or a salt thereof, sodium caprylate, colloidal silicon dioxide, and magnesium stearate.

33. The pharmaceutical composition according to any one of claims 25 to 31, selected from any one of the following compositions:
a composition (i-2) comprising, relative to the total mass of the composition, 1%-5% of the compound shown as 18# in FIG. 3 or a pharmaceutically acceptable salt, amide or ester thereof, 70%-80% of *N*-(8-(2-hydroxybenzoyl)amino)caprylate, about 20% of a filler, about 2% of a binder, and 0.5%-5% of a lubricant;
a composition (i-3) comprising, relative to the total mass of the composition, 1%-5% of the compound shown as 18# in FIG. 3 or a pharmaceutically acceptable salt, amide or ester thereof, at least 90% of *N*-(8-(2-hydroxybenzoyl)amino)caprylate, and 0.5%-5% of a lubricant;
a composition (ii-2) comprising, relative to the total mass of the composition, 1%-20%, e.g., 1%-5%, of the compound shown as 18# in FIG. 3 or a pharmaceutically acceptable salt, amide or ester thereof, 70%-90% of *N*-(8-(2-hydroxybenzoyl)amino)caprylate, 5%-10% or 5%-25% of sodium caprylate, 1%-1.5% of a glidant, and 0.5%-5% of a lubricant;
a composition (ii-3) comprising, relative to the total mass of the composition, 1%-20%, e.g., 1%-5%, of the compound shown as 18# in FIG. 3 or a pharmaceutically acceptable salt, amide or ester thereof, 50%-70% or 50%-85% of N (8-(2-hydroxybenzoyl)amino)caprylate, 10%-45% of sodium caprylate, 1%-1.5% of a glidant, and 0.5%-5% of a lubricant;
a composition (ii-4) comprising, relative to the total mass of the composition, 1%-20%, e.g., 1%-5%, of the compound shown as 18# in FIG. 3 or a pharmaceutically acceptable salt, amide or ester thereof, 20%-50% of N (8-(2-hydroxybenzoyl)amino)caprylate, 10%-75% or 40%-75% of sodium caprylate, 1%-1.5% of a glidant, and 0.5%-5% of a lubricant;
a composition (iii-2) comprising, relative to the total mass of the composition, 1%-20%, e.g., 1%-5%, of the compound shown as 18# in FIG. 3 or a pharmaceutically acceptable salt, amide or ester thereof, 70%-90% of *N*-(8-(2-hydroxybenzoyl)amino)caprylate, 5%-10% or 1%-20% of arginine, 1%-1.5% of a glidant, and 0.5%-5% of a lubricant;
a composition (iii-3) comprising, relative to the total mass of the composition, 1%-20%, e.g., 1%-5%, of the compound shown as 18# in FIG. 3 or a pharmaceutically acceptable salt, amide or ester thereof, 50%-70% or 50%-85% of N (8-(2-hydroxybenzoyl)amino)caprylate, 10%-45% of arginine, 1%-1.5% of a glidant, and 0.5%-5% of a lubricant;
a composition (iii-4) comprising, relative to the total mass of the composition, 1%-20%, e.g., 1%-5%, of the compound shown as 18# in FIG. 3 or a pharmaceutically acceptable salt, amide or ester thereof, 20%-50% of N (8-(2-hydroxybenzoyl)amino)caprylate, 10%-75% or 40%-75% of arginine, 1%-1.5% of a glidant, and 0.5%-5% of a lubricant;
a composition (iii-5) comprising, relative to the total mass of the composition, 1%-20%, e.g., 1%-5%, of the compound shown as 18# in FIG. 3 or a pharmaceutically acceptable salt, amide or ester thereof, 70%-90% of *N*-(8-(2-hydroxybenzoyl)amino)caprylate, 5%-25% of arginine, 1%-1.5% of a glidant, and 0.5%-5% of a lubricant;
a composition (iv-2) comprising, relative to the total mass of the composition, 1%-20%, e.g., 1%-5%, of the compound shown as 18# in FIG. 3 or a pharmaceutically acceptable salt, amide or ester thereof, 70%-90% of *N*-(8-(2-hydroxybenzoyl)amino)caprylate, 5%-10% or 1%-20% of β-cyclodextrin, 1%-1.5% of a glidant, and 0.5%-5% of a lubricant;
a composition (iv-3) comprising, relative to the total mass of the composition, 1%-20%, e.g., 1%-5%, of the compound shown as 18# in FIG. 3 or a pharmaceutically acceptable salt, amide or ester thereof, 50%-70% or 50%-85% of N (8-(2-hydroxybenzoyl)amino)caprylate, 10%-45% of β-cyclodextrin, 1%-1.5% of a glidant, and 0.5%-5% of a lubricant;
a composition (iv-4) comprising, relative to the total mass of the composition, 1%-20%, e.g., 1%-5%, of the compound shown as 18# in FIG. 3 or a pharmaceutically acceptable salt, amide or ester thereof, 20%-50% of N (8-(2-hydroxybenzoyl)amino)caprylate, 10%-75% or 40%-75% of β-cyclodextrin, 1%-1.5% of a glidant, and 0.5%-5% of a lubricant;
a composition (v-2) comprising, relative to the total mass of the composition, 1%-2% or 1%-5% of the compound shown as 18# in FIG. 3 or a pharmaceutically acceptable salt, amide or ester thereof, 70%-80% of sodium caprate, and 15%-25% of a protease inhibitor; a composition (v-3) comprising, relative to the total mass of the composition, 1%-5% of the compound shown as 18# in FIG. 3 or a pharmaceutically acceptable salt, amide or ester thereof, 65%-80% of sodium caprate, and 15%-30% of a protease inhibitor;
a composition (vi-2) comprising, relative to the total mass of the composition, 1%-20%, e.g., 1%-5%, of the compound shown as 18# in FIG. 3 or a pharmaceutically acceptable salt, amide or ester thereof, 50%-90% of *N*-(8-(2-hydroxybenzoyl)amino)caprylate, 1%-30% of arginine or a salt thereof, 1%-30% of sodium caprate, 1%-1.5% of a glidant, and 0.5%-5% of a lubricant;
a composition (vi-3) comprising, relative to the total mass of the composition, 1%-20%, e.g., 1%-5%, of the compound shown as 18# in FIG. 3 or a pharmaceutically acceptable salt, amide or ester thereof, 20%-50% of N-(8-(2-hydroxybenzoyl)amino)caprylate, 15%-40% of arginine or a salt thereof, 10%-35% of sodium caprate, 1%-1.5% of a glidant, and 0.5%-5% of a lubricant;
a composition (vi-4) comprising, relative to the total mass of the composition, 1%-20%, e.g., 1%-5%, of the compound shown as 18# in FIG. 3 or a pharmaceutically acceptable salt, amide or ester thereof, 50%-70% of N (8-(2-hydroxybenzoyl)amino)caprylate, 10%-35% of arginine or a salt thereof, 10%-35% of sodium caprate, 1%-1.5% of a glidant, and 0.5%-5% of a lubricant;
a composition (vii-2) comprising, relative to the total mass of the composition, 1%-20%, e.g., 1%-5%, of the compound shown as 18# in FIG. 3 or a pharmaceutically acceptable salt, amide or ester thereof, 50%-90% of *N*-(8-(2-hydroxybenzoyl)amino)caprylate, 1%-30% of arginine or a salt thereof, 1%-30% of leucine or a salt thereof, 1%-1.5% of a glidant, and 0.5%-5% of a lubricant;
a composition (vii-3) comprising, relative to the total mass of the composition, 1%-20%, e.g., 1%-5%, of the compound shown as 18# in FIG. 3 or a pharmaceutically acceptable salt, amide or ester thereof, 20%-50% of N (8-(2-hydroxybenzoyl)amino)caprylate, 10%-35% of arginine or a salt thereof, 15%-40% of leucine or a salt thereof, 1%-1.5% of a glidant, and 0.5%-5% of a lubricant;
a composition (vii-4) comprising, relative to the total mass of the composition, 1%-20%, e.g., 1%-5%, of the compound shown as 18# in FIG. 3 or a pharmaceutically acceptable salt, amide or ester thereof, 50%-70% of N (8-(2-hydroxybenzoyl)amino)caprylate, 10%-35% of arginine or a salt thereof, 10%-35% of leucine or a salt thereof, 1%-1.5% of a glidant, and 0.5%-5% of a lubricant;
a composition (viii-2) comprising, relative to the total mass of the composition, 1%-20%, e.g., 1%-5%, of the compound shown as 18# in FIG. 3 or a pharmaceutically acceptable salt, amide or ester thereof, 50%-90% of *N*-(8-(2-hydroxybenzoyl)amino)caprylate, 1%-30% of arginine or a salt thereof, 1%-30% of lysine or a salt thereof, 1%-1.5% of a glidant, and 0.5%-5% of a lubricant;
a composition (viii-3) comprising, relative to the total mass of the composition, 1%-20%, e.g., 1%-5%, of the compound shown as 18# in FIG. 3 or a pharmaceutically acceptable salt, amide or ester thereof, 20%-50% of N (8-(2-hydroxybenzoyl)amino)caprylate, 10%-35% of arginine or a salt thereof, 15%-40% of lysine or a salt thereof, 1%-1.5% of a glidant, and 0.5%-5% of a lubricant;
a composition (viii-4) comprising, relative to the total mass of the composition, 1%-20%, e.g., 1%-5%, of the compound shown as 18# in FIG. 3 or a pharmaceutically acceptable salt, amide or ester thereof, 50%-70% of N (8-(2-hydroxybenzoyl)amino)caprylate, 10%-35% of arginine or a salt thereof, 10%-35% of lysine or a salt thereof, 1%-1.5% of a glidant, and 0.5%-5% of a lubricant;
a composition (ix-4) comprising, relative to the total mass of the composition, 1%-20%, e.g., 1%-5%, of the compound shown as 18# in FIG. 3 or a pharmaceutically acceptable salt, amide or ester thereof, 70%-90% of *N*-(8-(2-hydroxybenzoyl)amino)caprylate, 5%-25% of arginine or a salt thereof, 1%-1.5% of a glidant, 0.5%-5% of a lubricant, 1%-20% of a filler, and/or 0.5%-5% of a binder;
a composition (ix-5) comprising, relative to the total mass of the composition, 1%-20%, e.g., 1%-5%, of the compound shown as 18# in FIG. 3 or a pharmaceutically acceptable salt, amide or ester thereof, 50%-70% of N (8-(2-hydroxybenzoyl)amino)caprylate, 10%-40% of arginine, 1%-1.5% of a glidant and 0.5%-5% of a lubricant, 5%-35% of a filler, and/or 0.5%-5% of a binder;
a composition (ix-6) comprising, relative to the total mass of the composition, 1%-20%, e.g., 1%-5%, of the compound shown as 18# in FIG. 3 or a pharmaceutically acceptable salt, amide or ester thereof, 20%-50% of N (8-(2-hydroxybenzoyl)amino)caprylate, 10%-75% of arginine, 1%-1.5% of a glidant and 0.5%-5% of a lubricant, 1%-40% of a filler, and/or 0.5%-5% of a binder;
a composition (x-2) comprising, relative to the total mass of the composition, 1%-20%, e.g., 1%-5%, of the compound shown as 18# in FIG. 3 or a pharmaceutically acceptable salt, amide or ester thereof, 50%-90% of *N*-(8-(2-hydroxybenzoyl)amino)caprylate, 1%-30% of arginine or a salt thereof, 1%-30% of sodium caprylate, 1%-1.5% of a glidant, and 0.5%-5% of a lubricant;
a composition (x-3) comprising, relative to the total mass of the composition, 1%-20%, e.g., 1%-5%, of the compound shown as 18# in FIG. 3 or a pharmaceutically acceptable salt, amide or ester thereof, 20%-50% of N-(8-(2-hydroxybenzoyl)amino)caprylate, 15%-40% of arginine or a salt thereof, 10%-35% of sodium caprylate, 1%-1.5% of a glidant, and 0.5%-5% of a lubricant;
a composition (x-4) comprising, relative to the total mass of the composition, 1%-20%, e.g., 1%-5%, of the compound shown as 18# in FIG. 3 or a pharmaceutically acceptable salt, amide or ester thereof, 50%-70% of N (8-(2-hydroxybenzoyl)amino)caprylate, 10%-35% of arginine or a salt thereof, 10%-35% of sodium caprylate, 1%-1.5% of a glidant, and 0.5%-5% of a lubricant.

34. The pharmaceutical composition according to any one of claims 25 to 33, selected from any one of the following compositions:
a composition (1) comprising, relative to the total mass of the composition, 1%-2% of the compound shown as 18# in FIG. 3 or a pharmaceutically acceptable salt, amide or ester thereof, about 75% of *N-*(8-(2-hydroxybenzoyl)amino)caprylate, about 20% of microcrystalline cellulose, about 2% of povidone, and about 0.5% of magnesium stearate;
a composition (2) comprising, relative to the total mass of the composition, 3%-3.5% of the compound shown as 18# in FIG. 3 or a pharmaceutically acceptable salt, amide or ester thereof, 94%-95% of *N*-(8-(2-hydroxybenzoyl)amino)caprylate, and about 2.5% of magnesium stearate;
a composition (3) comprising, relative to the total mass of the composition, about 8% of the compound shown as 18# in FIG. 3 or a pharmaceutically acceptable salt, amide or ester thereof, about 80% of *N-*(8-(2-hydroxybenzoyl)amino)caprylate, about 9% of sodium caprylate, about 1% of colloidal silicon dioxide, and about 2.5% of magnesium stearate;
a composition (4) comprising, relative to the total mass of the composition, about 3% of the compound shown as 18# in FIG. 3 or a pharmaceutically acceptable salt, amide or ester thereof, about 85% of *N-*(8-(2-hydroxybenzoyl)amino)caprylate, about 10% of sodium caprylate, about 1% of colloidal silicon dioxide, and about 2.5% of magnesium stearate;
a composition (5) comprising, relative to the total mass of the composition, about 1.5% of the compound shown as 18# in FIG. 3 or a pharmaceutically acceptable salt, amide or ester thereof, about 85% of *N-*(8-(2-hydroxybenzoyl)amino)caprylate, about 10% of sodium caprylate, about 1% of colloidal silicon dioxide, and about 2.5% of magnesium stearate;
a composition (6) comprising, relative to the total mass of the composition, about 3% of the compound shown as 18# in FIG. 3 or a pharmaceutically acceptable salt, amide or ester thereof, about 85% of *N-*(8-(2-hydroxybenzoyl)amino)caprylate, about 10% of sodium caprylate, about 1% of colloidal silicon dioxide, and about 2.5% of magnesium stearate;
a composition (7) comprising, relative to the total mass of the composition, about 5% of the compound shown as 18# in FIG. 3 or a pharmaceutically acceptable salt, amide or ester thereof, about 85% of N (8-(2-hydroxybenzoyl)amino)caprylate, about 10% of sodium caprylate, about 1% of colloidal silicon dioxide, and about 2.5% of magnesium stearate;
a composition (8) comprising, relative to the total mass of the composition, about 5.5% of the compound shown as 18# in FIG. 3 or a pharmaceutically acceptable salt, amide or ester thereof, 80%-85% of N (8-(2-hydroxybenzoyl)amino)caprylate, about 10% of sodium caprylate, about 1% of colloidal silicon dioxide, and about 2.5% of magnesium stearate;
a composition (9) comprising, relative to the total mass of the composition, about 15% of the compound shown as 18# in FIG. 3 or a pharmaceutically acceptable salt, amide or ester thereof, 70%-75% of *N*-(8-(2-hydroxybenzoyl)amino)caprylate, 8%-10% of sodium caprylate, about 1% of colloidal silicon dioxide, and about 2.5% of magnesium stearate;
a composition (10) comprising, relative to the total mass of the composition, about 3% of the compound shown as 18# in FIG. 3 or a pharmaceutically acceptable salt, amide or ester thereof, about 85% of N (8-(2-hydroxybenzoyl)amino)caprylate, about 10% of arginine or a salt thereof, about 1.5% of colloidal silicon dioxide, and about 1.5% of magnesium stearate;
a composition (11) comprising, relative to the total mass of the composition, about 3% of the compound shown as 18# in FIG. 3 or a pharmaceutically acceptable salt, amide or ester thereof, about 60% of *N*-(8-(2-hydroxybenzoyl)amino)caprylate, about 35% of arginine or a salt thereof, about 1.5% of colloidal silicon dioxide, and about 1% of magnesium stearate;
a composition (12) comprising, relative to the total mass of the composition, about 2.5% of the compound shown as 18# in FIG. 3 or a pharmaceutically acceptable salt, amide or ester thereof, about 45% of *N*-(8-(2-hydroxybenzoyl)amino)caprylate, about 50% of arginine or a salt thereof, about 1.5% of colloidal silicon dioxide, and about 1% of magnesium stearate;
a composition (13) comprising, relative to the total mass of the composition, about 4% of the compound shown as 18# in FIG. 3 or a pharmaceutically acceptable salt, amide or ester thereof, about 75% of *N*-(8-(2-hydroxybenzoyl)amino)caprylate, about 18% of arginine or a salt thereof, about 1% of colloidal silicon dioxide, and about 2% of magnesium stearate;
a composition (14) comprising, relative to the total mass of the composition, 2%-5% or 5% of the compound shown as 18# in FIG. 3 or a pharmaceutically acceptable salt, amide or ester thereof, about 58% of N (8-(2-hydroxybenzoyl)amino)caprylate, about 35% of arginine or a salt thereof, about 1% of colloidal silicon dioxide, and about 2% of magnesium stearate;
a composition (15) comprising, relative to the total mass of the composition, about 3% of the compound shown as 18# in FIG. 3 or a pharmaceutically acceptable salt, amide or ester thereof, about 85% of *N-*(8-(2-hydroxybenzoyl)amino)caprylate, about 10% of histidine, about 1.5% of colloidal silicon dioxide, and about 1.5% of magnesium stearate;
a composition (16) comprising, relative to the total mass of the composition, about 3% of the compound shown as 18# in FIG. 3 or a pharmaceutically acceptable salt, amide or ester thereof, about 85% of *N-*(8-(2-hydroxybenzoyl)amino)caprylate, about 10% of β-cyclodextrin, about 0.5% of colloidal silicon dioxide, and about 2.5% of magnesium stearate;
a composition (17) comprising, relative to the total mass of the composition, about 1.5% of the compound shown as 18# in FIG. 3 or a pharmaceutically acceptable salt, amide or ester thereof, about 80% of sodium caprate, and about 20% of PMSF;
a composition (18) comprising, relative to the total mass of the composition, about 2.5% of the compound shown as 18# in FIG. 3 or a pharmaceutically acceptable salt, amide or ester thereof, about 47% of *N-*(8-(2-hydroxybenzoyl)amino)caprylate, about 28% of arginine or a salt thereof, about 20% of sodium caprate, about 1% of colloidal silicon dioxide, and about 2% of magnesium stearate;
a composition (19) comprising, relative to the total mass of the composition, about 4% of the compound shown as 18# in FIG. 3 or a pharmaceutically acceptable salt, amide or ester thereof, about 47% of *N-*(8-(2-hydroxybenzoyl)amino)caprylate, about 19% of arginine or a salt thereof, about 28% of sodium caprate, about 1% of colloidal silicon dioxide, and about 2% of magnesium stearate;
a composition (20) comprising, relative to the total mass of the composition, about 5% of the compound shown as 18# in FIG. 3 or a pharmaceutically acceptable salt, amide or ester thereof, about 58% of *N-*(8-(2-hydroxybenzoyl)amino)caprylate, about 23% of arginine or a salt thereof, about 12% of sodium caprate, about 1% of colloidal silicon dioxide, and about 2% of magnesium stearate;
a composition (21) comprising, relative to the total mass of the composition, about 4% of the compound shown as 18# in FIG. 3 or a pharmaceutically acceptable salt, amide or ester thereof, about 47% of N (8-(2-hydroxybenzoyl)amino)caprylate, about 19% of arginine or a salt thereof, about 28% of leucine or a salt thereof, about 1% of colloidal silicon dioxide, and about 2% of magnesium stearate;
a composition (22) comprising, relative to the total mass of the composition, about 4% of the compound shown as 18# in FIG. 3 or a pharmaceutically acceptable salt, amide or ester thereof, about 47% of N (8-(2-hydroxybenzoyl)amino)caprylate, about 19% of arginine or a salt thereof, about 28% of lysine or a salt thereof, about 1% of colloidal silicon dioxide, and about 2% of magnesium stearate;
a composition (23) comprising, relative to the total mass of the composition, about 2%-4% of the compound shown as 18# in FIG. 3 or a pharmaceutically acceptable salt, amide or ester thereof, about 47% of *N-*(8-(2-hydroxybenzoyl)amino)caprylate, about 28% of arginine or a salt thereof, about 20% of sorbitol, about 1% of colloidal silicon dioxide, and about 2% of magnesium stearate;
a composition (24) comprising, relative to the total mass of the composition, about 4% of the compound shown as 18# in FIG. 3 or a pharmaceutically acceptable salt, amide or ester thereof, about 45% of *N-*(8-(2-hydroxybenzoyl)amino)caprylate, about 20% of arginine or a salt thereof, about 28% of sorbitol, about 1% of colloidal silicon dioxide, and about 2% of magnesium stearate;
a composition (25) comprising, relative to the total mass of the composition, about 4% of the compound shown as 18# in FIG. 3 or a pharmaceutically acceptable salt, amide or ester thereof, about 47% of N (8-(2-hydroxybenzoyl)amino)caprylate, about 20% of arginine or a salt thereof, about 25% of sorbitol, about 1.5% of povidone or hydroxypropyl cellulose, about 1% of colloidal silicon dioxide, and about 2% of magnesium stearate;
a composition (26) comprising, relative to the total mass of the composition, about 2.5% of the compound shown as 18# in FIG. 3 or a pharmaceutically acceptable salt, amide or ester thereof, about 47% of *N-*(8-(2-hydroxybenzoyl)amino)caprylate, about 28% of arginine or a salt thereof, about 20% of sodium caprylate, about 1% of colloidal silicon dioxide, and about 2% of magnesium stearate.

35. The pharmaceutical composition according to any one of claims 1 to 34, wherein the pharmaceutical composition is a powder, a granule, a tablet, a capsule, or a lyophilized formulation;
preferably, the pharmaceutical composition is a tablet or a capsule;
more preferably, the pharmaceutical composition is an immediate release formulation or an enteric-coated formulation.

36. A method for preparing the pharmaceutical composition according to any one of claims 1 to 35, comprising the step of mixing the GLP-1 analog or the pharmaceutically acceptable salt, amide or ester thereof with the absorption enhancer, wherein
preferably, the method comprises the step of granulating, tableting, or capsule filling;
more preferably, the method comprises the step of direct powder compression, wet granulation, dry granulation, tableting, or filling enteric capsule shells.

37. Use of the pharmaceutical composition according to any one of claims 1 to 35 in the preparation of a medicament for treating non-insulin-dependent diabetes, insulin-dependent diabetes, obesity, non-alcoholic fatty liver, hepatic steatosis, diabetic retinopathy, diabetic neuropathy, diabetic nephropathy, insulin resistance, dyslipidemia associated with insulin resistance, and/or dyslipidemia associated with diabetes.
